# EUROPÄISCHE PATENTANMELDUNG

(11) **EP 2 633 885 A1**
(43) Veröffentlichungstag der Anmeldung: **04.09.2013**
(21) Anmeldenummer: 12157903.1
(22) Anmeldetag: 02.03.2012
(51) Int. Cl.: A61P 17/00, A61P 29/00, A61P 1/00, A61K 31/192, A61K 31/353

(54) **Verbindungen und Mischungen zur Beeinflussung von entzündlichen Zuständen**

(71) Anmelder: Symrise AG, 37603 Holzminden (DE)
(72) Erfinder: Reichelt, Katharina, 37603 Holzminden (DE); Ley, Jakob Peter, 37603 Holzminden (DE); Blings, Maria, 37603 Holzminden (DE); Götz, Marcus Rudolf, 34399 Oberweser (DE)
(74) Vertreter: Fabry, Bernd

(57) **Zusammenfassung**

Die Erfindung betrifft bestimmte Verbindungen, Salze dieser Verbindungen und Mischungen umfassend oder bestehend aus zwei oder mehr solchen Verbindungen, zwei oder mehr solchen Salzen oder einer oder mehreren solchen Verbindungen und einem oder mehreren solchen Salzen, jeweils zur Anwendung in einem Verfahren zur Prophylaxe und/oder Behandlung von Entzündungen, insbesondere von Entzündungen der Haut.

## Beschreibung

Die vorliegende Erfindung betrifft primär neue Anwendungen zur Prophylaxe und/oder Behandlung von Entzündungen, nämlich Verbindungen der Formel (X)

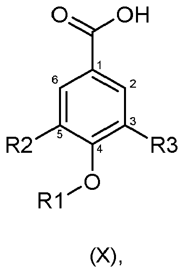

Salze von Verbindungen der Formel (X) und Mischungen umfassend oder bestehend aus zwei oder mehr unterschiedlichen Verbindungen der Formel (X), zwei oder mehr unterschiedlichen Salzen von Verbindungen der Formel (X) oder einer oder mehreren unterschiedlichen Verbindungen der Formel (X) und einem oder mehreren unterschiedlichen Salzen von Verbindungen der Formel (X), wobei für R1, R2 und R3 das hierin Beschriebene, insbesondere das in den Ansprüchen Gesagte, gilt, zur Anwendung in einem Verfahren zur Prophylaxe und/oder Behandlung von Entzündungen, insbesondere von Entzündungen der Haut, insbesondere in einem Verfahren zum Reduzieren der Freisetzung von TNF-alpha, und/oder zum Reduzieren der Freisetzung eines Interleukins, vorzugsweise von IL-1, IL-6 und/oder IL-8, und/oder zum Reduzieren der Freisetzung eines Prostaglandins, vorzugsweise von PGE2, und/oder zum Reduzieren der Freisetzung von Interferon-gamma und/oder NF-κB.

Die vorliegende Erfindung betrifft auch Zubereitungen, insbesondere der Ernährung oder dem Genuss dienende Zubereitungen, pharmazeutische Zubereitungen, kosmetische Zubereitungen und dermatologische Zubereitungen, die eine erfindungsgemäß anzuwendende Verbindung, ein erfindungsgemäß anzuwendendes Salz oder eine erfindungsgemäß anzuwendende Mischung (wie hierin beschrieben) umfassen, zur Anwendung in einem Verfahren zur Prophylaxe und/oder Behandlung von Entzündungen, insbesondere von Entzündungen der Haut, insbesondere in einem Verfahren zum Reduzieren der Freisetzung von TNF-alpha, und/oder zum Reduzieren der Freisetzung eines Interleukins, vorzugsweise von IL-1, IL-6 und/oder IL-8, und/oder zum Reduzieren der Freisetzung eines Prostaglandins, vorzugsweise von PGE2, und/oder zum Reduzieren der Freisetzung von Interferon-gamma und/oder NF-κB.

Weitere Aspekte der vorliegenden Erfindung ergeben sich aus der nachfolgenden Beschreibung und insbesondere den Patentansprüchen.

Es besteht ein ständiges Bedürfnis, entzündungshemmende Stoffe zum Schutz von Zellen bzw. Gewebe (von Menschen und Tieren), insbesondere der Haut, zur Verfügung zu stellen, vor allem zur Verwendung in kosmetischen Zubereitungen, pharmazeutischen Zubereitungen, Nahrungs- oder Genussmitteln. Insbesondere besteht ein ständiges Bedürfnis, neue anti-inflammatorisch wirksame Stoffe zu finden, die in physiologischen Systemen (von Mensch und Tier) die natürlichen Abwehrmechanismen gegen Entzündungen unterstützen. Diesbezüglich besteht ein besonders großes Interesse an Substanzen aus natürlichen Extrakten. Attraktiv für einen Einsatz in Lebensmitteln sind insbesondere Pflanzen bzw. Teile oder Extrakte von Pflanzen, die eine lange Verzehrhistorie aufweisen.

Im Rahmen des vorliegenden Textes umfasst der Begriff "Haut" nicht nur die (menschliche bzw. tierische) Haut im üblichen Sinne, sondern generell Zellschichten, die innere und/oder äußere Oberflächen am/im menschlichen bzw. tierischen Körper bedecken. Demnach umfasst der Begriff "Haut" im Rahmen des vorliegenden Textes Oberflächen- und Drüsenepithelien, d.h. insbesondere auch Schleimhäute, z.B. die Mundschleimhaut, die Magenschleimhaut und die Darmschleimhaut. Die Schleimhäute sind - als Barriere-Organe des (menschlichen) Organismus - in besonderem Maße äußeren Einflüssen ausgesetzt. Sie kleiden die verschiedenen Körperhöhlen aus, die entweder in Kontakt mit der äußeren Umgebung (z.B. Mund und Rachen) oder den inneren Organen eines Körpers (z.B. Darmlumen) stehen.

Viele intrinsische Faktoren (z.B. genetische Prädisposition) und extrinsische Faktoren (z.B. Schäden der Hautbarriere, Einfluss von UV-Licht, hautreizenden oder allergieauslösende Substanzen) können zu Hautirritationen oder Fehlfunktionen der Haut führen.

Im Rahmen des vorliegenden Textes werden "Hautirritationen" verstanden als jegliche Veränderung der Haut, die Unwohlsein ("sensorial malaise") bei Menschen oder Tieren auslöst, und/oder charakterisiert ist durch Symptome trockener, geröteter und/oder entzündeter Haut. Der Begriff "sensorial malaise" beinhaltet ebenfalls Zustände, die mit Juckreiz oder Schmerzen einhergehen.

Hautirritation kann folgende Zustände der Haut beinhalten: empfindliche Haut, zum Beispiel empfindliche Kopfhaut, leicht verletzbare Haut, atopische Haut (Atopie), gereizte oder entzündete Haut, was sich in Form von Hautrötung (Erythem) darstellen kann.

Hautreizungen können insbesondere auch
- Reizungen der Schleimhäute in der Mundhöhle, zum Beispiel Parodontitis, und Gingivitis (wie weiter unten im Detail beschrieben),
- Reizungen und Infektionen der Atemwege (wie weiter unten im Detail beschrieben; s. hierzu auch US 2009/0238905), zum Beispiel Rhinosinusitis (Erkältung), Sinusitis und Pharyngitis/Tonsillitis, und
- Reizungen des Gastrointestinaltrakts (wie weiter unten im Detail beschrieben; s. hierzu auch US 2009/0238905)
betreffen bzw. umfassen.

Das Problem empfindlicher Haut betrifft eine wachsende Anzahl Erwachsener und Kinder. Es wird angenommen, dass ein Anteil von bis zu 50 % der Bevölkerung empfindliche Haut hat (L. Misery et al., Ann. Dermatol. Venereol. 2005, 132, 425-429). Empfindliche Haut beschreibt eine Haut, die eine verringerte Schwelle für Reizstoffe aufweist, sowie hyper-reaktive, intolerante und auch atopische Haut. Im Falle von Menschen mit empfindlicher oder leicht verletzbarer Haut, kann das so genannte "stinging" (engl. "to sting" = brennen, stechen, schmerzhaft sein) beobachtet werden. Typische Symptome, die assoziiert sind mit "stinging" oder "empfindlicher Haut" im Allgemeinen sind Hautrötungen, Kribbeln, Spannungsgefühle sowie Brennen der Haut und Juckreiz. Sie können durch bestimmte Umwelteinflüsse, z.B. Massage, Einfluss von Tensiden, Wetter (Hitze, Kälte, Trockenheit oder hohe Luftfeuchtigkeit), thermische oder UV Strahlung (z.B. ausgehend von der Sonne) oder auch durch psychologischen Stress ausgelöst werden.

Empfindliche Kopfhaut ist ebenfalls gekennzeichnet durch Hautrötung, Kribbeln, Brennen und Stechen. Auslöser sind zum Beispiel Seife, Shampoos oder andere Haarpflegemittel, Tenside, Wasser mit hohem Gehalt an Kalziumkarbonat und/oder (mechanischer) Stress. Erytheme und Hyperseborrhoea (übermäßige Talgproduktion) der Kopfhaut und Schuppen gehen oft mit den beschriebenen Symptomen einher.

Bei ca. 10-20 % der Bevölkerung in industrialisierten Ländern wird (mit steigender Tendenz) Atopie (atopisches Syndrom) beobachtet. Dabei handelt es sich um eine Überempfindlichkeit der Haut gegenüber Stoffen aus der Umwelt mit einer erhöhten Neigung zur Entwicklung überempfindlicher Reaktionen vom Sofort-Typ (Allergien) gegen Stoffe aus der natürlichen Umgebung. Es wird angenommen, dass Atopie genetische Ursachen hat. Atopie kann sich als atopische Dermatitis manifestieren. In diesem Fall ist die Hautbarriere geschädigt, die Haut ist häufig entzündet und juckt.

Parodontitis (als Beispiel einer entzündlichen Reaktion des Zahnfleisches oder der Mundschleimhaut) ist eine Entzündung des Periodontiums (Wurzelhaut), also des Gewebes, welches die Zähne umgibt und stützt. Das Periodontium besteht aus verschiedenen Geweben: Zahnfleischepithel (Gingiva; Zahnfleischs), Verbindungsgewebe der Gingiva, Zahnwurzelhaut (Periodontium, Desmodontium), Zahnzement und umgebender Alveolarknochen. Die Zahnwurzelhaut liegt zwischen der Oberfläche der Zahnwurzel und dem Alveolarknochen und ist ein zellreiches Bindegewebe, welches die Zähne im knöchernen Zahnfach, der Alveole, hält. 53 bis 74% des Parodontalspalts bestehen aus Kollagen- und Oxytalan-Faserbündeln. Parodontalfasern, die sich im Zahnzement und den Alveolarknochen befinden, halten den Zahn im Alveolarknochen. Die Hauptcharakteristika von Parodontitis beinhalten Entzündung des Zahnfleisches, Verlust von Stabilität, Bildung von Taschen in der Zahnwurzelhaut und Abbau des Alveolarknochens.

Die Hauptursache von Parodontitis ist Plaque. Diese besteht aus bestimmten Inhaltsstoffen des Speichels, Essensresten sowie Bakterien und deren Abbauprodukten. Diese spezielle Form einer Infektionskrankheit wird in den meisten Fällen von *Porphyromonas gingivalis, Bacteroides forsythus* und *Actinobacillus actinomycetemcomitans* verursacht. Die kontinuierliche Freisetzung von Bakterientoxinen, insbesondere Lipopolysaccharide (LPS), führt zu einer unspezifischen Reaktion der Immunabwehr. LPS-stimulierte Makrophagen setzen Prostaglandin E2 (PGE2) und pro-inflammatorische Mediatoren in das betroffene Gewebe des Patienten frei, wie z.B. Interleukine (e.g. IL-1 beta) und TNF-alpha. Die pro-inflammatorischen Mediatoren lösen die Freisetzung weiterer PGE2s und matrix-zerstörender Metalloproteinasen (Matrix Metalloproteinasen, MMPs) aus den angesiedelten Fibroblasten aus, die die extrazelluläre Matrix des umgebenden Bindegewebes zerstören. Das wiederum erlaubt Bakterien, die eigentlich in Kontakt mit dem freien Zahnfleisch stehen, tiefer in das darunter liegende Bindegewebe einzudringen und dort die Entzündungsprozesse weiter voranzutreiben, so dass im Weiteren die Verbindung zwischen der obersten Schicht des Epitheliums und der Zahnwurzel verlorengeht. Dadurch entsteht eine Tasche im Zahnfleisch. Die Reaktion des Körpers darauf ist eine Entzündung des Zahnfleisches und der Zahnwurzelhaut mit Schädigung des Alveolarknochens. Im letzten Stadium der Parodontitis besteht für die betroffene Person die Gefahr des Zahnverlusts.

Zusätzlich zu Bakterien können aber auch chemische oder mechanische Schädigungen Irritationen oder Entzündungsreaktionen des Zahnfleisches oder der Mundschleimhäute hervorrufen. Pro-inflammatorische Mediatoren, insbesondere Interleukine wie IL-1 alpha und PGE2, werden in diesem Prozess freigesetzt (Reilly, D. M. and M. R. Green (1999)).

Reizungen und Infektionen der Atemwege betreffen den Respirationstrakt (von Mensch bzw. Tier). Der Respirationstrakt ist in drei Abschnitte unterteilt: (i) die oberen Atemwege, inkl. Nase und Nasennebenhöhlen und Rachen, (ii) die unteren Atemwege mit Kehlkopf und Luftröhre und (iii) die Lungen mit Bronchien, Bronchiolen, Lungenbläschen, etc.

"Reizungen und Infektionen der oberen Atemwege" bezeichnen insbesondere eine akute Infektion, die die oberen Atemwege, insbesondere Nase, Sinus, Pharynx und/oder Larynx, betreffen. In den Vereinigten Staaten von Amerika werden jedes Jahr ca. eine Milliarde akute Erkrankungen der oberen Atemwege registriert. Reizungen und Infektionen der oberen Atemwege beinhalten Rhinosinusitis (Erkältung), Sinusitis, Pharyngitis/Tonsillitis, Laryngitis und manchmal Bronchitis. Zu den Symptomen dieser Infektionen zählen häufig Schwellungen der Nasenschleimhäute, Husten, Schnupfen, Halsschmerzen, Fieber, Niese und Druckgefühl. Die Symptome beginnen in der Regel 1 bis 3 Tage nach Kontakt mit pathogenen Keimen, meist Viren. Die Symptome halten typischerweise 7 bis 10 Tage an, können aber auch länger andauern.

Eine häufig auftretende (Atemwegs-)Infektion ist die Pharyngitis. Pharyngitis ist, in den meisten Fällen eine schmerzhafte Entzündung des Rachens und wird daher gemeinhin auch als Halsschmerzen bezeichnet. Entzündungen der Mandeln, Mandelentzündung oder Tonsillitis können zeitgleich auftreten.

Für Infektionen der oberen Atemwege gibt es grundsätzlich drei Behandlungsansätze: symptomatisch, remedial und vorbeugend. Symptomatische Behandlung zielt darauf ab, Symptome und Schmerzen zu lindern. Remediale Behandlungen sollen die Pharyngitis heilen, indem sie die Ausbreitung verhindern und den Heilungsprozess beschleunigen. Vorbeugende Behandlung soll den Ausbruch einer Infektion verhindern.

Remediale Behandlungen sind am effektivsten gegen bakterielle Infektionen, z.B. Streptokokken. Viele vorbeugende Behandlungen sind ebenfalls remedial.

Bei viralen Infektionen erfolgt die Erholung von einer Rachenentzündung in der Regel spontan innerhalb weniger Tage. Daher ist die beliebteste Methode die symptomatische Behandlung.

Verschiedene nicht-antibiotische Behandlungen gegen Halsentzündungen wurden in kontrollierten Versuchen getestet. Schmerzstillende Behandlungen zählen dabei zu den effektivsten.

Zu den symptomatischen Behandlungen für Infektionen der oberen Atemwege zählen: Formulierungen, die den Zweck haben, remedial oder symptomatisch zu wirken und in den folgenden Formen vorkommen können:
feste galenische Formen (wie z.B. Tabletten (mit Überzug und ohne, mit modifizierter Freisetzung und ohne), Dragees (mit Überzug und ohne, mit modifizierter Freisetzung und ohne), Kapseln (Weich- oder Hartgelatinekapseln mit modifizierter Freisetzung und ohne) Granulate (mit modifizierter Freisetzung und ohne), Pulver (mit modifizierter Freisetzung und ohne), Suppositorien (mit Überzug und ohne, mit modifizierter Freisetzung und ohne) Lutschbonbons, Kaugummis),
flüssige Formen (wie z.B. Lösungen, Suspensionen, Emulsionen, Sirupe (umgangssprachlich Hustensaft), Mundspülungen, Gurgellösungen, Halssprays oder Nasensprays, Nasentropfen, Nasenspüllösungen, Nasenpulver, Nasensalben oder Ohrentropfen, Ohrensprays, Ohrenspüllösungen, Ohrenpuder, Ohrentampons),
halbfeste Formen (wie z.B. Hydrophobe Salben darunter z.B.: Kohlenwasserstoffgele, Lipogele. Silikongele, Oleogele. sowie wasseraufnehmende Salben darunter z.B. Absorptionsbasen, hydrophile Salben, hydrophile Gele (Hydrogele) oder Pasten,
Inhalate (wie z.B. Druckgas-Dosierinhalatoren, Pulver-Inhalatoren, Inhalatoren mit Zerstäuber, Inhalationskonzentrate zur Inhalation),
wirkstoffhaltige Pflaster oder andere therapeutische Systeme.

Der Gastrointestinaltrakt (auch Verdauungstrakt genannt) ist das System der inneren Organe, die die Nahrung aufnehmen und verdauen, um daraus Nährstoffe aufzunehmen, Energie zu gewinnen und die übrig gebliebenen Nahrungsbestandteile auszuscheiden Die Hauptfunktionen des Verdauungstrakts sind demnach Nahrungsaufnahme, Verdauung, Absorption und Ausscheidung.

Der obere Verdauungstrakt besteht aus Mund, Rachen, Speiseröhre und Magen. Der Mund enthält die Mundschleimhäute, die die Öffnungen der Speicheldrüsen enthalten, die Zunge und die Zähne. Hinter dem Mund liegt der Rachen, der zu einem hohlen Muskelschlauch, der Speiseröhre, führt, die wiederum in den Magen führt. An den Magen schließt der Dünndarm an. Der untere Verdauungstrakt besteht aus den Eingeweiden und dem Anus. Die Eingeweide bestehen aus dem Darm, dem Dünndarm, der aus drei Teilen besteht, Duodenum, Jejunum, Ileum, dem Dickdarm, der ebenfalls aus drei Abschnitten besteht, Caecum mit Wurmfortsatz (Blinddarm), dem Colon (aufsteigendes Colon, transversales Colon, absteigendes Colon) und dem Rektum.

Mit zu den häufigsten Entzündungen des Verdauungstrakts gehören gastro-ösophageale Refluxerkrankungen, Sodbrennen und Magengeschwüre. Zur Behandlung gehören üblicherweise zum einen eine Reduzierung der Symptome und eine Reduzierung der Entzündungen im Gewebe und zum anderen längerfristige Behandlungen um ein wiederholtes Auftreten der Symptome zu verhindern.

Andere entzündliche Erkrankungen des Verdauungssystems sind unter Anderem leichtere entzündliche Erkrankungen wie das irritatable bowel syndrom (IBD) sowie entzündliche Erkrankungen unbekannter Genese und chronisch entzündliche Darmerkrankungen (CED), wie zum Beispiel chronische Colitis (colitis ulcerosa).

Für geeignete Anwendungen zur Vorbeugung bzw. Behandlung chronisch entzündlicher Darmerkrankungen, insbesondere chronischer Colitis (colitis ulcerosa), besteht ein besonders großer Bedarf.

Chronische Entzündungen können als Ursache verschiedener Krankheiten und Lebensbedingungen erscheinen. Sie können mit unterschiedlichsten Bedingungen wie Arthritis, einigen Arten von Krebs, Colitis, Diabetes Melitus, koronaren Herzkrankheiten, Übergewicht, Alzheimer, Immundisfunktion assoziiert sein.

Es gibt grundsätzlich zwei enzymatische Wege der Regulierung der Inflammation. Der Lipoxygenase-Weg (5-LOX) resultiert in der Produktion von Leukotrienen, welche eine pro-inflammatorische Wirkung aufweisen. Der zweite Weg ist die Cyclooxygenase-Weg (COX-1 und COX-2). Ein hoher Level von COX-2 weist auf eine Entzündung hin. Weitere Entzündungsmarker sind Tumornekrose-Faktor (TNF-α), Nuklearfaktor- κB (NF- κB), Interleukin-6 (IL-6), Interleukin-17 (IL-17) und Interleukin-1-β (IL1-β). Die Enzyme, Cytokine und ihre Metaboliten, erhöhen die Produktion von Prostaglandinen und Leukotrienen, welche als interzelluläre Mediatoren fungieren, und im Zusammenhang mit dem Inflammationsprozess stehen. Eine Regulierung insbesondere der Enzyme LOX-5 und COX-2 kann sich bei der Entwicklung/ Suppression von Entzündungen positiv auswirken.

Eine Ernährungsweise, die auf viel Zucker und Stärke, sowie Fett und trans-Fettsäuren basiert, hat einen direkten Zusammenhang mit chronischer Inflammation. Oxidation von mehrfach ungesättigten Fetten und Fettsäuren *in vitro* und *in vivo* führt zur Bildung von reaktiven Sauerstoffspezies (Radikalen), sowie zur Bildung von Stickoxiden. Diese Verbindungen können die erste Phase eines Entzündungsprozesses hervorrufen und/oder fördern. Die Schädigung der DNA kann eine Folge davon sein.

Der Gastrointestinaltrakt ist auf seiner ganzen Länge, insbesondere im Bereich des Darmes, anfällig für Entzündungen, weswegen es sehr wichtig ist, entsprechende Vorgänge zu hemmen und Entzündungen vorzubeugen. Ohne Behandlung können schädliche Prozesse zu Irritationen, akuten und chronischen Entzündungen, bis hin zu Krebs führen.

Chronisch entzündliche Erkrankungen der Schleimhaut des Verdauungstraktes stellen ein erhebliches gesundheitspolitisches Problem dar. Es erkranken gerade jüngere Menschen, die dadurch in ihrer gesamten Lebensführung stark beeinträchtigt sind und Zeit ihres Lebens medizinische Versorgung in Anspruch nehmen müssen. Die Ätiopathogenese der chronisch entzündlichen Erkrankungen des Verdauungstraktes ist nicht vollkommen klar. Es wird jedoch als Ursache für die Entstehung eine Störung der Darmbarriere angenommen.

Colitis Ulcerosa und Morbus Crohn sind Entzündungen des Darmes, welche charakteristische Begleiterscheinungen wie Diahrrö, Blut im Stuhl, abdominale Schmerzen und Krämpfe, Gewichtsabnahme aufweisen. Die Darmschleimhaut sieht dabei rot und geschwollen aus und blutet schon bei geringster Berührung.

Die oberflächlichste Zellschicht stellen die Epithelzellen der Mukosa dar. Die intestinalen Epithelzellen bilden die größte Kontaktfläche des Körpers zur Außenwelt. Sie resorbieren Nahrung und verhindern gleichzeitig das Eindringen von pathogenen Keimen. Letzteres wird durch eine chronische physiologische Entzündung unterstützt. Diese unterliegt einer Reihe von Kontroll- und Regelmechanismen, um einerseits das Eindringen von pathogenen Keimen und andererseits Schäden durch die Entzündungsmediatoren selbst zu vermeiden. Dazu interagieren die Epithelzellen mit den Zellen des Mukosa-assoziierten Immunsystems.

Intestinale Epithelzellen spielen möglicherweise bei der Pathogenese chronisch entzündlicher Darmerkrankungen eine wichtige Rolle. Das bdeutendes Modell der Entstehung chronisch entzündlicher Darmerkrankungen beschreibt folgendes Szenario: Ein Defekt der strukturellen Integrität des intestinalen Epithels führt zu einer Invasion von Antigenen aus dem Darmlumen. Dieser Vorgang kann durch Aktivierung des Mukosa-assoziierten lymphatischen Gewebes in genetisch empfänglichen Patienten eine chronische Entzündung auslösen. Eine Störung des Zell-Zell-Kontakts durch genetische Veränderung des N-Cadherins oder des Keratin 8 löst eine chronische Darmentzündung aus. Epithelzellen besitzen eine Vielzahl von Rezeptoren zur Signalaufnahme. Hierzu zählen vor allem Rezeptoren zur Erkennung bakterieller Motive, so genannte Pattern Recognition Rezeptoren.

Ein solcher Erkennungsrezeptor für bakterielle Motive ist das so genannte NOD2/CARD15 Protein. NOD2/CARD15 ist ein Mitglied der NBS-LRR-Protein-Familie (für nucleotide-binding site and leucine-rich repeat), deren Mitglieder alle eine Rolle bei der intrazellulären Erkennung von Mikroben und ihren Bestandteilen spielen und zu denen z. B. auch Apaf-1 und CARD4/NOD1 zählen, die möglicherweise ebenso bei bestimmten Patienten eine Rolle spielen können. Wenn bakterielle Bestandteile an NOD2/CARD15 binden, führt das normalerweise zu einer Aktivierung des proentzündlichen Transkriptionsfaktors NF- κB.

In Ulcerationen bei Patienten mit Morbus Crohn wurden adhärente *E*. *coli*-Stämme gefunden. Generell sind bei Patienten mit CED oder IBD wesentlich mehr Bakterien den intestinalen Epithelzellen unmittelbar anliegend als in der normalen Mukosa, die von einer Mukusschicht vor dem Kontakt mit Bakterien geschützt ist. Diese Beobachtung unterstützt die Hypothese von der Bedeutung der bakteriellen Translokation in der Pathogenese der CED.

Die heute verfügbaren Therapien zur Behandlung von Morbus Crohn und Colitis Ulcerosa können die Krankheitssymptome lindern, aber nicht heilen. Die meisten Therapien enden in einer Resistenz dem Antibiotikum gegenüber und dem operativen Eingriff.

JP 2007/077122 beschreibt die Anwendung von Pflanzen-Proanthocyanidinen wie beispielsweise aus Apfel, Birne, Aprikose, Traube, Guave, Hopfen, Gerste oder Adzuki Bohne für die Prävention von Darmentzündungen, speziell im Fall von Colitis ulcerosa. Die empfohlene tägliche Aufnahme beträgt 100 bis 2500 mg Apfelextrakt oder entsprechende Mengen Apfel-Proanthocyanidinen. Die Wirkung der Apfel-Proanthocyanidine wurde an Mäusen mit einer durch Dextransulfat (DSS, 2,5 %) verursachten akuten Colitis Ulcerosa in einem Zeitraum von 20 Tagen bestätigt.

Modelle der DSS-induzierten Colitis (akut oder chronisch) sind schnell, einfach durchführbar, gut reproduzierbar und preiswert. Sie erlauben die dynamische Studie des Entzündungsprozesses von der Entstehung bis zur Remission und sind somit für Untersuchungen der epithelialen Regeneration und Wundheilung sowie zum Arzneimittelscreening gut geeignet.

Die S3-Leitlinie "Diagnostik und Therapie des Morbus Crohn" fasst die Ergebnisse (zur Behandlung von vorbezeichneten Erkrankungen) einer Evidenz-basierten Konsensuskonferenz der Deutschen Gesellschaft für Verdauungs- und Stoffwechselkrankheiten mit dem Kompetenznetz Chronisch entzündliche Darmerkrankungen zusammen (Z Gastroenterol 2008; 46: 1094-1146). Unter anderem werden zur Therapie solcher Erkrankungen bisher Budesonid, systemisch wirksame Steroide, Sulfasalazin, Azathioprin/6-Mercaptopurin, Methotrexat und Anti-TNF-alpha-Antikörper eingesetzt.

Insgesamt besteht jedoch weiterhin Bedarf an geeigneten Anwendungen zur Prophylaxe und/oder Behandlung von Entzündungen.

Primäre Aufgabe der vorliegenden Erfindung war es daher, entsprechende Anwendungen bereitzustellen, insbesondere Anwendungen zur Prophylaxe und/oder Behandlung einer, mehrerer oder vorzugsweise sämtlicher der vorstehend beschriebenen Erkrankungen bzw. Symptome. Weitere sowie besonders bevorzugte Aufgabenstellungen der vorliegenden Erfindung ergeben sich aus der nachfolgenden Beschreibung, den Beispielen sowie den beigefügten Patentansprüchen.

Die primäre Aufgabe der vorliegenden Erfindung wird gelöst durch
- eine Verbindung der Formel (X) 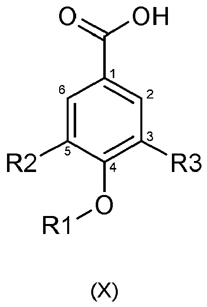 oder
- ein Salz einer Verbindung der Formel (X)
   oder
- eine Mischung umfassend oder bestehend aus
   zwei oder mehr unterschiedlichen Verbindungen der Formel (X),
   zwei oder mehr unterschiedlichen Salzen von Verbindungen der Formel (X)
   oder
   einer oder mehreren unterschiedlichen Verbindungen der Formel (X) und einem oder mehreren unterschiedlichen Salzen von Verbindungen der Formel (X),
   wobei für R1, R2 und R3 unabhängig voneinander in jeder Verbindung der Formel (X) gilt:
- R1 bedeutet Wasserstoff oder Methyl,
- R2 bedeutet einen organischen Rest mit 5 Kohlenstoffatomen und einem oder keinem Sauerstoffatom und
- R3 bedeutet einen organischen Rest mit 10 Kohlenstoffatomen und einem oder mehreren, vorzugsweise einem oder zwei, Sauerstoffatomen,
   oder
- R1 und R2 bilden zusammen mit den Kohlenstoffatomen in den Positionen 4 und 5 und dem an das Kohlenstoffatom in Position 4 gebundenen Sauerstoffatom einen Ring und umfassen 5 Kohlenstoffatome sowie ein oder kein Sauerstoffatom, und
- R3 bedeutet einen organischen Rest mit 10 Kohlenstoffatomen und einem oder mehreren, vorzugsweise einem oder zwei, Sauerstoffatomen,
   zur Anwendung in einem Verfahren zur Prophylaxe und/oder Behandlung von Entzündungen, insbesondere von Entzündungen der (menschlichen oder tierischen) Haut, insbesondere in einem Verfahren
- zum Reduzieren der Freisetzung von TNF-alpha, und/oder
- zum Reduzieren der Freisetzung eines Interleukins, vorzugsweise von IL-1, IL-6 und/oder IL-8, und/oder
- zum Reduzieren der Freisetzung eines Prostaglandins, vorzugsweise von PGE2, und/oder
- zum Reduzieren der Freisetzung von Interferon-gamma und/oder NF-κB, besonders bevorzugt in einem Verfahren
- zum Reduzieren der Freisetzung von TNF-alpha, und/oder
- zum Reduzieren der Freisetzung eines Interleukins, vorzugsweise von IL-1, IL-6 und/oder IL-8, und/oder
- zum Reduzieren der Freisetzung eines Prostaglandins, vorzugsweise von PGE2.

Grundsätzlich betrifft die vorliegende Erfindung also die vorstehend genannten Verbindungen, Salze bzw. Mischungen davon als anti-inflammatorische Wirkstoffe.

Für den Begriff "Haut" gilt dabei das oben Gesagte entsprechend. Bei der erfindungsgemäß zu behandelnden Haut handelt es sich also vorzugsweise um menschliche oder tierische äußere Haut (im herkömmlichen Sinne) und/oder eine Schleimhaut, insbesondere die Mundschleimhaut, die Magenschleimhaut und/oder die Darmschleimhaut, insbesondere zur Prophylaxe und/oder Behandlung einer oder mehrere der oben beschriebenen Erkrankungen bzw. Symptome.

Vorzugsweise ist das Verfahren zur Prophylaxe und/oder Behandlung von Entzündungen
(a) ein Verfahren zur Prophylaxe und/oder zum Behandeln von chronisch entzündlichen Erkrankungen, insbesondere Darmerkrankungen,
   und/oder
(b) ein Verfahren zum Stärken von geschädigter oder ungeschädigter Haut, insbesondere Schleimhaut,
   und/oder
(c) ein Verfahren zum Reduzieren von Gewebeschäden, insbesondere Gewebeschäden im Darm,
   und/oder
(d) ein Verfahren zum Wiederherstellen einer normalen zellulären Zusammensetzung im Darm,
   und/oder
(e) ein Verfahren zum Wiederherstellen oder Stabilisieren der Funktion von Haut, insbesondere von Schleimhaut.

Besonders vorteilhaft und daher erfindungsgemäß bevorzugt ist eine Anwendung wie oben beschrieben, wobei für die Gruppen R1, R2 und R3 in der Verbindung der Formel (X) bzw. unabhängig voneinander in einer, mehreren oder sämtlichen, vorzugsweise sämtlichen, Verbindung(en) der Formel (X) gilt:
R3 bedeutet 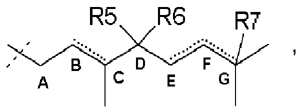
wobei
die gepunktete Linie, welche die mit B und C bezeichneten Kohlenstoffatome verbindet, bedeutet, dass zwischen diesen Kohlenstoffatomen eine Einfachbindung oder eine Doppelbindung vorhanden ist, und
die gepunktete Linie, welche die mit E und G bezeichneten Kohlenstoffatome verbindet,
eine einzelne Doppelbindung bedeutet, die entweder zwischen den mit F und G bezeichneten Kohlenstoffatomen oder zwischen den mit E und F bezeichneten Kohlenstoffatomen angeordnet ist,
R7, für den Fall, dass die Doppelbindung zwischen den mit E und F bezeichneten Kohlenstoffatomen angeordnet ist, eine Hydroxygruppe bedeutet oder, für den Fall, dass die Doppelbindung zwischen den mit F und G bezeichneten Kohlenstoffatomen angeordnet ist, entfällt,
R5 und R6 ein Wasserstoffatom und eine Hydroxygruppe bedeuten oder zusammen ein Sauerstoffatom bedeuten,
die gestrichelte Linie die Bindung markiert, die R3 mit dem Kohlenstoffatom in Position 3 verknüpft;
   - R1 bedeutet Wasserstoff oder Methyl und R2 bedeutet 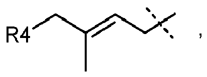 wobei R4 Wasserstoff oder eine Hydroxygruppe bedeutet und die gestrichelte Linie die Bindung markiert, die R2 mit dem Kohlenstoffatom in Position 5 verknüpft
      oder
   - R1 und R2 zusammen bedeuten 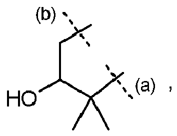
wobei die gestrichelte Linie (a) die Bindung markiert, die das tertiäre Kohlenstoffatom mit dem an das Kohlenstoffatom in Position 4 gebundenen Sauerstoffatom verknüpft und die gestrichelte Linie (b) die Bindung markiert, die das sekundäre Kohlenstoffatom mit dem Kohlenstoffatom in Position 5 verknüpft.

Besonders bevorzugt ist eine erfindungsgemäße Anwendung (wie oben beschrieben), wobei die bzw. eine, mehrere oder sämtliche Verbindung(en) der Formel (X) ausgewählt ist bzw. jeweils unabhängig voneinander ausgewählt sind aus der Gruppe bestehend aus den folgenden Verbindungen **(1)** bis **(10)**:

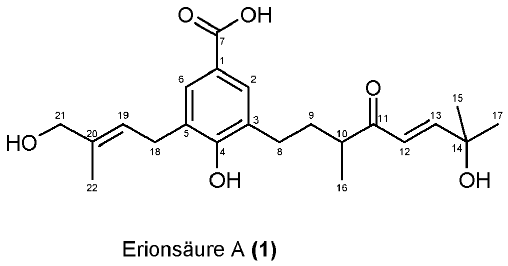

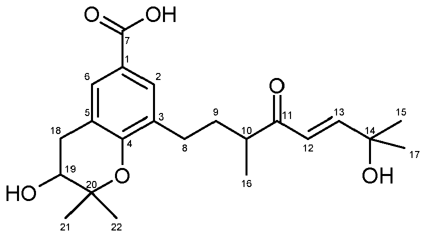

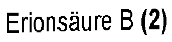

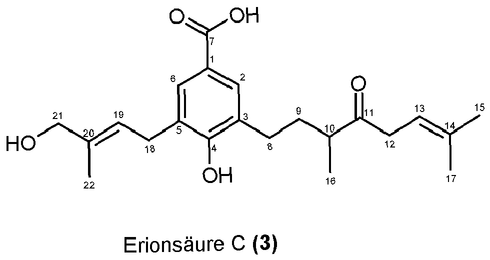

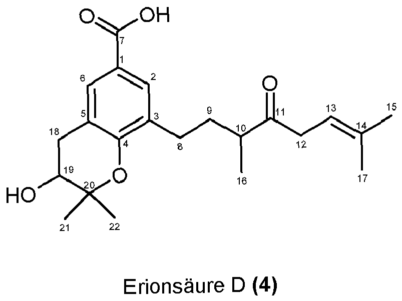

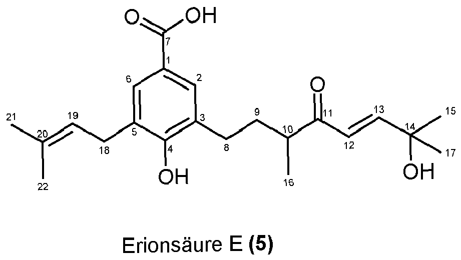

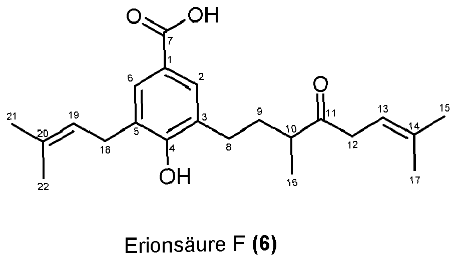

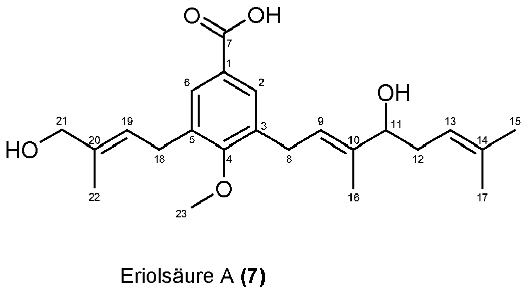

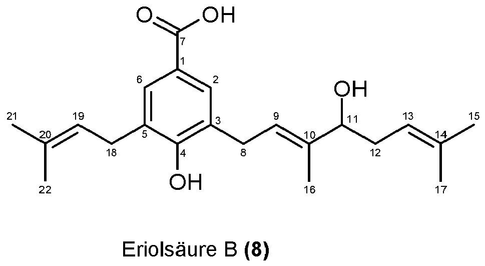

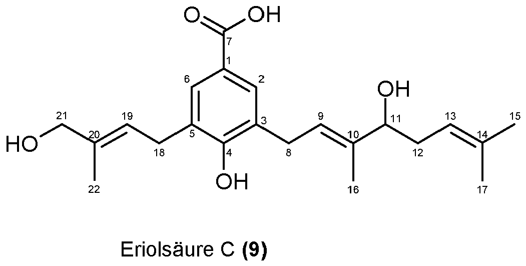

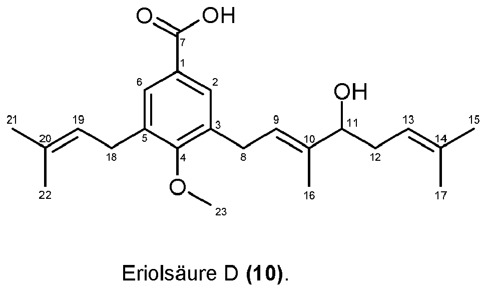

**Erionsäure A** entspricht: 4-Hydroxy-3-((*E*)-7-hydroxy-3,7-dimethyl-4-oxo-oct-5-enyl)-5-((*E*)-4-hydroxy-3-methyl-but-2-enyl)-benzoesäure **(1)**

**Erionsäure B** entspricht: 3-Hydroxy-8-((*E*)-7-hydroxy-3,7-dimethyl-4-oxo-oct-5-enyl)-2,2-dimethyl-chroman-6-carbonsäure **(2)**

**Erionsäure C** entspricht: 3-(3,7-Dimethyl-4-oxo-oct-6-enyl)-4-hydroxy-5-((*E*)-4-hydroxy-3-methyl-but-2-enyl)-benzoesäure **(3)**

**Erionsäure D** entspricht: 8-((*E*)-3,7-Dimethyl-4-oxo-oct-5-enyl)-3-hydroxy-2,2-dimethyl-chroman-6-carbonsäure **(4)**

**Erionsäure E** entspricht: 4-Hydroxy-3-((*E*)-7-hydroxy-3,7-dimethyl-4-oxo-oct-5-enyl)-5-(3-methyl-but-2-enyl)-benzoesäure **(5)**

**Erionsäure F entspricht:** 3-(3,7-Dimethyl-4-oxo-oct-6-enyl)-4-hydroxy-5-(3-methyl-but-2-enyl)-benzoesäure **(6)**

**Eriolsäure A** entspricht: 3-((*E*)-4-Hydroxy-3,7-dimethyl-octa-2,6-dienyl)-5-((E)-4-hydroxy-3-methyl-but-2-enyl)-4-methoxy-benzoesäure **(7)**

**Eriolsäure B** entspricht: 4-Hydroxy-3-((*E*)-4-hydroxy-3,7-dimethyl-octa-2,6-dienyl)-5-(3-methyl-but-2-enyl)-benzoesäure **(8)**

**Eriolsäure C** entspricht: 4-Hydroxy-3-((*E*)-4-hydroxy-3,7-dimethyl-octa-2,6-dienyl)-5-((*E*)-4-hydroxy-3-methyl-but-2-enyl)-benzoesäure **(9)**

**Eriolsäure D** entspricht: 3-((*E*)-4-Hydroxy-3,7-dimethyl-octa-2,6-dienyl)-4-methoxy-5-(3-methyl-but-2-enyl)-benzoesäure **(10).**

Die erfindungsgemäß anzuwendenden Benzoesäuren der Formel (X) können ein oder mehrere asymmetrische Kohlenstoffatome umfassen. Diese können jeweils in (R)- oder (S)-Konfiguration vorliegen. Diese Stereoisomere können als Enantiomere, Diastereomere oder Epimere vorliegen, insbesondere als (R)-, (S)-, (R,R)-, (R,S)-, (S,R)- oder (S,S)-konfigurierte Verbindungen oder als beliebiges Gemisch dieser Verbindungen, beispiels**weise** als Racemat, oder auch als beliebiges Gemisch der entsprechenden Diastereomere.

Erfindungsgemäß besonders bevorzugt ist eine Mischung (wie oben beschrieben), umfassend oder bestehend aus zwei oder mehr unterschiedlichen Verbindungen der Formel (X), vorzugsweise aus zwei, drei, vier, fünf, sechs, sieben, acht, neun oder zehn unterschiedlichen Verbindungen der Formel (X), vorzugsweise ausgewählt aus der Gruppe bestehend aus den Verbindungen **(1)** bis **(10)**.

Die vorstehend genannten Verbindungen **(7)** und **(9)** sind zwei in Herba Santa identifizierte, kommerziell erhältliche Verbindungen (beispielsweise angeboten von der Firma Ambinter). Hinweise auf eine anti-inflammatorische Wirkung dieser Verbindungen sind im Stand der Technik jedoch nicht bekannt. Es war besonders überraschend, dass sich die erfindungsgemäß anzuwendenden Verbindungen der Formel (X) aus Herba Santa besonders gut zur Verwendung als anti-inflammatorische Wirkstoffe eignen.

Herba Santa (auch Yerba Santa, mountain balm) bezeichnet allgemein *Eriodyction ssp.,* insbesondere *Eriodictyon californicum* (H. & A.) Torr sowie *Eriodictyon angustifolium* (aus der Familie der *Hydrophyllaceen*). Herba Santa-Kraut wird schon seit langer Zeit auf Grund seiner medizinischen Wirkung als Heilpflanze verwendet. Traditionell wurden die Pflanzen, die ursprünglich in Mexiko und dem Westen der USA vorkommen, von amerikanischen Ureinwohnern und später von spanischen Siedlern benutzt (Heinsen, 1972; Munz, 1973; Barrett und Gifford, 1933; Immel, 2006). Die antibakterielle Wirkung von Extrakten aus *Eriodictyon californicum* wurde von Salle et al. 1951 beschrieben (Arch. Biochem. Biophys. 1951, 32, 121-123). Zu den Hauptinhaltsstoffen von Eriodictyon sp. zählen verschiedene Flavanone, unter anderem Homoeriodictyol, Hesperetin, Sterubin, Chrysoeriol und Luteolin (Hadleyy and Gisvold, 1942; Ley et al., J. Agric. Food Chem., 2005). Die unterschiedlichen biologischen Wirkungen von Herba Santa wurden bisher hauptsächlich den enthaltenen Flavanonen zugeschrieben, deren Zusammensetzung und Strukturen bereits untersucht wurden. Über die Eigenschaften der Bestandteile aus Herba Santa, die keine Flavanoidstruktur aufweisen ist bisher kaum etwas in der Literatur bekannt. Insbesondere ist nichts über eine anti-inflammatorische Wirkung der erfindungemäß anzuwendenden Verbindungen bekannt. Die auch in verschiedenen *Eriodictyon* sp. vorkommenden Verbindungen **(1)** bis **(10)** wurden erst kürzlich erstmals beschrieben. Ihre Fähigkeiten als Antioxidationsmittel werden in DE 10 2009 020729 A1 dargelegt. Über (zusätzliche) anti-inflammatorische Eigenschaften war bisher jedoch nichts bekannt. Erfindungsgemäß besonders bevorzugt ist eine Mischung zur Anwendung in einem Verfahren zur Prophylaxe und/oder Behandlung von Entzündungen (wie oben beschrieben), umfassend eine Verbindung der Formel (X), ein Salz der Formel (X) oder eine Mischung davon (wie jeweils oben beschrieben) sowie zudem
- ein Hydroxyflavon der Formel (Y) 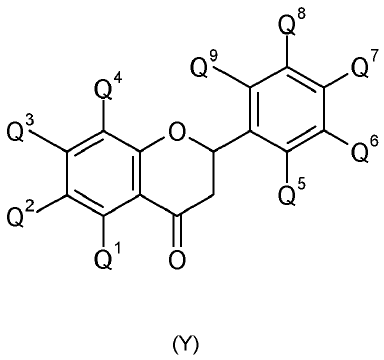 oder
- ein Salz eines Hydroxyflavons der Formel (Y) oder
- eine Mischung umfassend oder bestehend aus zwei oder mehr unterschiedlichen Hydroxyflavonen der Formel (Y), zwei oder mehr unterschiedlichen Salzen von Hydroxyflavonen der Formel (Y) oder einem oder mehreren unterschiedlichen Hydroxyflavonen der Formel (Y) und einem oder mehreren unterschiedlichen Salzen von Hydroxyflavonen der Formel (Y),
   wobei für Q1, Q2, Q3, Q4, Q5, Q6, Q7, Q8 und Q9 unabhängig voneinander in jedem Hydroxyflavon der Formel (Y) gilt:
   Q1 bis Q9 bedeuten unabhängig voneinander Wasserstoffatome, Hydroxygruppen, Methyl-, Ethyl-, 1-Propyl-, Methoxy-, Ethoxy-, 1-Propyloxy- oder 2-Propyloxygruppen, mit der Maßgabe, dass mindestens einer der Reste Q1 bis Q9 eine Hydroxygruppe darstellt,
      und wobei vorzugsweise gilt:
      Q2, Q4, Q5, Q8 und Q9 stellen Wasserstoffatome dar,
      Q1, Q3 und Q6 bedeuten unabhängig voneinander Wasserstoffatome, Hydroxy- oder Methoxygruppen, mit der Maßgabe, dass mindestens einer der Reste Q1 und Q3 eine Hydroxygruppe darstellt,
      und
      Q7 stellt eine Hydroxygruppe dar.

Die Hydroxyflavone der Formel (Y) können als ein- oder (im Falle mehrerer Hydroxygruppen) mehrwertige Anionen vorliegen, wobei als Gegenkationen die einfach positiv geladenen Kationen der ersten Haupt- und Nebengruppe, das Ammoniumion, ein Triaalkylammoniumion, die zweiwertiggeladenen Kationen der zweiten Haupt- und Nebengruppe, sowie die dreiwertigen Kationen der 3. Haupt- und Nebengruppe dienen, bevorzugt, Na⁺, K⁺, NH⁴⁺, Ca²⁺, Mg²⁺, Al³⁺ und Zn²⁺.

Die Hydroxyflavone der Formel (Y) können als (2S)- oder (2R)-Enantiomer oder als Mischung der beiden vorliegen. Bevorzugt liegen die Hydroxyflavone der Formel (Y) als (2S)-Enantiomer oder als an (2S)-Enantiomer angereichertes Gemisch vor.

Ohne die Erfindung damit einzuschränken, seien als beispielhafte Verbindungen genannt: 2-(4-Hydroxyphenyl)-5,7-dihydroxychroman-4-on (Naringenin), 2-(3,4-Dihydroxyphenyl)-5,7-dihydroxychroman-4-on (Eriodictyol), 2-(3,4-Dihydroxyphenyl)-5-hydroxy-7-methoxychroman-4-on (Eriodictyol-7-methylether), 2-(3,4-Dihydroxyphenyl)-7-hydroxy-5-methoxychroman-4-on (Eriodictyol-5-methylether), 2-(4-Hydroxy-3-methoxyphenyl)-5,7-dihydroxychroman-4-on (Homoeriodictyol) und 2-(3-Hydroxy-4-methoxyphenyl)-5,7-dihydroxychroman-4-on (Hesperetin) deren (2S)- oder (2R)-Enantiomere oder Gemische derselben, sowie deren ein- oder mehrwertige Phenolatsalze mit Na⁺, K⁺, NH⁴⁺, Ca²⁺, Mg²⁺ oder Al³⁺ als Gegenkationen.

Im Folgenden sind die Strukturen bevorzugter Beispiele von Hydroxyflavonen der Formel (Y) gezeigt (s. Verbindungen **(11)** bis **(16))**:

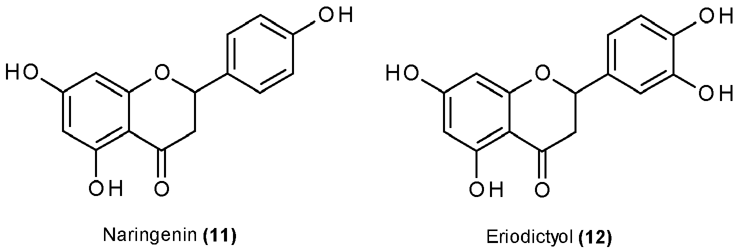

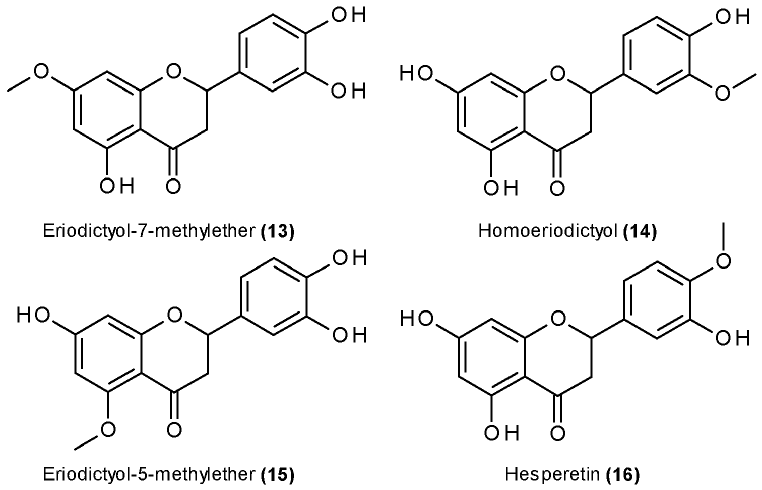

Besonders bevorzugt ist eine Mischung (wie oben beschrieben), umfassend eine, mehrere oder sämtliche Verbindungen der Formel (Y) ausgewählt aus der Gruppe bestehend aus Homoeriodictyol, Sterubin, Eriodictyol, Hesperetin, Chrysoeriol und Luteolin.

Besonders bevorzugt umfasst eine solche Mischung zumindest Homoeriodictyol als Verbindung der Formel (Y).

Gemäß einer bevorzugten Ausgestaltung der vorliegenden Erfindung wir eine Mischung (wie oben beschrieben) zur Anwendung bereitgestellt, wobei
- der Anteil der Gesamtmenge an Verbindungen der Formel (X) und Salzen von Verbindungen der Formel (X) in der Mischung, bezogen auf das Gesamtgewicht der Mischung, 1 bis 99 Gew.-%, vorzugsweise 10 bis 99 Gew.-%, besonders bevorzugt 20 bis 80 Gew.-%, beträgt,
   und/oder
- der Anteil der Gesamtmenge an Verbindungen der Formel (Y) und Salzen von Verbindungen der Formel (Y) in der Mischung, bezogen auf das Gesamtgewicht der Mischung, 1 bis 99 Gew.-%, vorzugsweise 10 bis 99 Gew.-%, besonders bevorzugt 20 bis 80 Gew.-%, beträgt,
vorzugsweise wobei der Anteil der Gesamtmenge an Verbindungen der Formel (X), Verbindungen der Fomel (Y), Salzen von Verbindungen der Formel (X) und Salzen von Verbindungen der Formel (Y) in der Mischung, bezogen auf das Gesamtgewicht der Mischung, 0,0001 bis 100 Gew.-%, vorzugsweise 0,001 bis 100 Gew.-%, besonders bevorzugt 0,1 bis 90 Gew.-%, weiter bevorzugt 1 bis 90 Gew.-% beträgt. Gemäß einer besonders bevorzugten Ausgestaltung beträgt der Anteil 10 bis 90 Gew.-%, insbesondere 25 bis 90 Gew.-% (bevorzugt bis 100 Gew.-%), weiter bevorzugt 45 bis 90 Gew.-% (bevorzugt bis 100 Gew.-%).

Überraschenderweise hat sich in eigenen Untersuchungen gezeigt, dass bestimmte aus Herba Santa-Kraut hergestellte Extrakt oder Fraktionen davon besonders dazu geeignet sind, entzündliche Vorgänge, wie z.B. bei Entzündungen im Gastrointestinaltrakt oder Gingivitis, zu behandeln und/oder solchen vorzubeugen. Gemäß einem weiteren Aspekt der vorliegenden Erfindung wird daher eine Mischung zur erfindungsgemäß beschriebenen Anwendung angegeben, wobei die Mischung einen pflanzlichen Extrakt umfasst oder daraus besteht, vorzugsweise einen Extrakt aus *Eriodictyon ssp.,* besonders bevorzugt einen Extrakt aus *Eriodictyon californicum* und/oder *Eriodictyon angustifolium,* wobei der Anteil der Gesamtmenge an Verbindungen der Formel (X) und Salzen von Verbindungen der Formel (X) in der Mischung, bezogen auf das Gesamtgewicht der Mischung, vorzugsweise 0,1 bis 100 Gew.-%, weiter bevorzugt 1 bis 100 Gew.-%, besonders bevorzugt 10 bis 100 Gew.-%, weiter bevorzugt von 10 bis 90 Gew.-% beträgt.

Besonders bevorzugt liegt das Verhältnis der Gesamtmenge an Verbindungen der Formel (X) und Salzen von Verbindungen der Formel (X) zu der Gesamtmenge an Verbindungen der Formel (Y) und Salzen von Verbindungen der Formel (Y) in einer hierin beschriebenen erfindungsgemäß anwendbaren Mischung im Bereich von 0,00001 : 1 bis 1 : 0,00001, insbesondere im Bereich von 0,0001 : 1 bis 1 : 0,0001, bevorzugt im Bereich von 0,001 : 1 bis 1 : 0,001, vorzugsweise im Bereich von 0,01 : 1 bis 1 : 0,01, besonders bevorzugt im Bereich von 0,1 : 1 bis 1 : 0,1, weiter bevorzugt im Bereich von 0,5 : 1 bis 1 : 0,5, jeweils bezogen auf das Gewicht.

Gemäß einer bevorzugten Ausgestaltung der vorliegenden Erfindung umfasst eine hierin beschriebene Mischung als Verbindungen der Formel (Y) Homoeriodictyol und Sterubin bzw. Salz(e) davon. Dabei gilt hinsichtlich bevorzugter Mengenangaben und - verhältnisse das oben Gesagte entsprechend.

In einer bevorzugten Ausgestaltung einer solchen Mischung umfasst die Mischung eine Gesamtmenge an Homoeriodictyol und Sterubin (und/oder Salzen davon), die größer ist als die Gesamtmenge an Verbindungen der Formel (X) (sowie gegebenenfalls Salzen davon).

Die vorliegende Erfindung betrifft auch Zubereitungen, insbesondere der Ernährung oder dem Genuss dienende Zubereitungen, pharmazeutische Zubereitungen, kosmetische Zubereitungen oder dermatologische Zubereitungen zur Anwendung in einem Verfahren zur Prophylaxe und/oder Behandlung von Entzündungen, umfassend eine Verbindung, ein Salz oder eine Mischung wie oben definiert, zur Anwendung in einem Verfahren zur Prophylaxe und/oder Behandlung von Entzündungen.

Bevorzugt handelt es sich dabei um Zubereitungen zur Prophylaxe und/oder Behandlung von Entzündungen der Haut (wie oben beschrieben), insbesondere in einem Verfahren
- zum Reduzieren der Freisetzung von TNF-alpha, und/oder
- zum Reduzieren der Freisetzung eines Interleukins, vorzugsweise von IL-1, IL-6 und/oder IL-8, und/oder
- zum Reduzieren der Freisetzung eines Prostaglandins, vorzugsweise von PGE2, und/oder
- zum Reduzieren der Freisetzung von Interferon-gamma und/oder NF-κB,
   besonders bevorzugt in einem Verfahren
- zum Reduzieren der Freisetzung von TNF-alpha, und/oder
- zum Reduzieren der Freisetzung eines Interleukins, vorzugsweise von IL-1, IL-6 und/oder IL-8, und/oder
- zum Reduzieren der Freisetzung eines Prostaglandins, vorzugsweise von PGE2.

Vorzugsweise handelt sich dabei auch hier um
(a) ein Verfahren zur Prophylaxe und/oder zum Behandeln von chronisch entzündlichen Erkrankungen, insbesondere Darmerkrankungen,
   und/oder
(b) ein Verfahren zum Stärken von geschädigter oder ungeschädigter Haut, insbesondere Schleimhaut,
   und/oder
(c) ein Verfahren zum Reduzieren von Gewebeschäden, insbesondere Gewebeschäden im Darm,
   und/oder
(d) ein Verfahren zum Wiederherstellen einer normalen zellulären Zusammensetzung im Darm,
   und/oder
(e) ein Verfahren zum Wiederherstellen oder Stabilisieren der Funktion von Haut, insbesondere von Schleimhaut.

Auch im Übrigen gilt das oben Gesagte entsprechend, insbesondere hinsichtlich der enthaltenen Verbindungen der Formel (X) bzw. Salzen davon sowie der gegebenenfalls enthaltenen Verbindungen der Formel (Y) bzw. Salzen davon.

Bevorzugt enthält eine vorstehend beschriebene Zubereitung eine erfindungsgemäß bevorzugte Mischung (wie oben beschrieben).

Eine erfindungsgemäße Mischung bzw. eine erfindungsgemäß anwendbare Mischung, vorzugsweise eine oben als bevorzugt bezeichnete Mischung, ist vorzugsweise herstellbar durch ein Verfahren mit folgenden Schritten:
(a) Extrahieren von Pflanzenmaterial aus *Eriodictyon ssp.,* bevorzugt aus *Eriodictyon californicum* und/oder *Eriodictyon angustifolium,* so dass eine Mischung entsteht, die Verbindungen der Formel (X), gegebenenfalls Verbindungen der Formel (Y) sowie sonstige extrahierte Verbindungen umfasst, und
(b) Anreichern von extrahierten Verbindungen der Formel (X) und/oder Salzen der extrahierten Verbindungen der Formel (X) sowie gegebenenfalls Verbindungen der Formel (Y) und/oder Salzen der extrahierten Verbindungen der Formel (Y) in der Mischung durch teilweises oder vollständiges Entfernen sonstiger extrahierter Verbindungen und gegebenenfalls Entfernen von Extraktions- und/oder Lösemitteln,
vorzugsweise so dass der Anteil der Gesamtmenge an Verbindungen der Formel (X) und Salzen von Verbindungen der Formel (X) in der Mischung bezogen auf das Gesamtgewicht der Mischung 0,1 bis 100 Gew.-%, weiter bevorzugt 1 bis 100 Gew.-%, besonders bevorzugt 10 bis 100 Gew.-%, weiter bevorzugt von 10 bis 90 Gew.-% beträgt.

Vorzugsweise ist das Pflanzenmaterial dabei ausgewählt aus der Gruppe bestehend aus:
- *Eriodictyon altissimum P.V. Wells* - *Indian Knob mountain balm*
- *Eriodictyon angustifolium* Nutt. - Narrow-leaved Yerba Santa
- *Eriodictyon californicum* (Hook. & Arn.) Torr.- California Yerba Santa
- *Eriodictyon capitatum* Eastw. - Lompoc Yerba Santa
- *Eriodictyon crassifolium* Benth.- Thick-Leaved Yerba Santa
- *Eriodictyon tomentosum* Benth
- *Eriodictyon traskiae*
- *Eriodictyon trichocalyx* (Syn.: *Eriodictyon lanatum* (Brand) Abrams) - Hairy Yerba Santa

Gemäß einem bevorzugten Aspekt der vorliegenden Erfindung umfasst oder besteht eine hierin beschriebene Mischung oder eine hierin beschriebene Zubereitung aus einem entsprechend angereicherten Extrakt aus Herba santa, vorzugsweise aus Pflanzenmaterial wie vorstehend beschrieben.

Im Rahmen der vorliegenden Erfindung ist ein Extrakt aus frischen oder getrockneten Herba Santa-Pflanzen oder -Pflanzenteilen bevorzugt, besonders bevorzugt aus Pflanzen bzw. Pflanzenteilen mit einem Feststoffgehalt von 90 Gew.-% oder mehr. Besonders bevorzugt ist der Extrakt aus oberirdischen Pflanzenteilen, insbesondere aus Blättern, Knospen, Stängeln, Rinden, Blüten und/oder Früchten von *E. angustifolium* oder *E*. *californicum.*

Extrakte aus Herba Santa-Kraut (wie oben beschrieben) können durch an sich bekannte Extraktionsverfahren aus dem frischen oder getrockneten Kraut der Pflanzen gewonnen werden. Hierzu zählen z.B. die Mazeration oder Perkolation. Als Extraktionsmedium kann z. B Wasser und Ethanol bzw. Mischungen daraus verwendet werden. Anstelle von Ethanol können auch Methanol und andere wasserlösliche Lösungsmittel verwendet werden. Ebenso kann Ethylacetat als Lösungsmittel verwendet werden. Die Temperaturwahl und mechanische Desintegration der Früchte können die Extraktion unterstützen. Nach dem Stand der Technik empfiehlt sich auch die mechanische Desintegration des getrockneten Krautes z.B. durch Rührer, Homogenisatoren oder Ultraschall. Ferner können weitere extraktionsfördernde Stoffe, wie Säuren, Basen und Enzyme eingesetzt werden.

Der Begriff "Herba Santa-Kraut" umfasst im Rahmen des vorliegenden Textes insbesondere Blätter, Knospen, Rinden, Blüten, Früchten und Stängel von *Eriodictyon angustifolium, E. californicum, E. trichocalyx, E. traskiae, sowie E. crassifolium.*

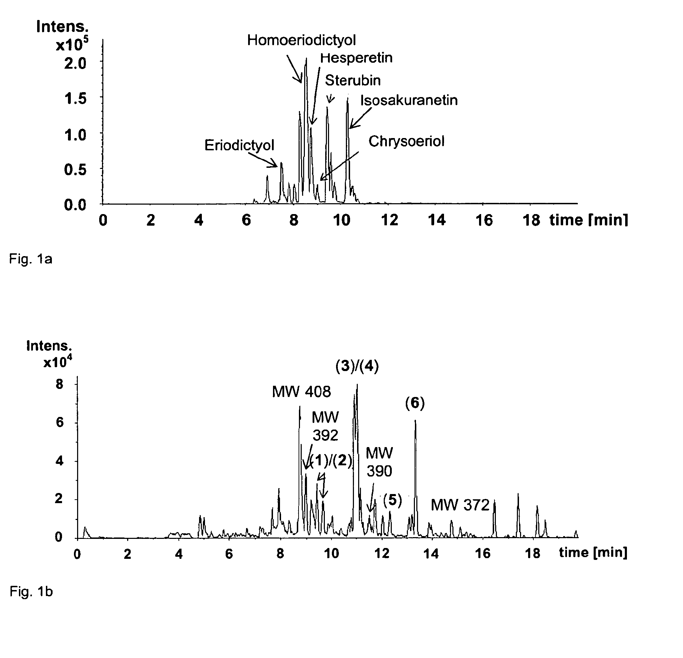

Die Identifizierung und Quantifizierung von Flavanonen und bisprenylierten Benzoesäure-Derivaten in verschiedenen *Eriodictyon sp.* kann, wie im Rahmen eigener Untersuchungen durchgeführt, nach der methanolischen Extraktion mittels RP-HPLC-UV/Vis und RP-HPLC-MS/MS vorgenommen werden. Die Flavonoidgehalte, bzw. Gehalte an bisprenylierten Benzoesäure-Derivaten können mittels externer Kalibrierung unter Verwendung von Standardsubstanzen ermittelt werden. Die beigefügten Figuren 1 a und 1 b zeigen die Flavonoid- bzw. Erion-/Eriolsäureprofile beispielhaft untersuchter Extrakte (Abb. 1a: LC-MS Chromatogramm der Flavonoid-Fraktion aus Eriodicyton angustifolium Extrakt; Abb. 1 b: LC-MS Chromatogramm der Erionsäure-Fraktion aus *Eriodicyton angustifolium-*Extrakt; jeweils nach Fraktionierung über Sephadex LH-20).

In Anbetracht der vorliegenden Ausführungen umfasst oder besteht eine erfindungsgemäße Zubereitung oder eine erfindungsgemäße Mischung (wie jeweils oben beschrieben) vorzugsweise aus (i) Herba Santa-Kraut, (ii) einem daraus hergestellten, gegebenenfalls angereicherten Extrakt oder (iii) einer Fraktion davon.

In den erfindungsgemäßen Mischungen oder Zubereitungen können neben den oben beschriebenen Bestandteilen eine oder mehrere weitere Substanzen enthalten sein. Vorzugsweise sind eine oder mehrere (weitere) Substanzen, die zur Beeinflussung von entzündlichen Zuständen der Haut, insbesondere für prophylaktische und/oder therapeutische Anwendungen wie oben beschrieben, geeignet sind, enthalten. Weiterhin können eine oder mehrere Substanzen zur Behandlung einer bei Entzündungen der Haut auftretenden Mangelerscheinung (insbesondere ein Mangel an Kalium, Natrium, Eisen, Calcium, Vitamin D und/oder Folsäure) enthalten sein.

In den erfindungsgemäßen Mischungen oder Zubereitungen sind vorzugsweise (auch) ein oder mehrere weitere Bestandteile ausgewählt aus folgender Gruppe enthalten: probiotische Bakterien (z.B. Lactobacillen, Bifidobakterien, Enterokokken), Präbiotika (z.B. Inulin, Fructooligosaccharide), Synbiotika (Pro- und Präbiotika), Ballaststoffe (z.B. Cellulose, Stärke, resistente Stärke, Fasern, wie beispielsweise Apfelfasern), Molkenproteine, Sojaproteine, Mineralstoffe (insbesondere Ca, Mg, wobei eine Kombination von Ca, Mg und Inulin besonders bevorzugt ist), Tocopherole (z.B. Vitamin E, Vitamin-E-acteat), Vanille, Vanille-Extrakte, Omega-3-Fettsäuren (vorzugsweise Fischöl), Citrus-, Apfel-, Traubenkern-, Grüntee-, Rosmarin-, Estragon-, Thymian-, Meerrettich- und Macis-Extrakte, Tannine, Tomaten-, Melonen- und Hagebutten-Extrakte (insbesondere Lycopinhaltige Extrakte), beta-Carotin; Aubergine, Rhabarber, rote Zwiebel, Rotkohl, Schwarzkarotte, Superfruits, insbesondere Açai, Noni, Goji, Granatapfel, Mangosteen, Johannisbeeren, Erdbeeren, Aronia, Blaubeeren und/oder Holunderbeeren, bevorzugt in Form von getrockneten Früchten, Extrakten oder Fruchtzbereitungen; Soja-Isoflavone, nichtsteroidale Antiphlogistika, Antibiotika, Budesonid, systemisch wirksame Steroide, Sulfasalazin, Azathioprin/6-Mercaptopurin, Methotrexat, anti-TNF-alpha-Antikörper, Bisabolol, Natriumlaurylsulfat, Chlorhexidin, Metall-Fluoride (z.B. Aluminium- und Zinn-Fluorid), organische und anorganische Fluoride, Aromastoffe, ätherische Öle, Kühlwirkstoffe, insbesondere Menthol, Extrakte oder Reinstoffe aus Eukalyptus, Thymian, Wintergrün, Spearmint und Pfefferminze.

Erfindungsgemäße Zubereitungen (insbesondere die vorstehend als bevorzugt bezeichneten Zubereitungen) sind vorzugsweise ausgewählt aus der Gruppe bestehend aus:
fruchtsafthaltige Getränke; gemüsesafthaltige Getränke; Backwaren; Süßwaren; Snacks; Instantprodukte; Suppen; Saucen; Würzmischungen; Speiseeis; Fruchtzubereitungen; Desserts; Milchprodukte; Sojaprodukte; Cerealien; Nahrungsergänzungsmittel, Medizinprodukte und pharmazeutische Produkte.

Fruchtsafthaltige Getränke sind dabei insbesondere Fruchtsäfte und Smoothies (Vollfruchtgetränke). Gemüsesafthaltige Getränke sind insbesondere Säfte von Rote Beete und Schwarzkarotte.

Backwaren sind insbesondere Kuchen, Waffeln und Kekse.

Süßwaren sind insbesondere Lutschbonbons, Kaugummis, Fruchtgummis, Kaubonbons, (Erfrischungs-)Dragees, Komprimate, Hartkaramellen, Schokocremes, Konfekt und Schokolade.

Instantprodukte sind insbesondere Instantmahlzeiten sowie andere Instantprodukte, z. B. Getränkepulver und -granulate.

Fruchtzubereitungen sind insbesondere Marmelade, Konfitüre, Fruchtsoßen.

Desserts sind insbesondere Puddings und Götterspeise.

Milchprodukte umfassen insbesondere Quark, Yoghurt, Milchdrinks, Molkezubereitungen.

Cerealien sind insbesondere Cornflakes, Müsli und Müsliriegel.

Weitere bevorzugte Zubereitungen, insbesondere Nahrungsergänzungsmittel, Medizinprodukte und pharmazeutische Produkte, sind
feste galenische Formen (wie z.B. Tabletten (mit Überzug und ohne, mit modifizierter Freisetzung und ohne), Dragees (mit Überzug und ohne, mit modifizierter Freisetzung und ohne), Kapseln (Weich- oder Hartgelatinekapseln mit modifizierter Freisetzung und ohne) Granulate (mit modifizierter Freisetzung und ohne), Pulver (mit modifizierter Freisetzung und ohne), Suppositorien (mit Überzug und ohne, mit modifizierter Freisetzung und ohne) Lutschbonbons, Kaugummis),
flüssige Formen (wie z.B. Lösungen, Suspensionen, Emulsionen, Sirupe (umgangssprachlich Hustensaft), Mundspülungen ,Gurgellösungen, Halssprays oder Nasensprays, Nasentropfen, Nasenspüllösungen, Nasenpulver, Nasensalben oder Ohrentropfen, Ohrensprays, Ohrenspüllösungen, Ohrenpuder, Ohrentampons),
halbfeste Formen (wie z.B. Hydrophobe Salben darunter z.B.: Kohlenwasserstoffgele, Lipogele. Silikongele, Oleogele. sowie wasseraufnehmende Salben darunter z.B. Absorptionsbasen, hydrophile Salben, hydrophile Gele (Hydrogele) oder Pasten,
Inhalate (wie z.B. Druckgas-Dosierinhalatoren, Pulver-Inhalatoren, Inhalatoren mit Zerstäuber, Inhalationskonzentrate zur Inhalation),
wirkstoffhaltige Pflaster oder andere therapeutische Systeme und
kosmetische und/oder dermatologische Zubereitungen, die (abgesehen von den erfindungsgemäß zu verwendenden Substanzen) wie üblich zusammengesetzt sind und dem kosmetischen, insbesondere dermatologischen Lichtschutz, zur Behandlung, der Pflege und der Reinigung der Haut und/oder der Haare oder als Schminkprodukt in der dekorativen Kosmetik dienen. Entsprechend können derartige Zubereitungen z. B. als Reinigungsmittel wie z. B. Seife, Syndet, flüssige Wasch-, Dusch-, und Badepräparat, Hautpflegemittel wie z. B. Emulsion (als Lösung, Dispersion, Suspension; Creme, Lotion oder Milch je nach Herstellungsverfahren und Inhaltsstoffen vom Typ W/O, O/W oder multiple Emulsion, PIT Emulsion, Emulsionsschaum, Mikro-, Nanoemulsion, Pickering Emulsion), Salbe, Paste, Gel (inklusive Hydro-, Hydrodispersions-, Oleogel), alkoholische oder wässrig/alkoholische Lösung, Öl, Toner, Balsam, Serum, Puder, Wipe, Eau de Toilette, Eau de Cologne, Perfum, Wachs, inklusive der Darreichungsform als Stift, Roll-On, (Pump-)Spray, Aerosol (schäumend, nicht schäumend oder nach-schäumend), Hautpflegemittel (wie oben beschrieben) als Fusspflegemittel (inklusive Keratolytika, Desodorant), als Insekten abwehrendes Mittel, als Sonnenschutzmittel, als Selbstbräunungsmittel und / oder Aftersun-Präparat, Hautpflegemittel als Rasiermittel oder After-Shave, als Haarentfernungsmittel, als Haarpflegemittel wie z.B. Shampoo (inklusive Shampoo für normales Haar, für schnellfettendes Haar, für trockenes, strapaziertes (geschädigtes) Haar, 2-in-1 Shampoo, Anti-Schuppen-shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (z.B. Gel oder Wachs); Blondiermittel, Haarfärbemittel wie z.B. temporäre, direktziehende, semipermanente Haarfärbemittel, permanente Haarfärbemittel), Hautpflegemittel als dekorative Körperpflegemittel, wie z. B. Nagelpflegemittel (Nagellack und Nagellackentferner), dekorative Kosmetik (z.B. Puder, Lidschatten, Kajalstift, Lippenstift), Hautpflegemittel als Deodorant und / oder Antitranspirant; Mundwasser und Munddusche vorliegen, und

Mundpflegeprodukte (z.B. Zahnpasta, Zahncreme, Zahngel, Zahnpulver, Zahnreinigungsflüssigkeit, -schaum, Mundwasser, Zahncreme und Mundwasser als 2-in-1 Produkt, Mundspray, Zahnseide oder Zahnpflegekaugummi). Solche Mund-, bzw. Zahnpflegeprodukte enthalten in der Regel abrasive Systeme (abrasive oder polierende Inhaltsstoffe), wie Silicate, Calciumcarbonate, Calciumphosphate, Aluminiumoxide und/oder Hydroxyapatite, Oberflächenaktive Substanzen, z.B. Sodiumlaurylsulfat, Sodiumlaurylsarcosinat und/oder Cocamidopropylbetain, Feuchthaltemittel, wie Glycerol und/oder Sorbitol, Verdickungsmittel, z.B. Carboxymethylcellulose, Polyethyleneglycole, Carrageenan und/oder Laponite^{®}, Süßstoffe, wie Saccharin, Aroma-/ Geschmackskorrigenzien für unangenehme Geschmackseindrücke, Geschmacksmodulierende Substanzen (z.B. Inositolphosphat, Nucleotide, z.B. Guanosinmonophosphat, Adenosinmonophosphat oder andere Substanzen, z.B. Sodiumglutamat oder 2-Phenoxypropionsäure), Kühlwirkstoffe, z.B. Mentholderivate (z.B. L-Menthyllactat, L-menthylalkylcarbonate, Menthonketale), Icilin und Icilin-Derivate, Stabilisatoren und Wirkstoffe, z.B. Natriumfluorid, Natriummonofluorophosphat, Zinndifluorid, quaternäre Ammoniumfluoride, Zinkzitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromastoffe, Natriumbicarbonat und/oder Geruchskorrigentien, und
Kaugummis oder Zahnpflegekaugummis bestehend aus einer Kaugummibase enthaltend Elastomere, z.B. Polyvinylacetate (PVA), Polyethylene, (nieder- oder mittelmolekulare) Polyisobutene (PIB), Polybutadiene, Isobuten-isopren Copolymere,, Polyvinylethylether (PVE), Polyvinylbutylether, Copolymere von Vinylestern und Vinylethern, Styren/Butadien Copolymere (SBR) oder Vinylelastomere, z.B. basierend auf Vinylacetat/ Vinyllaurat, Vinyl acetat/Vinylstearat oder Ethylen/vinylacetat, und Mischungen der genannten Elastomerewie z.B. becshrieben in EP 0 242 325, US 4,518,615, US 5,093,136, US 5,266,336 US 5,601,858 oder US 6,986,709. Zusätzlich enthalten Kaugummibasen weitere Inhaltsstoffe, z.B. (mineralische) Füllstoffe (z.B. Calciumcarbonat, Titaniumdioxid, Silicondioxid, Talcum, Aluminiumoxid, Dicalciumphosphat, Tricalciumphosphat, Magnesiumhydroxid und Mischungen daraus, Weichmacher (z.B. Lanolin, Stearinsäure, Natriumstearat, Ethylacetat, Diacetin (Glyceroldiacetat), Triacetin (Glyceroltriacetat) und Triethylcitrat), Emulgatoren (z.B. Phosphatide, wie Lecithin und Mono- und Diglyceride von Fettsäuren, z.B. Glycerolmonostearat), Antioxidantien, Wachse (z.B. Paraffinwachse, Candelillawachse, Carnaubawachs, microkristalline Wachse und Polyethylenewachse)" Fette oder fette Öle (z.B. gehärtete (hydrogenierte) pflanzliche oder tierische Fette) und Mono-, Di- oder Triglyceride.

Bevorzugte erfindungsgemäße, der Ernährung oder dem Genuss dienende Zubereitungen sind:
Süßwaren wie z.B. Lutschbonbons, Kaugummis, Fruchtgummis, Kaubonbons, (Erfrischungs-)Dragees, Komprimate, Hartkaramellen, Schokocremes, Konfekt und Schokolade, Backwaren wie Kuchen, Waffeln und Kekse, Snacks, Instantmahlzeiten sowie anderen Instantprodukten (Getränkepulver und -granulate), Speiseeis, Fruchtzubereitungen (Marmelade, Konfitüre, Fruchtsaucen), Desserts (Puddings, Götterspeise), Milchprodukte (Quark, Joghurts, probiotische Joghurts, Milchdrinks, Molkezubereitungen) sowie Cerealien (Cornflakes, Müsli, Müsliriegel).

Besonders bevorzugte erfindungsgemäße, der Ernährung oder dem Genuss dienende Zubereitungen sind Fruchtgummis, Fruchtzubereitungen (Marmelade, Konfitüre, Fruchtsaucen), Milchprodukte (Quark, Joghurts, probiotische Joghurts, Milchdrinks, Molkezubereitungen) sowie Cerealien (Cornflakes, Müsli, Müsliriegel), wobei wiederum die Milchprodukte Joghurts, probiotische Joghurts und Milchdrinks am meisten bevorzugt sind.

Als weitere Bestandteile für erfindungsgemäße, insbesondere der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Amylose, Amylopektin, Inulin, Xylane, Cellulose), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), ätherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, Kühlwirkstoffe wie z.B. Menthol, Mentholderivate (z.B. L-Menthol, L-Menthyllactat, L-Menthylglutarat, L-Menthylsuccinat) oder Cubebol, synthetische, natürliche oder naturidentische Aromastoffe oder Riechstoffe sowie Geruchskorrigenzien.

Erfindungsgemäße, insbesondere der Ernährung oder dem Genuss dienende Zubereitungen können zusätzlich ein oder mehrere Geschmackskorrigenzien enthalten, vorzugsweise gewählt aus der folgenden Liste: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxy-benzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid , 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl) ethyl]amid (Aduncamid), 4-Hydroxybenzoesäurevanillylamid), bittermaskierende Hydroxy-deoxybenzoine gemäß WO 2006/106023 sowie den darauf basierenden Dokumenten (Symrise) (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen nach WO 2007/045566, Diacetyltrimere nach WO 2006/058893, Divanillin, Gemische von Molkeproteinen mit Lecithinen und/oder bittermaskierende Substanzen wie Gingerdione gemäß WO 2007/003527.

Erfindungsgemäße, insbesondere der Ernährung oder dem Genuss dienende Zubereitungen können zusätzlich ein oder mehrere Alkamide enthalten, vorzugsweise ausgewählt aus der Gruppe bestehend aus: 2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutyl-amid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homo-spilanthol), 2E,6Z,8E-Decatriensäure-N-([2R]-2-methylbutyl)amid, 2E-Decen-4-insäure-N-isobutylamid, 2Z-Decen-4-insäure-N-isobutylamid, Sanshoole.

Erfindungsgemäße, insbesondere der Prävention und Ergänzung der Nahrung sowie zur Therapie von krankhaften Zuständen und der Körperpflege dienende Zubereitungen können vorzugweise Stoffe oder Kombinationen von Stoffen aus den folgende Gruppen enthalten.

Füllstoffe (z.B. Cellulose, Calciumcarbonat), Fließ- und Rieselmittel (z.B. Talkum, Magnesiumstearat), Überzüge (z.B. Polyvinylacetatphtalat, Hydroxypropylmethylcellulosephtalat), Sprengmittel (z.B. Stärke, quervernetztes Polyvinylpyrrolidon), Weichmacher (z.B. Triethylcitrat, Dibutylphtalat) Stoffe zur Granulierung (Lactose, Gelatine), Retardierung (z.B. Poly(meth)acrylsäure-methyl/ethyl/2-trimethylaminoethylester-Copolymerisate in Dispersion, Vinylacetat/ Crotonsäure-Copolymerisate), Kompaktierung (z.B. Mikrokristalline Cellulose, Lactose), Lösungs-, Suspendier oder Dispergiermittel (z.B. Wasser, Ethanol), Emulgatoren (z.B. Cetylalkohol, Lecithin), Stoffe zur Veränderung der rheologischen Eigenschaften (Siliciumdioxid, Natriumalginat), Stoffe zur mikrobiellen Stabilisierung (z.B. benzalkoniumchlorid, Kaliumsorbat), Konservierungsmittel und Antioxidantien (z.B. DL-alpha-tocopherol, Ascorbinsäure), Stoffe zur Veränderung des pHs (Milchsäure, Citronensäure), Treib- oder Inert-Gase (z.B. Fluorierte Chlorkohlenwasserstoffe, Kohlendioxid), Farbstoffe (Eisenoxide, Titandioxid), Salbengrundstoffe (z.B. Paraffine, Bienenwachs), u.a. wie sie in der Fachliteratur (z.B. Schmidt, Christin. Wirk- und Hilfsstoffe für Rezeptur, Defektur und Großherstellung. 1999; Wissenschaftliche Verlagsgesellschaft mbH Stuttgart oder Bauer, Frömming Führer. Lehrbuch der Pharmazeutischen Technologie. 8. Auflage, 2006. Wissenschaftliche Verlagsgesellschaft mbH Stuttgart).

Je nach erfindungsgemäßer Ausgestaltung und gewünschtem Zweck können auch erfindungsgemäße Mischungen (wie oben beschrieben) eine oder mehrere der vorangehend im Zusammenhang mit erfindungsgemäßen Zubereitungen genannten Bestandteile enthalten.

Ein weiterer Aspekt der vorliegenden Erfindung betrifft eine Verbindung, ein Salz, eine Mischung oder eine Zubereitung, wie jeweils oben beschrieben bzw. definiert, zur Anwendung in einem Verfahren zur Behandlung von tierischer oder menschlicher Haut, die einer Behandlung mit anti-inflammatorischen Wirkstoffen bedarf. Bezüglich der Auswahl der Verbindungen bzw. der Salze und der bevorzugten Zusammensetzung der Mischungen und Zubereitungen gilt jeweils das vorangehend Gesagte entsprechend.

Die hierin beschriebenen Verbindungen **(1)** bis **(16)** besitzen vorteilhafterweise eine besonders starke anti-inflammatorische Wirkung. Die Verbindungen **(1)** bis **(16)** eignen sich vorteilhafterweise dazu, um in physiologischen Systemen (von Mensch und Tier) die natürlichen Abwehrmechanismen gegen entzündliche Prozesse zu unterstützen. Ferner kommen diese Verbindungen vorteilhafterweise in Pflanzen mit einer langen Verzehrhistorie (z.B. Herba Santa) vor, wodurch sie für einen Einsatz in Lebensmitteln besonders gut geeignet sind.

Erfindungsgemäß besonders vorteilhaft ist daher eine Mischung oder eine Zubereitung wie oben beschrieben, wobei diese eine, zwei, drei, vier, fünf, sechs, sieben, acht, neun oder sämtliche Verbindungen **(1)** bis **(10)** und zudem eine, zwei, drei, vier, fünf oder sämtliche Verbindungen **(11)** bis **(16)** umfasst bzw. daraus besteht. Besonders bevorzugt ist dabei eine Mischung bzw. eine Zubereitung, die sämtliche der Verbindungen **(1)** bis **(16)** umfasst bzw. daraus besteht.

Besonders bevorzugt ist eine solche Mischung bzw. Zubereitung, wobei eine, mehrere oder sämtliche Verbindungen der Gruppe der Verbindungen **(1)** bis **(16)** Bestandteil eines pflanzlichen Extrakts, vorzugsweise eines Extrakts aus *Eriodictyon ssp.,* besonders bevorzugt eines Extrakts aus *Eriodictyon californicum* und/oder *Eriodictyon angustifolium,* sind.

Es war besonders überraschend, dass die erfindungsgemäß zu verwendenden Verbindungen der Formel (X) bzw. Salze davon starke anti-inflammatorische Wirkungen vermitteln können bzw. besitzen. Erfindungsgemäße Verbindungen, Salze, Mischungen und Zubereitungen (wie jeweils oben beschrieben) sind vorteilhafterweise dazu in der Lage, inflammatorische Parameter in Monocyten positiv zu beeinflussen. In Zellmodellen, in denen gereizte und entzündliche Erscheinungen der Schleimhäute, speziell der Gingiva und des Gastronintestinaltraktes, simuliert werden, zeigen diese eine anti-inflammatorische Wirkung. Insbesondere folgende inflammatorische Parameter werden erfindungsgemäß positiv beeinflusst: PGE2, IL-1, TNF, IL-6 und IL 8, insbesondere PGE2. Entsprechende Experimente hierzu wurden wie in TS1 (siehe unten, "Beispiel TS: Teststudie") beschrieben durchgeführt. So zeigen beispielsweise Eriol-/Erionhaltige Fraktionen bereits ab einer Konzentration von 1 µg/ml eine hochsignifikante Wirkung auf einzelne der vorstehend genannten Parameter. Besonders geeignet sind Konzentrationen von 10 µg/ml und mehr. Ein Vollextrakt aus Herba Santa-Kraut, der erfindungsgemäß anwendbare Verbindungen bzw. Einzelextrakte davon enthält, zeigt beispielsweise signifikante Wirkungen auf einzelne Parameter ab einer Konzentration von 1 µg/ml und hochsignifikante Wirkungen bis 250 µg/ml.

Für erfindungsgemäß anwendbare Salze von Verbindungen der Formel (X) gilt hinsichtlich der bevorzugten Bedeutungen der Reste das weiter oben Gesagte entsprechend. Die Carbonsäuregruppe, die an das Kohlenstoffatom in Position 1 (gemäß der in Formel (X) gezeigten Nummerierung) gebunden ist, liegt dabei deprotoniert vor. Gegebenenfalls liegen zudem eine oder mehrere Hydroxygruppen (sofern vorhanden) deprotoniert vor. Neben der bzw. den (deprotonierten) Verbindung(en) der Formel (X) liegt dabei eine entsprechende Menge an Gegenkationen vor, wobei diese vorzugsweise ausgewählt sind aus der Gruppe bestehend aus: einfach positiv geladene Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, sowie Mischungen davon. Der maximale Deprotonierungsgrad einer einem solchen Salz zugrunde liegenden Verbindung der Formel (X) ergibt sich aus der Carboxylgruppe und den daneben vorliegenden Hydroxygruppen dieser Verbindung. Aus der Zahl an deprotonierten Gruppen ergibt sich wiederum die entsprechende Anzahl an Gegenkationen (in Abhängigkeit von ihrer Ladung). So ergibt sich beispielsweise für eine einem solchen Salz zugrundeliegende Verbindung der Formel (X) mit einer Carboxyl- und einer Hydroxygruppe, dass bei vollständiger Deprotonierung der Gruppen ein zweifach negativ geladenes Anion vorliegt, woraus sich wiederum die Zahl an positiven Ladungen ergibt (hier: zwei), die durch das bzw. die Gegenkation(en) bereitgestellt werden muss. Besonders bevorzugt handelt es sich bei diesen Gegenkationen um Kationen ausgewählt aus der Gruppe bestehend aus Na⁺, K⁺, NH₄⁺, Ca²⁺, Mg²⁺, Al³⁺ und Zn²⁺.

Besonders bevorzugt ist dementsprechend ein Salz einer Verbindung der Formel (X) oder eine Mischung (wie jeweils oben beschrieben) umfassend oder bestehend aus
- einem, zwei oder mehr unterschiedlichen Salzen von Verbindungen der Formel (X), vorzugsweise von vorangehend als bevorzugt bezeichneten Verbindungen der Formel (X), sowie gegebenenfalls
- einem, zwei oder mehr unterschiedlichen Salzen von Verbindungen der Formel (Y), vorzugsweise von vorangehend als bevorzugt bezeichneten Verbindungen der Formel (Y),
   oder
- einer oder mehreren unterschiedlichen Verbindungen der Formel (X) und/oder einem oder mehreren unterschiedlichen Salzen von Verbindungen der Formel (X), sowie gegebenenfalls
- einer oder mehreren unterschiedlichen Verbindungen der Formel (Y) und/oder einem oder mehreren unterschiedlichen Salzen von Verbindungen der Formel (Y),
wobei das bzw. die Gegenkation(en) des bzw. eines, mehrerer oder sämtlicher der Salze von Verbindungen der Formel (X) und/oder Verbindungen der Formel (Y) ausgewählt ist bzw. sind aus der Gruppe bestehend aus Na⁺, K⁺, NH₄⁺, Ca²⁺, Mg²⁺, Al³⁺ und Zn²⁺.

Wie oben erwähnt, kann eine bzw. können mehrere oder sämtliche der erfindungsgemäß zu verwendenden Verbindungen der Formel (X) bzw. Salze von Verbindungen der Formel (X), sowie gegebenenfalls eine, mehrere oder sämtliche der Verbindungen der Formel (Y) bzw. Salze von Verbindungen der Formel (Y) auch in Form von pflanzlichen Extrakten, insbesondere in Form von Extrakten aus *Eriodictyon ssp.,* insbesondere aus *Eriodictyon californicum* und/oder *Eriodictyon angustifolium,* eingesetzt werden, gegebenenfalls nach Behandlung mit einer Base zum Überführen der Verbindung(en) der Formel (X) bzw. (Y) in ein Salz.

Vorzugsweise werden die im Rahmen der vorliegenden Erfindung eingesetzten getrockneten Pflanzenteile (vgl. oben), z.B. frische oder getrocknete Wurzeln, Wurzelrinde, Knollen, Zwiebeln, andere unter- oder oberirdische Speicherorgane, Scheinfrüchte, Früchte, Samen, Rinde, Holz, Mark, Bast, Stängel, Stiele, Blätter oder Blüten[teile], bevorzugt die Stängel, Stiele, Blätter und Blüten[teile], vorzugsweise in zerkleinerter Form, mit einem für Nahrungs- und Genussmittel geeigneten Lösungsmittel bei Temperaturen im Bereich zwischen dem Gefrierpunkt und dem Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise gegenextrahiert, über Verteilungs-, Absorptions-, Ausschluss-, Affinitäts- oder Ionenchromatographie gereinigt, destilliert, sublimiert, mit Adsorptionsmitteln wie Aktivkohlen, Bentonit, Kieselgur etc. gereinigt, enzymatisch (z.B. mit Glycosidasen zur Ausbeutesteigerung an nicht-zuckerhaltigen Molekülen), mit Säure (z.B. unter Druck), mit geeigneten basischen Lösungen z.B. von Hydroxiden, Carbonaten oder Hydrogencarbonaten von Natrium, Kalium, Calcium, Magnesium und Zink, mit sauren Ionentauschern oder mit Wasserdampf behandelt werden, in der Regel bei Drücken von 0,01 mbar bis 100 bar, vorzugsweise bei 1 mbar bis 20 bar, mit einem Hilfs- und Trägerstoff versetzt und gegebenenfalls getrocknet (z.B. sprühgetrocknet) und/oder in einem für Nahrungs- und Genussmittel und/oder für kosmetische und dermatologische Anwendungen geeigneten Lösungsmittel aufgenommen werden.

Zur Extraktion geeignete Lösungsmittel sind insbesondere Wasser, Ethanol, Methanol, Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Bevorzugte Hilfs- oder Trägerstoffe sind Maltodextrin, Stärke, natürliche oder künstliche Polysaccharide und/oder Pflanzengummen wie modifizierte Stärken oder Gummi Arabicum, färbende Mittel, z.B. zugelassene Lebensmittelfarbstoffe, färbende Pflanzenextrakte, Stabilisatoren, Konservierungsstoffe, Antioxidantien und viskositäts-beeinflussende Stoffe.

Besonders bevorzugt ist eine erfindungsgemäße Mischung (wie oben beschrieben), wobei die Mischung einen pflanzlichen Extrakt umfasst oder daraus besteht, vorzugsweise einen Extrakt aus *Eriodictyon ssp.,* besonders bevorzugt einen Extrakt aus *Eriodictyon californicum* und/oder *Eriodictyon angustifolium.* Eine erfindungsgemäß bevorzugte Mischung bzw. eine erfindungsgemäß bevorzugt zu verwendende Mischung (wie oben beschrieben) umfasst oder besteht gemäß einer Ausgestaltung der vorliegenden Erfindung besonders bevorzugt (1.) aus einem Extrakt aus *Eriodictyon californicum*, (2.) einem Extrakt aus *Eriodictyon angustifolium* oder (3.) einem Extrakt aus *Eriodictyon californicum* und *Eriodictyon angustifolium,* d. h. einem Extrakt aus Pflanzen bzw. Pflanzenteilen von sowohl *Eriodictyon californicum* als auch *Eriodictyon angustifolium,* oder (4.) einem Gemisch aus einem Extrakt aus *Eriodictyon californicum* und einem Extrakt aus *Eriodictyon angustifolium.*

Besonders bevorzugt besteht eine erfindungsgemäße Mischung bzw. eine erfindungsgemäß bevorzugt zu verwendende Mischung (wie jeweils oben beschrieben) aus einem Extrakt aus *Eriodictyon ssp.,* besonders bevorzugt aus einem Extrakt aus *Eriodictyon californicum* und/oder *Eriodictyon angustifolium.* Die Herstellung eines pflanzlichen Extrakts aus *Eriodictyon californicum* und/oder *Eriodictyon angustifolium* wird weiter unten beschrieben.

Wie oben beschrieben betrifft ein Aspekt der vorliegenden Erfindung insbesondere eine der Ernährung oder dem Genuss dienende Zubereitung, insbesondere ein Nahrungsmittel, Genussmittel oder Getränk, oder eine kosmetische oder dermatologische Zubereitung, insbesondere eine zur Behandlung, zum Schutz und/oder zur Pflege der Haut, der Nägel und/oder der Haare sowie der oralen Kavität (insbesondere der Gingiva und der Zähne), oder eine pharmazeutische, zur Behandlung von entzündlichen Zuständen des Körpers von Warmblüter, geeignete Zubereitung. Hinsichtlich der Zusammensetzung einer solchen Zubereitung wird grundsätzlich auf die obigen Ausführungen verwiesen.

Gemäß einer bevorzugten Ausgestaltung der vorliegenden Erfindung liegt der Anteil der Gesamtmenge an Verbindungen der Formel (X) und (gegebenenfalls) Verbindungen der Formel (Y) und Salzen davon in der Zubereitung im Bereich von 0,0001 bis 30 Gew.-%, vorzugsweise im Bereich von 0,001 bis 20 Gew.-%, besonders bevorzugt im Bereich von 0,001 bis 5 Gew.-%, bezogen auf das Gesamtgewicht der Zubereitung.

Erfindungsgemäße Zubereitungen, insbesondere erfindungsgemäße der Ernährung oder dem Genuss dienende Zubereitungen, können im Rahmen der vorliegenden Erfindung insbesondere als zum Verzehr geeignete Zusammensetzungen (wie unten beschrieben) ausgestaltet sein. Die der Ernährung oder dem Genuss dienenden Zubereitungen im Sinne der vorliegenden Erfindung können auch als Halbfertigware zur Herstellung weiterer der Ernährung oder dem Genuss dienender Zubereitungen dienen.

Die erfindungsgemäßen der Ernährung oder dem Genuss dienenden Zubereitungen sowie entsprechende Halbfertigwaren und zum Verzehr geeignete Zubereitungen bzw. Zusammensetzungen sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe unten) oder wieder aus der Mundhöhle entfernt zu werden (z.B. Kaugummis). Zu diesen Produkten gehören dabei sämtliche Erzeugnisse oder Stoffe, die dazu bestimmt sind, in verarbeitetem, teilweise verarbeitetem oder unverarbeitetem Zustand vom Menschen aufgenommen zu werden. Insbesondere sind zum Verzehr geeignete Zusammensetzungen Erzeugnisse, die Lebensmitteln bei ihrer Herstellung, Verarbeitung oder Bearbeitung zugesetzt werden und dazu vorgesehen sind, in die menschliche Mundhöhle eingebracht zu werden, insbesondere mit dem besagten Lebensmittel. Demnach können solche Zusammensetzungen wiederum in (weiteren) der Ernährung oder dem Genuss dienenden gebrauchs- oder verzehrfertigen Zubereitungen enthalten sein (der Ernährung oder dem Genuss dienende gebrauchs- oder verzehrfertige Zubereitungen sind im Rahmen des vorliegenden Textes insbesondere Lebensmittel, speziell verzehrfertige Lebensmittel (siehe unten)). Zudem können solche Zusammensetzungen Bestandteil einer Halbfertigware sein, welche gegebenenfalls wiederum zur Herstellung von der Ernährung oder dem Genuss dienenden gebrauchs- oder verzehrfertigen Zubereitungen verwendet werden kann.

Der Ernährung oder dem Genuss dienende Zubereitungen im Sinne der vorliegenden Erfindung sind insbesondere gebrauchs- oder verzehrfertigen Zubereitungen, insbesondere Lebensmittel, speziell verzehrfertige Lebensmittel, z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchproteinhaltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Bevorzugte in solchen erfindungsgemäßen (vorzugsweise sprühgetrockneten) Zusammensetzungen enthaltene Trägerstoffe sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum.

Bevorzugte Stärkehydrolysate sind Maltodextrine und Dextrine, wobei hier wiederum Maltodextrine mit DE-Werten im Bereich 5 bis 20 besonders bevorzugt sind. Dabei ist unerheblich, welche Pflanze ursprünglich die Stärke zur Herstellung der Stärkehydrolysate geliefert hat. Geeignet und leicht verfügbar sind insbesondere Mais-basierende Stärken sowie Stärken aus Tapioka, Reis, Weizen oder Kartoffeln. Dabei können weitere vorangehend beschriebene Trägerstoffe (z.B. Siliciumdioxid) vorteilhafterweise als Fließhilfsmittel fungieren.

Die erfindungsgemäßen Zubereitungen, die neben einer oder mehreren Verbindungen der Formel (X) und/oder Salzen davon bzw. einer entsprechenden Mischung noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind, wie oben beschrieben, erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte Trägerstoffe umfassende Zubereitungen (wie oben beschrieben), die mittels Sprühtrocknung hergestellt wurden, besitzen vorzugsweise eine mittlere Partikelgröße im Bereich von 30 bis 300 µm und vorzugsweise eine Restfeuchte von 5 Gew.-% oder weniger.

Gemäß einer Ausgestaltung der vorliegenden Erfindung liegt das Gewichtsverhältnis der Gesamtmasse an Verbindungen der Formel (X) sowie gegebenenfalls der Formel (Y) und Salzen davon in einer hierin beschriebenen Zubereitung umfassend einen oder mehrere (zum Verzehr geeignete, feste) Trägerstoffe (wie oben beschrieben) zur Gesamtmasse an (zum Verzehr geeigneten, festen) Trägerstoffen vorzugsweise im Bereich von 1:10 bis 1 : 100000, bevorzugt im Bereich von 1 : 50 (vorzugsweise von 1 : 100) bis 1 : 20000, besonders bevorzugt im Bereich von 1 : 100 (vorzugsweise von 1 : 1000) bis 1 : 5000, bezogen auf die Trockenmasse der Zubereitung.

In einer einen oder mehrere (zum Verzehr geeignete, feste) Trägerstoffe enthaltenden Zubereitung (wie oben beschrieben) liegt der Anteil der Gesamtmenge an Verbindungen der Formel (X) sowie gegebenenfalls der Formel (Y), Salzen davon und (zum Verzehr geeigneten, festen) Trägerstoffen, bezogen auf das Gesamtgewicht der Zubereitung, vorzugsweise im Bereich von 70 bis 100 Gew.-%, bevorzugt im Bereich von 85 bis 100 Gew.-%.

Die erfindungsgemäßen der Ernährung oder dem Genuss dienenden Zubereitungen können neben üblicherweise verwendeten tierischen oder pflanzlichen Rohstoffen zusätzlich Wasser, Squalan oder Squalen, natürliche Öle (z.B. Olivenöl, Sonnenblumenöl, Sojaöl, Erdnussöl, Rapsöl, Mandelöl, Palmöl, Kokosöl, Palmkernöl, Borretschsamenöl und dergleichen mehr), natürliche Esteröle (z.B. Jojobaöl), Fette, Wachse und andere natürliche Fettkörper, Kohlenhydrate, beispielsweise Glucose, Sucrose oder Lactose, Süßstoffe, beispielsweise Aspartam, Cyclamat, Saccharin, Xylit oder Sorbitol, Bitterstoffe, beispielsweise Coffein oder Chinin, bitterunterdrückende Stoffe, beispielsweise Lactisol, geschmacksverstärkende Stoffe, beispielsweise Natriumglutamat oder Inositolphosphat, Aminosäuren, beispielsweise Glycin, Alanin, Leucin, Isoleucin, Valin, Prolin, Lysin, Asparagin, Asparaginsäure, Glutamin, Glutaminsäure, Tryptophan, Phenylalanin, Tyrosin, Threonin, Serin, Cystin, Cystein, Methionin, Hydroxyprolin, Arginin oder Histidin, Peptide, Proteine, Enzyme, Fruchtsäuren, vorzugsweise Milchsäure, Äpfelsäure oder Zitronensäure, daneben Emulgatoren, welche vorteilhaft ausgewählt werden können aus der Gruppe der ionischen, nichtionischen, polymeren, phosphathaltigen und zwitterionischen Emulgatoren, sowie insbesondere ein oder mehrere Verdickungsmittel, welches oder welche vorteilhaft ausgewählt werden können aus der Gruppe der Polysaccharide bzw. deren Derivate, z.B. Hyaluronsäure, Guarkernmehl, Johannisbrotkernmehl, Xanthangummi, oder Allulose-Derivate, natürliche, naturidentische oder synthetische Aromen sowie Salze, beispielsweise Natriumchlorid oder Kaliumchlorid enthalten.

Die erfindungsgemäßen kosmetischen und dermatologischen Zubereitungen können kosmetische Hilfs- und/oder Zusatzstoffe enthalten, wie sie üblicherweise in solchen Zubereitungen verwendet werden, z.B. Sonnenschutzmittel (z.B. organische oder anorganische Lichtfiltersubstanzen, bevorzugt Mikropigmente), Konservierungsmittel, Bakterizide, Fungizide, Viruzide, Kühlwirkstoffe, Pflanzenextrakte, entzündungshemmende Wirkstoffe, die Wundheilung beschleunigende Stoffe (z.B. Chitin oder Chitosan und dessen Derivate), filmbildende Substanzen (z.B. Polyvinylpyrrolidone oder Chitosan oder dessen Derivate), gebräuchliche Antioxidantien, Vitamine (z.B. Vitamin C und Derivate, Tocopherole und Derivate, Vitamin A und Derivate), 2-Hydroxycarbonsäuren (z.B. Citronensäure, Äpfelsäure, L-, D-, oder dl-Milchsäure), Hautaufhellungsmittel (z.B. Kojisäure, Hydrochinon oder Arbutin), Hautfärbungsmittel (z.B. Walnussextrakte oder Dihydroxyaceton), Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen (z.B. Glycerin oder Harnstoff), Fette, Öle, ungesättigte Fettsäuren oder deren Derivate (z.B. Linolsäure, alpha-Linolensäure, gamma-Linolensäure oder Arachidonsäure und deren jeweiligen natürlichen oder synthetischen Ester), Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner (z.B. Ethylendiamintetraessigsäure und Derivate).

Die jeweils einzusetzenden Mengen können in Abhängigkeit von der Art des jeweiligen Produkts vom Fachmann durch einfaches Ausprobieren leicht ermittelt werden.

Bevorzugt umfassen erfindungsgemäße Zubereitungen (wie oben beschrieben) zudem ein oder mehrere Antioxidantien, wobei das bzw. die Antioxidantien keine Verbindung(en) der Formel (X) oder ein Salz davon ist bzw. sind. Insbesondere können als solche Antioxidantien alle für die entsprechende Anwendung geeigneten oder gebräuchlichen Antioxidantien verwendet werden. Vorzugsweise ist das bzw. sind die Antioxidantien ausgewählt aus der Gruppe bestehend aus Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Carnosin, L-Carnosin, Anserin) und deren Derivate, Carotinoide, Carotine (z.B. beta-Carotin, alpha-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4-Dihydroxyphenylessigsäure-, Ferulasäure-, Sinapinsäure-, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), Catecholoxime oder Catecholoximether (z.B. 3,4-Dihydroxybenzaldoxim oder 3,4-Dihydroxybenzaldehyd-O-ethyloxim), ferner (Metall)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z.B. Ascorbylpalmitat, Magnesiumascorbylphosphat, Ascorbylacetat), Tocopherole und Derivate (z.B. alpha-Tocopherol, Vitamin-E-Acetat), Vitamin A und Derivate (z.B. Vitamin-A-Palmitat), Rutinsäure und deren Derivate, Flavonoide (z.B. Quercetin, alpha-Glucosylrutin) und deren Derivate, Phenolsäuren (z.B. Gallussäure, Ferulasäure) und deren Derivate (z.B. Gallussäurepropylester, -ethylester, -octylester), Furfurylidenglucitol, Dibutylhydroxytoluol, Butylhydroxyanisol, Harnsäure und deren Derivate, Mannose und deren Derivate, Zink und dessen Derivate (z.B. ZnO, ZnSO₄), Selen und dessen Derivate (z.B. Selenomethionin), Stilbene und deren Derivate (z.B. Stilbenoxid, Resveratrol) und die im Rahmen der vorliegenden Erfindung geeigneten Derivate dieser genannten (Wirk-) Stoffe.

Des Weiteren kann eine erfindungsgemäße Zubereitung (wie oben beschrieben), insbesondere eine erfindungsgemäße kosmetische oder dermatologische Zubereitung, eine oder mehrere UV-A- und/oder UV-B-Filtersubstanz umfassen. Das bzw. die Filtersubstanzen sind dabei vorzugsweise ausgewählt aus der Gruppe bestehend aus 3-Benzylidencampherderivate (z.B. 3-(4-Methylbenzyliden)-dl-campher), Aminobenzoesäurederivate (z.B. 4-(N,N-Dimethylamino)benzoesäure-2-ethylhexylester oder Menthylanthranilat), 4-Methoxycinnamate (z.B. 2-Ethylhexyl-p-methoxycinnamat oder Isoamylp-methoxycinnamat), Benzophenone (z.B. 2-Hydroxy-4-methoxybenzophenon), ein- oder mehrfach sulfonierte UV-Filter [z.B. 2-Phenylbenzimidazol-5-sulfonsäure, Sulisobenzone oder 1,4-Bis(benzimidazolyl)-benzol-4,4',6,6'-tetrasulfonsäure bzw. 3,3'-(1,4-Phenylendimethyliden)-bis-(7,7-dimethyl-2-oxo-bicyclo-[2,2,1]heptan-1-methansulfonsäure) und deren Salze], Salicylate (z.B. 2-Ethylhexylsalicylat oder Homomenthylsalicylat), Triazine {z.B. 2,4-Bis-[4-(2-ethylhexyloxy)-2-hydroxyphenyl]-6-(4-methoxyphenyl)-1,3,5-triazin, 4,4`-([6-([(1,1-dimethylethyl)-aminocarbonyl]phenylamino)-1,3,5-triazin-2,4-diyl]di-imino)bisbenzoesäurebis-(2-ethylhexyl)-ester)}, 2-Cyanopropensäurederivate (z.B. 2-Ethylhexyl-2-cyano-3,3-diphenyl-2-propenoat), Dibenzoylderivate (z.B. 4-tert-Butyl-4'-methoxydibenzoylmethan), polymergebundende UV-Filter (z.B. Polymer von N-[2-(bzw. 4)-(2-Oxo-3-bornyliden)methyl]benzylacrylamid) oder Pigmente (z.B. Titandioxide, Zirkondioxide, Eisenoxide, Siliciumdioxide, Manganoxide, Aluminiumoxide, Ceroxide oder Zinkoxide). Vorzugsweise ist eine solche erfindungsgemäße Zubereitung ein Sonnenschutzmittel für Haut und/oder Haar.

Besonders bevorzugt betrifft die vorliegenden Erfindung dementsprechend eine erfindungsgemäße Zubereitung (wie oben beschrieben) zudem umfassend
(II) ein oder mehrere Antioxidantien, wobei das bzw. die Antioxidantien keine Verbindung(en) der Formel (x) oder ein Salz davon ist bzw. sind und vorzugsweise ausgewählt ist bzw. sind aus der Gruppe bestehend aus beta-Carotin, Lycopin, Chlorogensäure, 2-Hydroxyfettsäuren, Bilirubin, Folsäure, Ubichinon, Ubichinol, Vitamin C und dessen Derivate, insbesondere Ascorbyl-palmitat, Magnesiumascorbylphosphat und Ascorbylacetat; Tocopherole und dessen Derivate, insbesondere alpha-Tocopherol und Vitamin-E-Acetat; Vitamin A und dessen Derivate, insbesondere Vitamin-A-Palmitat; Rutinsäure, Quercetin, Ferulasäure, Dibutylhydroxytoluol, Butylhydroxyanisol und Harnsäure,
   und/oder
(III) eine oder mehrere UV-A- und/oder UV-B-Filtersubstanzen, wobei die bzw. eine, mehrere oder sämtliche der UV-A- und/oder UV-B-Filtersubstanzen vorzugsweise ausgewählt ist bzw. sind aus der Gruppe bestehend aus 3-(4-Methylbenzyliden)-dl-campher, Menthylanthranilat, 2-Ethylhexyl-p-methoxycinnamat, Isoamyl-p-methoxycinnamat, 2-Hydroxy-4-methoxy-benzophenon, 2-Phenylbenzimidazol-5-sulfonsäure und deren Salze, 1,4-Bis(benzimidazolyl)-benzol-4,4`,6,6`-tetrasulfonsäure und deren Salze, 2-Ethyl-hexylsalicylat, Homomenthylsalicylat, 2,4-Bis-[4-(2-ethylhexyloxy)-2-hydroxy-phenyl]-6-(4-methoxyphenyl)-1,3,5-triazin, 4,4'-([6-([(1,1 -dimethylethyl)-amino-carbonyl]phenylamino)-1,3,5-triazin-2,4-diyl]diimino)bisbenzoe-säurebis-(2-ethyl-hexyl)-ester), 2-Ethylhexyl-2-cyano-3,3-diphenyl-2-propenoat, 4-tert-Butyl-4'-methoxydibenzoylmethan, Titandioxid, Siliciumdioxid und Zinkoxid.

Besonders bevorzugt ist eine solche erfindungsgemäße Zubereitung, wobei
- der Anteil der Gesamtmenge an Bestandteil (II) in der Zubereitung, bezogen auf das Gesamtgewicht der Zubereitung, 0,0001 bis 30 Gew.-% , vorzugsweise 0,001 bis 20 Gew.-%, besonders bevorzugt 0,001 bis 5 Gew.-%, beträgt
   und/oder
- der Anteil der Gesamtmenge an Bestandteil (III) in der Zubereitung, bezogen auf das Gesamtgewicht der Zubereitung, 0,1 bis 30 Gew.-% , vorzugsweise 0,5 bis 10 Gew.-%, beträgt.

Die der Behandlung von entzündlichen Zuständen von Warmblütern dienenden (pharmazeutischen) Zubereitungen im Sinne der vorliegenden Erfindung können auch als Halbfertigware zur Herstellung weiterer der Behandlung von entzündlichen Zuständen von Warmblütern dienende, pharmazeutische Zubereitungen dienen.

Die erfindungsgemäßen der Behandlung von entzündlichen Zuständen von Warmblütern dienende, pharmazeutische Zubereitungen sowie entsprechende Halbfertigwaren sind regelmäßig Produkte, die dazu bestimmt sind, in Körper von Warmblütern eingebracht oder am Köper von Warmblütern angewendet zu werden.

Die erfindungsgemäßen der Behandlung von entzündlichen Zuständen von Warmblütern dienende, pharmazeutische Zubereitungen im Sinne der vorliegenden Erfindung sind vorzugsweise gebrauchsfertigen Zubereitungen, insbesondere Arzneimittel und Medizinprodukte, vorzugsweise in folgenden Formen: feste galenische Formen (wie z.B. Tabletten (mit Überzug und ohne, mit modifizierter Freisetzung und ohne), Dragees (mit Überzug und ohne, mit modifizierter Freisetzung und ohne), Kapseln (Weich- oder Hartgelatinekapseln mit modifizierter Freisetzung und ohne) Granulate (mit modifizierter Freisetzung und ohne), Pulver (mit modifizierter Freisetzung und ohne), Suppositorien (mit Überzug und ohne, mit modifizierter Freisetzung und ohne) Lutschbonbons, Kaugummis), sowie flüssige Formen (wie z.B. Lösungen, Suspensionen, Emulsionen, Sirupe (umgangssprachlich Hustensaft), Mundspülungen, Gurgellösungen, Halssprays oder Nasensprays, Nasentropfen, Nasenspüllösungen, Nasenpulver, Nasensalben oder Ohrentropfen, Ohrensprays, Ohrenspüllösungen, Ohrenpuder, Ohrentampons) sowie halbfeste Formen (wie z.B. Hydrophobe Salben darunter z.B.: Kohlenwasserstoffgele, Lipogele. Silikongele, Oleogele. sowie wasseraufnehmende Salben darunter z.B. Absorptionsbasen, hydrophile Salben, hydrophile Gele (Hydrogele) oder Pasten sowie Inhalate (wie z.B. Druckgas-Dosierinhalatoren, Pulver-Inhalatoren, Inhaltoren mit Zerstäuber, Inhalationskonzentrate zur Bereitung von Inhalationen) sowie wirkstoffhaltige Pflaster oder andere therapeutische Systeme.

Die erfindungsgemäßen pharmazeutischen Zubereitungen können (weitere) pharmazeutische Hilfs- und/oder Zusatzstoffe enthalten, wie sie üblicherweise in solchen Zubereitungen verwendet werden, z.B. Wirkstoffe aus der Gruppe der nichtsteroidalen Antiphlogistika, Antibiotika, systemisch wirksame Steroide, Anti-TNF-alpha-Antikörper oder andere biotechnologisch hergestellte Wirkstoffe und /oder Reinstoffe wie Budesonid, Sulfasalazin, Azathioprin/6-Mercaptopurin, Methotrexat. Sowie z. B. Füllstoffe (z.B. Cellulose, Calciumcarbonat), Fließ- und Rieselmittel (z.B. Talkum, Magnesiumstearat), Überzüge (z.B. Polyvinylacetatphtalat, Hydroxypropylmethylcellulosephtalat), Sprengmittel (z.B. Stärke, quervernetztes Polyvinylpyrrolidon), Weichmacher (z.B. Triethylcitrat, Dibutylphtalat) Stoffe zur Granulierung (Lactose, Gelatine), Retardierung (z.B. Poly(meth)acrylsäure-methyl/ethyl/2-trimethylaminoethylester-Copolymerisate in Dispersion, Vinylacetat/ Crotonsäure-Copolymerisate), Kompaktierung (z.B. Mikrokristalline Cellulose, lactose), Lösungs-, Suspendier oder Dispergiermittel (z.B. Wasser, Ethanol), Emulgatoren (z.B. Cetylalkohol, Lecithin), Stoffe zur Veränderung der rheologischen Eigenschaften (Siliciumdioxid, Natriumalginat), Stoffe zur mikrobiellen Stabilisierung (z.B. benzalkoniumchlorid, Kaliumsorbat), Konservierungsmittel und Antioxidantien (z.B. DL-alpha-tocopherol, Ascorbinsäure), Stoffe zur Veränderung des pHs (Milchsäure, Citronensäure), Treib- oder Inert-Gase (z.B. Fluorierte Chlorkohlenwasserstoffe, Kohlendioxid), Farbstoffe (Eisenoxide, Titandioxid), Salbengrundstoffe (z.B. Paraffine, Bienenwachs), u.a. wie sie in der Fachliteratur (z.B. Schmidt, Christin. Wirk- und Hilfsstoffe für Rezeptur, Defektur und Großherstellung. 1999; Wissenschaftliche Verlagsgesellschaft mbH Stuttgart oder Bauer, Frömming Führer. Lehrbuch der Pharmazeutischen Technologie. 8. Auflage, 2006. Wissenschaftliche Verlagsgesellschaft mbH Stuttgart).

Die jeweils einzusetzenden Mengen können in Abhängigkeit von der Art des jeweiligen Produkts vom Fachmann durch einfaches Ausprobieren leicht ermittelt werden.

Gemäß einem Aspekt der vorliegenden Erfindung ist eine solche erfindungsgemäße Zubereitung bevorzugt, wobei der Anteil der Gesamtmenge an Verbindungen der Formel (X) sowie gegebenenfalls der Formel (Y) und Salzen davon in der Zubereitung im Bereich von 0,0001 bis 30 Gew.-%, vorzugsweise im Bereich von 0,001 bis 20 Gew.-%, besonders bevorzugt im Bereich von 0,001 bis 5 Gew.-%, liegt, bezogen auf das Gesamtgewicht der Zubereitung.

Im Folgenden werden (weitere) bevorzugte Verfahren zur Herstellung einer bevorzugten erfindungsgemäßen Mischung oder Zubereitung (wie oben beschrieben) beschrieben.

Ein solches Verfahren umfasst vorzugsweise die Schritte
(a) Extrahieren von Pflanzenmaterial (wie oben beschrieben), vorzugsweise wie oben als bevorzugt beschrieben, und
(b) Anreichern von extrahierten Verbindungen der Formel (X) sowie gegebenenfalls zudem der Formel (Y) und/oder Salzen davon (wie oben beschrieben) durch teilweises oder vollständiges Entfernen sonstiger extrahierter Verbindungen und gegebenenfalls Entfernen von Extraktions- und/oder Lösemitteln,

vorzugsweise so dass der Anteil der Gesamtmenge an Verbindungen der Formel (X) sowie gegebenenfalls (Y) und Salzen davon in der erhaltenen Mischung, bezogen auf das Gesamtgewicht der Mischung, 0,0001, vorzugsweise 15 bis 100 Gew.-%, vorzugsweise 25 bis 90 Gew.-% (bevorzugt 100 Gew.-%), besonders bevorzugt 45 bis 85 Gew.-% (bevorzugt 100 Gew.-%), beträgt.

Beispielsweise kann eine erfindungsgemäße Mischung oder Zubereitung (wie oben beschrieben) auch durch ein erfindungsgemäßes Verfahren (wie oben beschrieben) hergestellt werden, wobei das Verfahren in Anlehnung an das in der Veröffentlichung WO2004041804 beschriebe Verfahren (zur Gewinnung eines Rohextraktes aus *Eriodictyon californicum* und/oder *Eriodictyon angustifolium*) durchgeführt wird.

Die Schritte eines solchen erfindungsgemäßen Verfahrens sind im Folgenden (teilweise basierend auf dem Verfahren gemäß WO2004041804) kurz zusammengefasst:
a) Pflanzenmaterial aus *Eriodictyon californicum* und/oder *Eriodictyon angustifolium* wird mit einem nicht-wassermischbaren Extraktionsmittel ein- oder mehrfach extrahiert (ggf. unter Erhitzen bis zum jeweiligen Siedepunkt).
b) Der so gewonnene Rohextrakt (umfassend erfindungsgemäß zu verwendende Verbindungen der Formel X und/oder der Formel Y und sonstige extrahierte Verbindungen wie beispielsweise Rosmarinsäure) wird vom Pflanzenmaterial abgetrennt.
c) Der Rohextrakt (umfassend erfindungsgemäß zu verwendende Verbindungen der Formel X und/oder der Formel Y und sonstige extrahierte Verbindungen wie beispielsweise Rosmarinsäure) wird vorzugsweise aufkonzentriert und zwischengelagert.
d) Ausgefallene Festkörper (Wachse) werden gegebenenfalls abgetrennt.
e) Der (gegebenenfalls entwachste) Rohextrakt (umfassend erfindungsgemäß zu verwendende Verbindungen der Formel (X) sowie gegebenenfalls der Formel (Y) und sonstige extrahierte Verbindungen wie beispielsweise Rosmarinsäure) wird gegebenenfalls mit Aktivkohle behandelt und vom Festkörper abgetrennt.

Durch folgende weiteren Schritte lassen sich die erfindungsgemäß zu verwendenden Verbindungen der Formel (X) sowie gegebenenfalls der Formel (Y) in der organischen Phase weiter anreichern:
f) (Vorzugsweise vollständiges) Entfernen des organischen Lösungsmittels der organischen Phase durch evaporative oder permeative Verfahren.
g) Aufnehmen des Rückstandes (umfassend erfindungsgemäß zu verwendende Verbindungen der Formel (X) sowie gegebenenfalls der Formel (Y)) in Methanol.
h) Entfernen der in Methanol unlöslichen Bestandteile der Mischung (umfassend erfindungsgemäß zu verwendende Verbindungen der Formel (X) sowie gegebenenfalls der Formel (Y) und sonstige extrahierte Verbindungen) durch Filtration.

In der gemäß diesem Verfahren nach Schritt e) vorliegenden Mischung liegt der Anteil der Gesamtmenge an Verbindungen der Formel (X) sowie gegebenenfalls der Formel (Y) und Salzen davon, bezogen auf die Trockenmasse der Mischung (j), gemäß einer bevorzugten Ausgestaltung der vorliegenden Erfindung im Bereich von 1 bis 35 Gew.-%, bevorzugt im Bereich von 20 bis 35 Gew.-%.

In der gemäß diesem erfindungsgemäßen Verfahren nach Schritt h) vorliegenden Mischung (h) liegt der Anteil der Gesamtmenge an Verbindungen der Formel Formel (X) sowie gegebenenfalls der Formel (Y) und Salzen davon, bezogen auf die Trockenmasse der Mischung (h), vorzugsweise im Bereich von 40 bis 100 Gew.-%, bevorzugt im Bereich von 45 bis 85 Gew.-%.

Die Mischungen (e) und (h) sowie die nach den Schritten f) und g) vorliegenden Mischungen, insbesondere Mischung (h), können jeweils erfindungsgemäße Mischungen (wie oben beschrieben) darstellen.

Evaporative oder pervaporative Verfahren können z.B. Destillation, Sublimation, Wasserdampfdestillation, Gefriertrocknung, pervaporative Membranverfahren oder Sprühtrocknung sein, wobei hierzu jeweils geeignete Hilfs- und/oder Trägerstoffe zugesetzt werden können.

Gemäß einer alternativen Ausgestaltung eines erfindungsgemäßen Verfahrens zur Herstellung einer wie oben beschriebenen Mischung wird ein methanolischer Rohextrakt aus *Eriodictyon ssp.,* bevorzugt *Eriodictyon californicum* und/oder *Eriodictyon angustifolium,* gewonnen, welcher extrahierte Verbindungen der Formel (X) sowie gegebenenfalls der Formel (Y) und sonstige extrahierte Verbindungen (insbesondere Rosmarinsäure) umfasst. Auch hierbei werden die extrahierten Verbindungen der Formel (X) sowie gegebenenfalls der Formel (Y) und/oder Salze dieser extrahierten Verbindungen in der Mischung durch teilweises oder vollständiges Entfernen sonstiger extrahierter Verbindungen und gegebenenfalls Entfernen von Extraktions- und/oder Lösemitteln (jeweils vorzugsweise analog der vorangehend beschriebenen Verfahrensgestaltung) angereichert, so dass der Anteil der Gesamtmenge an Verbindungen der Formel (X) sowie gegebenenfalls der Formel (Y) und Salzen davon in der Mischung, bezogen auf die Trockenmasse der Mischung, zum Beispiel bevorzugt im Bereich von 40 bis 100 Gew.-%, besonders bevorzugt 45 bis 85 Gew.-%, liegt.

Eine solche Anreicherung wird vorzugsweise durch FCPC (Fast Centrifugal Partition Chromatography, Guido F. Pauli, Samuel M. Pro, J. Brent Friesen Countercurrent Separation of Natural Products J. Nat. Prod. 2008, 71, 1489-1508) und/oder HTLC (High Temperature Liquid Chromatograpy; W02006111476) und/oder präparative HPLC (High pressure liquid chromatography) erreicht.

Gemäß einer bevorzugten Ausführungsform der vorliegenden Erfindung werden die hierin beschriebenen Extrakte bzw. Mischungen in Form von Emulsionen in Liposomen, beispielsweise ausgehend von Phosphatidylcholin, in Microsphären, in Nanosphären oder auch in Kapseln, Granulaten oder Extrudaten aus, beispielsweise aus Stärke, Stärkederivaten, Cellulose oder Cellulosederivaten (beispielsweise Hydroxypropylcellulose), anderen Polysacchariden (beispielsweise Alginat), natürlichen Fetten, natürlichen Wachsen (beispielsweise Bienenwachs, Carnaubawachs) oder aus Proteinen, beispielsweise Gelatine, eingearbeitet.

Im Zusammenhang mit der vorliegenden Erfindung wird auch ein wie oben beschriebenes prophylaktisches und/oder therapeutisches Verfahren beschrieben, mit folgendem Schritt:
Kontaktieren von (menschlichem oder tierischem) Gewebes und/oder der (menschlichen oder tierischen) Zellen mit einer entzündungshemmend wirksamen Menge an einer Verbindung der Formel (X), einem Salz einer Verbindung der Formel (X), einer oben beschriebenen Mischung, insbesondere einer Mischung, die zudem eine Verbindung der Formel (Y) und/oder ein Salz davon umfasst, wie jeweils oben beschrieben, oder einer Zubreitung wie oben beschrieben.

Das Kontaktieren des Gewebes bzw. der Zellen mit einer oder mehreren Verbindungen der Formel (X) sowie gegebenenfalls zudem einer oder mehreren Verbindungen der Formel (Y) und/oder Salzen davon bzw. einer erfindungsgemäßen Mischung oder Zubereitung (wie jeweils oben beschrieben) kann dabei - abhängig von dem zu behandelndem Gewebe bzw. den zu behandelnden Zellen - durch äußere (z.B. topisch) oder innere Anwendung (z.B. orale Applikation) erfolgen.

Die folgenden Beispiele dienen zur Verdeutlichung der Erfindung, ohne diese dadurch einzuschränken.

### Beispiele:

### Beispiel 1

### Herstellung eines methanolischen Extrakts aus Eriodictyon angustifolium

500 g getrocknete Blätter von *Eriodictyon angustifolium* wurden mit kochendem Wasser übergossen und für eine Stunde gerührt, um das Pflanzenmaterial aufzuquellen und für die weitere Extraktion vorzubereiten. Das Pflanzenmaterial wurde abfiltriert, getrocknet und zweimal mit je 2.0 I Methanol bei Raumtemperatur für eine Stunde unter Rühren extrahiert. Der methanolische Extrakt wurde abfiltriert, unter Vakuum getrocknet und über Nacht im Hochvakuum-Trockenofen gelagert, um Restlösungsmittel zu entfernen. Die Extraktion erbrachte 84.53 g dunkelgrünen Extrakt.

### Beispiel 2

### Isolierung der Einzelverbindungen aus Eriodictyon angustifolium mittels FCPC

Der methanolische Extrakt von *E. angustifolium* gemäß Beispiel 1 wurde mittels FCPC mit Hilfe eines zwei-phasigen Lösungsmittelsystems (Heptan/ Ethylacetat/ Methanol/ Wasser 5:4:4:5) aufgetrennt und fraktioniert. Neben in der Literatur beschriebenen Flavanonen konnten vier Verbindungen der Formel (X) isoliert werden. Strukturaufklärung erfolgte mit Hilfe ein- und zweidimensionaler NMR-Experimente.

| **Verbindung** | **Molekulargewicht [g/mol]** | **Retentionszeit [min]** |
|---|---|---|
| Erionsäure F **(6)** | 358 | 17.0 - 19.0 (ascending) |
| Erionsäure C **(3)** | 374 | 64.0 - 69.0 (ascending) |
| Erionsäure A **(1)** | 390 | 52.0 - 54.0 (descending) |
| Erionsäure B **(2)** | 390 | 55.0 - 56.0 (descending) |

### Bedingungen FCPC:

| | |
|---|---|
| *FCPC:* | bench scale FCPC model, Version A (Kromaton Technologies, Angers, France) |
| *Rotor:* | 200 ml (semipräparativ) |
| *Injektor:* | Kronlab High Speed Valve (Kronlab Chromatography Technology, Dinslaken) |
| *Injektionsschleife:* | 10 ml |
| *Pumpen:* | Knauer HPLC Pumpe 64 (Knauer Berlin) |
| *Pulsationsdämpfer:* | Typ 55073 (BESTA-Technik, Wilhelmsfeld) |
| *Detektor:* | ELSD SEDEX 75 Lichtstreudetektor (S.E.D.E.R.E, Alfortville, Cedex, France) |
| *Fractionssammler:* | Labocol Vario 2000 (Labomatic, Weil am Rhein) |
| *Software:* | PrepCon (SCPA GmbH, Weye-Leeste); Version 5.03.009, SCPA GmbH 2003 |
| *Lösungsmittelsystem:* | obere Phase: Heptan/ Ethylacetat (5/4) |
| | untere Phase: Methanol/ Wasser (4/5) |
| *Ascending Modus:* | Methanol/ Wasser als Stationäre Phase |
| *Descending Modus:* | Heptan/ Ethylacetat als Stationäre Phase |
| *Stammlösung:* | 60 mg/ 10 ml Stationäre/ Mobile Phase (1:1) |
| *Flußrate:* | 8 ml/min (ascending Modus) |
| | 10 ml/min (descending Modus) |
| *Fraktionierung:* | 40 Fraktionen à 8 ml (ascending Modus) |
| | 30 Fraktionen à 10 ml (descending Modus) |

### Analytische Daten:

| | **Erionsäure F (6)** | | **Erionsäure C (3)** | |
|---|---|---|---|---|
| | (400 MHz, CH3OD) | | (400 MHz, CH3OD) | |
| **Pos.** | δ_{c}, **mult.** | δ_{H} **(*J* in Hz)** | δ_{c}, **mult.** | δ_{H} **(*J* in Hz)** |
| **1** | 123.2 | | n.d. | |
| **2** | 130.8 | 7.63, d (2.1) | 131.1^{a} | 6.92, s |
| **3** | 129.4 | | 130.3 | |
| **4** | 158.4 | | 158.4 | |
| **5** | 129.4 | | 125.6 | |
| **6** | 130.5 | 7.71, d (2.1) | 130.8^{a} | 6.92, s |
| **7** | 170.8 | | 171.1 | |
| **8** | 29.0 | 2.61, m | 29.2 | 2.59, m |
| **9** | 33.8 | 1.95, m | 33.9 | 1.95, m |
| | | 1.61, m | | 1.61, m |
| **10** | 46.2 | 2.65, m | 46.5 | 2.66, m |
| **11** | 216.1 | | 217.4 | |
| **12** | 44.5 | 3.18, m | 42.2 | 3.19, m |
| **13** | 117.2 | 5.23, dd (7.1, 7.1) | 117.5 | 5.24, m |
| **14** | 136.7 | | 136.8 | |
| **15** | 25.9 | 1.77, s^{a} | 25.9 | 1.72, s |
| **16** | 16.9 | 1.12, d (7.0) | 16.9 | 1.12, d (7.0) |
| **17** | 17.9a | 1.72, s^{a} | 18.3 | 1.61, s |
| **18** | 29.4 | 3.33, d (7.3) | 29.2 | 3.40, d (7.4) |
| **19** | 123.0 | 5.32, dd (7.3, 7.3) | 124.2 | 5.61, dd (7.4, 7.4) |
| **20** | 134.2 | | 137.8 | |
| **21** | 26.0 | 1.72, s^{b} | 69.1 | 3.99, s |
| **22** | 18.1a | 1.60, s^{b} | 13.9 | 1.76, s |

| | | | | |
|---|---|---|---|---|
| ^{a,b} austauschbare Signale | | | | |

| | **Erionsäure A (1)** | | **Erionsäure B (2)** | |
|---|---|---|---|---|
| | (400 MHz, DMSO) | | (600 MHz, CH₃OD) | |
| | δ_{c}, **mult**. | δ_{H} **(J in Hz)** | δ_{c}, **mult.** | δ_{H} **(J in Hz)** |
| **1** | 127.7^{a} | | 130.3^{a} | |
| **2** | 128.7^{b} | 7.52, s | 130.4^{a} | 7.57, d (2.1) |
| **3** | 128.3^{a} | | 129.6^{a} | |
| **4** | 156.5 | | 155.0^{b} | |
| **5** | 121.0 | | 131.4^{a} | |
| **6** | 128.9^{b} | 7.52, s | 130.4^{a} | 7.56, d (2.1) |
| **7** | 169.1 | | 170.2 | |
| **8** | 27.4 | 2.55, m | 28.9 | 2.58, m |
| **9** | 32.6 | 1.83, m | 34.6 | 1.96, m |
| | | 1.51, m | | 1.61, m |
| **10** | 42.7 | 2.85, ddq (6.8, 6.8, 6.8) | 44.4 | 2.86, ddq (6.8, 6.8, 6.9) |
| **11** | 203.4 | | 207.0 | |
| **12** | 123.8 | 6.27, d (15.8) | 125.6 | 6.29, d (15.8) |
| **13** | 154.3 | 6.83, d (15.8) | 154.9^{b} | 6.83, d (15.8) |
| **14** | 69.1 | | 71.2 | |
| **15** | 29.0 | 1.22, s^{a} | 29.3 | 1.30, s^{a} |
| **16** | 16.2 | 1.07, d (6.9) | 16.8 | 1.13, d (6.9) |
| **17** | 29.0 | 1.23, s^{a} | 29.3 | 1.29, s^{a} |
| **18** | 27.6 | 3.33, d (7.3) | 32.4 | 3.02, dd (5.4, 16.5) |
| | | | | 2.74, dd (7.8, 16.5) |
| **19** | 120.8 | 5.50, dd (7.3, 7.3) | 70.5 | 3.75, dd (7.8, 5.4) |
| **20** | 136.2 | | 78.4 | |
| **21** | 66.1 | 3.83, s | 24.3^{c} | 1.36, s^{a} |
| **22** | 13.1 | 1.65, s | 21.0^{c} | 1.25, s^{a} |

| | | | | |
|---|---|---|---|---|
| ^{a} austauschbare Signale | | | | |

### Beispiel 3

### Isolierung der Einzelverbindungen aus Eriodicyton angustifolium mittels Hochtemperatur-Flüssigkeitschromatographie (HTLC)

Der methanolische Extrakt von *E. angustifolium* gemäß Beispiel 1 wurde mittels HTLC unter Verwendung einer polymer-basierenden semi-präparativen Säule mit Wasser-Ethanol-Gradienten bei isothermen Bedingungen (120°C) separiert und fraktioniert. Zusätzlich zu in der Literatur beschriebenen Flavanonen konnten vier Substanzen isoliert werden.

| **Verbindung** | **Molekulargewicht [g/mol]** | **Retentionszeit [min]** |
|---|---|---|
| Erionsäure C **(3)** | 374 | 14,5 - 15,3 |
| Erionsäure D **(4)** | 374 | 15,4 - 16,5 |
| Erionsäure E **(5)** | 374 | 17,0 - 17,7 |
| Erionsäure F **(6)** | 358 | 22,0 - 23,0 |

### Bedingungen der HTLC:

| | | | |
|---|---|---|---|
| *Pumpen:* | 2 SunChrom HPLC Pumpen SunFlow 100 (SunChrom, Friedrichsdorf, Germany) | | |
| *Injektor:* | 100 µl Schleife; Midas, Spark, AJ Emmen, The Netherlands | | |
| *HPLC-Ofen:* | Polaratherm Series 9000 (Selerity Technologies Inc., Salt Lake City, USA) | | |
| *Detektoren:* | Lichtstreudetektor (ELSD) Sedex 85 LT-ELSD | | |
| | (Sedere, Alfortville, Cedex, France) | | |
| | Diode array detector (DAD) SunChrom SpectraFlow, Wellenlängen 200 - 400 nm (SunChrom, Friedrichsdorf, Germany) | | |
| *Säule:* | Hamilton PRP-1 reversed phase; 250 x 10 mm semi präparativ; 10 µm Partikelgröße (Hamilton, Bonaduz, Switzerland) | | |
| *Flußrate:* | 3 ml/min | | |
| *Fraktionssammler:* | Labocol Vario 2000 (Labomatic, Weil am Rhein) | | |
| *Software:* | PrepCon (SCPA GmbH, Weye-Leeste); Version 5.03.009, SCPA GmbH 2003 | | |
| *Stammlösung:* | 400 mg/ ml *E. angustifolium* Extrakt (Y) in Ethanol/Wasser (1:1) | | |
| *Injektionsvolumen:* | 100 µl | | |
| *Säule:* | Hamilton PRP-1 250x10 mm | | |
| *Temperatur:* | 120°C isotherm | | |
| *Eluent:* | A: Wasser C: Ethanol | | |
| *Gradient:* | 0 min: | 100 % A | 0 % C |
| | 30 min: | 50 % A | 50 % C |
| | 50 min: | 0 % A | 100 % C |
| | 60 min: | 0 % A | 100 % C |
| *Detektion:* | ELSD (3.5 bar N2, 45 °C, Gain 6); | | |
| | DAD 210 nm, 250 nm, 280 nm, 320 nm | | |

### Analytische Daten:

Die analytischen Daten für Erionsäure C (**3**) und Erionsäure F (**6**) entsprechen den unter Beispiel 2 angegebenen.

| | **Erionsäure D (4)** | | **Erionsäure E (5)** | |
|---|---|---|---|---|
| | (400 MHz, CH3OD) | | (400 MHz, CH₃OD) | |
| **Pos**. | δ_{c}, **mult.** | δ_{H} **(*J* in Hz)** | δ_{c}, **mult.** | δ_{H} **(*J* in Hz)** |
| **1** | 130.6^{a} | | 123.3 | |
| **2** | 130.6^{a} | 7.60, d (2.2) | 131.0^{a} | 7.60, d (2.2) |
| **3** | 130.6^{a} | | 130.3 | |
| **4** | 156.2 | | 158.1 | |
| **5** | 120.9/120.8 | | 128.9 | |
| **6** | 131.3 | 7.61, d (2.2) | 130.8^{a} | 7.63, d (2.2) |
| **7** | 171.0 | | 171.5 | |
| **8** | 28.9/28.8 | 2.56, m | 29.3 | 2.62, m |
| **9** | 34.0/34.0 | 1.94, m | 34.4 | 1.97, m |
| | | 1.60, m | | 1.63, m |
| **10** | 46.3/46.3 | 2.63, m | 44.5 | 2.89, ddq (6.9, 6.9, 6.9) |
| **11** | 215.6/215.5 | | 207.1 | |
| **12** | 41.9 | 3.17, m | 125.8 | 6.34, d (15.9) |
| **13** | 117.3 | 5.23, m | 155.5 | 6.88, d (15.9) |
| **14** | 136.6 | | 71.2 | |
| **15** | 25.9 | 1.72, s | 29.3 | 1.31, s |
| **16** | 16.7 | 1.11, d (6.9) | 17.2 | 1.15, d (7.0) |
| **17** | 18.3 | 1.60, s | 29.3 | 1.31, s |
| **18** | 32.2 | 3.05/3.06, dd/dd (16.7,5.2) | 29.7 | 3.31 (verdeckt durch Lösungsmittel), |
| | | 2.76, dd (16.6, 7.2) | | |
| **19** | 70.0/70.0 | 3.78, m | 123.4 | 5.33, dd (7.4, 7.4) |
| **20** | 79.1/79.1 | | 134.5 | |
| **21** | 26.1/26.0^{b} | 1.37/1.36^{a} | 26.2 | 1.77, s |
| **22** | 21.6/21.3^{b} | 1.30/1.29^{a} | 18.0 | 1.71, s |

| | | | | |
|---|---|---|---|---|
| ^{a,b} austauschbare Signale | | | | |

### Beispiel 4

### Herstellung eines angereicherten Extrakts aus E. angustifolium mittels Gelpermeationschromatographie

Aus 0,5 g des methanolischen Extrakts von *E. angustifolium* gemäß Beispiel 1 wurden mit einer Flussrate von 2,5 ml/min über eine Sephadex-LH 20-Säule die Flavanone abgetrennt, der verbleibende Extrakt unter Vakuum getrocknet und über Nacht im Hochvakuum-Trockenofen gelagert, um Restlösungsmittel zu entfernen. Der so gewonnene flavanoidfreie Trocken-Extrakt hatte einen Gehalt von 46 % erfindungsgemäß zu verwendender Benzoesäurederivate.

### Bedingungen der Gelpermeationschromatographie:

| | |
|---|---|
| Stammlösung: | 0,5 g/ 20 ml Methanol |
| Säule: | Kronlab 3,5 × 60 cm |
| Säulenmaterial: | Sephadex LH-20 |
| Lösungsmittel: | Methanol |
| Flussrate: | 2,5 ml/min |
| Detektion (UV): | 210 nm |

### Beispiel 5

### Herstellung eines methanolischen Extrakts aus Eriodictyon californicum

150 g getrocknete Blätter von *Eriodictyon californicum* wurden mit kochendem Wasser übergossen und für eine Stunde gerührt, um das Pflanzenmaterial aufzuquellen und für die weitere Extraktion vorzubereiten. Das Pflanzenmaterial wurde abfiltriert, getrocknet und zweimal mit je 1.5 I Methanol bei Raumtemperatur für eine Stunde unter Rühren extrahiert. Der Extrakt wurde abfiltriert, unter Vakuum getrocknet und über Nacht im Hochvakuum-Trockenofen gelagert, um Restlösungsmittel zu entfernen. Die Extraktion erbrachte 32.65 g dunkelgrünen Extrakt.

### Beispiel 6

### Isolierung der Einzelverbindungen aus Eriodicyton californicum mittels HTLC

Der methanolische Extrakt von *E. californicum* gemäß Beispiel 5 wurde mittels HTLC unter Verwendung einer polymer-basierenden semi-präparativen Säule mit Wasser-Ethanol-Gradienten bei isothermen Bedingungen (120°C) separiert und fraktioniert. Zusätzlich zu aus der Literatur bekannten Flavanonen konnten vier Verbindungen der Formel (X) isoliert werden.

| **Verbindung** | **Molekulargewicht [g/mol]** | **Retentionszeit [min]** |
|---|---|---|
| Eriolsäure A **(7)** | 388 | 15.0 - 16.5 |
| Eriolsäure B **(8)** | 358 | 21.0 - 22.9 |
| Eriolsäure C **(9)** | 374 | 13,5 - 14,8 |
| Eriolsäure D **(10)** | 372 | 20,0 - 21,0 |

### Bedingungen der HTLC:

| | | | |
|---|---|---|---|
| *Pumpen:* | 2 SunChrom HPLC Pumpen SunFlow 100 (SunChrom, Friedrichsdorf, Germany) | | |
| *Injektor:* | 100 µl Schleife; Midas, Spark, AJ Emmen, The Netherlands | | |
| *HPLC-Ofen:* | Polaratherm Series 9000 (Selerity Technologies Inc., Salt Lake City, USA) | | |
| *Detektoren:* | Lichtstreudetektor (ELSD) Sedex 85 LT-ELSD | | |
| | (Sedere, Alfortville, Cedex, France) | | |
| | Diode array detector (DAD) SunChrom SpectraFlow, Wellenlängen 200 - 400 nm (SunChrom, Friedrichsdorf, Germany) | | |
| *Säule:* | Hamilton PRP-1 reversed phase; 250 x 10 mm semi präparativ; 10 µm Partikelgröße (Hamilton, Bonaduz, Switzerland) | | |
| *Flußrate:* | 3 ml/min | | |
| *Fraktionssammler:* | Labocol Vario 2000 (Labomatic, Weil am Rhein) | | |
| *Software:* | PrepCon (SCPA GmbH, Weye-Leeste); Version 5.03.009, SCPA GmbH 2003 | | |
| *Stammlösung:* | 300 mg/ ml *E. californicum* Extrakt (Y) in Ethanol/Wasser (1:1) | | |
| *Injektionsvolumen:* | 100 µl | | |
| *Säule:* | Hamilton PRP-1 250x10 mm | | |
| *Temperatur:* | 120°C isotherm | | |
| *Eluent:* | A: Wasser C: Ethanol | | |
| *Gradient:* | 0 min: | 100 % A | 0 % C |
| | 30 min: | 50 % A | 50 % C |
| | 50 min: | 0 % A | 100 % C |
| | 60 min: | 0 % A | 100 % C |
| *Detektion:* | ELSD (3.5 bar N2, 45 °C, Gain 6); | | |
| | DAD 210 nm, 250 nm, 280 nm, 320 nm | | |

### Analytische Daten:

| | **Eriolsäure A (7)** | | **Eriolsäure B (8)** | |
|---|---|---|---|---|
| | (400 MHz, CH3OD) | | (400 MHz, CH₃OD) | |
| **Pos.** | δ_{c}, **mult.** | δ_{H} **(*J* in Hz)** | δ_{c}, **mult.** | δ_{H} **(*J* in Hz)** |
| **1** | 129.6 | | 123.1 | |
| **2** | 131.0 | 7.72, s | 130.3 | 7.64, d (2.2) |
| **3** | 135.7 | | 128.8 | |
| **4** | 161.4 | | 158.2 | |
| **5** | 135.7 | | 129.2 | |
| **6** | 130.9 | 7.72, s | 130.3 | 7.63, d (2.2) |
| **7** | 170.6 | | 170.9 | |
| **8** | 28.8^{b} | 3.43, d (7.1) | 29.0 | 3.38, d (7.4) |
| **9** | 125.5 | 5.50, dd (7.2, 7.2) | 124.9 | 5.50, dd (7.4, 7.4) |
| **10** | 139.6 | | 139.6 | |
| **11** | 78.6 | 3.98, dd (7.0, 7.0) | 78.7 | 3.99, dd (7.0, 7.0) |
| **12** | 34.8 | 2.26, dd (7.0, 7.0) | 34.8 | 2.26, dd (7.0, 7.0) |
| **13** | 121.7 | 5.07, dd (7.0, 7.0) | 121.7 | 5.08, dd (7.0, 7.0) |
| **14** | 134.0 | | 134.0 | |
| **15** | 26.0 | 1.63, s | 26.0 | 1.64, s |
| **16** | 11.8 | 1.74, s | 11.6 | 1.72, s |
| **17** | 18.0 | 1.60, s | 17.9^{a} | 1.59, s |
| **18** | 28.9^{a} | 3.45, d (7.1) | 29.4 | 3.33, d (7.1) |
| **19** | 124.5 | 5.57, dd (7.3, 7.3) | 123.0 | 5.32, dd (7.3, 7.3) |
| **20** | 137.5 | | 134.1 | |
| **21** | 68.7 | 3.98, s | 26.0 | 1.76, s |
| **22** | 13.9 | 1.78, s | 18.0^{a} | 1.72, s |
| **23** | 61.6 | | | |

| | | | | |
|---|---|---|---|---|
| ^{a,b} austauschbare Signale | | | | |

| | **Eriolsäure C (9)** | | **Eriolsäure D (10)** | |
|---|---|---|---|---|
| | (400 MHz, CH3OD) | | (400 MHz, CH₃OD) | |
| **Pos.** | δ_{c}, **mult.** | δ_{H} **(*J* in Hz)** | δ_{c}, **mult.** | δ_{H} **(*J* in Hz)** |
| **1** | 123.4 | | 128.4 | |
| **2** | 130.4 | 7.65, s | 130.8 | 7.71, d (2.2) |
| **3** | 128.8 | | 135.5 | |
| **4** | 158.1 | | 161.3 | |
| **5** | 128.8 | | 136.1 | |
| **6** | 130.4 | 7.65, s | 130.8 | 7.70, d (2.2) |
| **7** | 171.1 | | 170.7 | |
| **8** | 29.1^{a} | 3.38, d (7.2) | 28.8 | 3.43, d (7.3) |
| **9** | 124.7 | 5.55, dd (7.4, 7.4) | 125.5 | 5.50, dd (7.2, 7.2) |
| **10** | 139.6 | | 139.5 | |
| **11** | 78.6 | 3.99, dd (7.1, 7.1) | 78.6 | 3.98, dd (7.0, 7.0) |
| **12** | 34.8 | 2.26, dd (7.1, 7.1) | 34.8 | 2.26, dd (7.0, 7.0) |
| **13** | 121.6 | 5.09, dd (7.1, 7.1) | 121.6 | 5.06, dd (7.0, 7.0) |
| **14** | 134.0 | | 133.9 | |
| **15** | 26.0 | 1.64, s | 26.0^{a} | 1.63, s |
| **16** | 11.6 | 1.72, s | 11.7 | 1.74, s |
| **17** | 18.0 | 1.60, s | 18.0^{b} | 1.59, s |
| **18** | 28.9^{a} | 3.40, d (7.4) | 29.2 | 3.38, d (7.3) |
| **19** | 124.1 | 5.61, dd (7.4, 7.4) | 123.7 | 5.28, dd (7.3, 7.3) |
| **20** | 137.4 | | 133.9 | |
| **21** | 68.8 | 3.99, s | 25.9^{a} | 1.75, s |
| **22** | 13.8 | 1.76, s | 17.9^{b} | 1.74, s |
| **23** | | | 61.5 | 3.76, s |

| | | | | |
|---|---|---|---|---|
| ^{a,b} austauschbare Signale | | | | |

### Beispiel 7

### Herstellung eines angereicherten Extrakts aus Eriodicyton californicum mittels Fällung

5.0 g eines analog zu Beispiel 5 hergestellten Extrakts von *E. californicum* wurden in 100 ml Ethylacetat gelöst. Durch Zugabe von 10 ml 3%iger Natronlauge unter Rühren im Eisbad wurde das enthaltene Homoeriodictyol ausgefällt. Die verbleibende Lösung wurde über Natriumsulfat getrocknet und aufkonzentriert. Das ebenfalls im Extrakt enthaltene Sterubin wurde durch Lagern des Extrakts im Kühlschrank über 12 Stunden ausgefällt. Nach Filtration wurde das Filtrat im Vakuum getrocknet und über Nacht im Hochvakuum-Trockenofen gelagert, um Restlösungsmittel zu entfernen. Der so gewonnene flavanoidabgereicherte Trocken-Extrakt enthielt erfindungsgemäß zu verwendende Benzoesäurederivate mit einem Anteil von 50%.

### Beispiel 8:

### Herstellung eines angereicherten Extrakts aus E. californicum mittels Gelpermeationschromatographie

Aus 0,5 g des methanolischen Extrakts von *E. californicum gemäß* Beispiel 5 wurden mit einer Flussrate von 2,5 ml/min über eine Sephadex-LH 20-Säule die Flavanoide abgetrennt, der verbleibende Extrakt unter Vakuum getrocknet und über Nacht im Hochvakuum-Trockenofen gelagert, um Restlösungsmittel zu entfernen. Der so gewonnene aufgereinigte Trocken-Extrakt hatte einen Gehalt von 75 % der erfindungsgemäß zu verwendenden Benzoesäurederivate.

| | |
|---|---|
| Stammlösung: | 0,5 g/ 20 ml Methanol |
| Säule: | Kronlab 3,5 × 60 cm |
| Säulenmaterial: | Sephadex LH-20 |
| Lösungsmittel: | Methanol |
| Flussrate: | 2,5 ml/min |
| Detektion (UV): | 210 nm |

### Beispiel 9:

### Herstellung eines angereicherten Extrakts mittels Gelpermeationschromatographie

Ein nach WO2004041804 dargestellter Extrakt, welcher durch die dort beschriebenen Verfahren bereits an Homoeriodictyol und Sterubin abgereichert wurde, wurde analog zu Beispiel 4 zur Abtrennung der restlichen Flavanoide über eine Sephadex LH-20 Säule fraktioniert, unter Vakuum getrocknet und über Nacht im Hochvakuum-Trockenofen gelagert, um Restlösungsmittel zu entfernen. Der so gewonnene flavanoidfreie Trocken-Extrakt hatte einen Gehalt von 62% der erfindungsgemäß zu verwendenden Benzoesäurederivate.

### Anwendungsbeispiele:

### Anwendungsbeispiel 1: Fettarmer Joghurt, gesüßt

| | Zubereitung (Angaben in Gew.-%) | | |
|---|---|---|---|
| Inhaltsstoff | A | B | C |
| Sucrose | 10% | 8% | 6% |
| Tagatose | - | - | 0,5% |
| Fructose | - | - | 0,05% |
| Hesperetin | - | 0,1% | 0,005% |
| Phloretin | - | - | 0,005% |
| Erdbeeraroma | - | 0,25% | - |
| Pfirsicharoma | 0,3% | - | 0,4% |
| Herba Santa Extrakt (*E. angustifolium*) nach Beispiel 1 (10% in Ethanol) | 0,4% | 0, 4 | |
| Angereicherter Herba Santa Extrakt (*E. angustifolium*) nach Bsp. 4 (10% in Ethanol) | | | 0, 1% |
| Joghurt, 0,1% Fett | Ad 100% | Ad 100% | Ad 100% |

### Anwendungsbeispiel 2: Fettarmer Joghurt, zuckerreduziert

| | Zubereitung (Angaben in Gew.-%) | | | |
|---|---|---|---|---|
| Inhaltsstoff | A | B | C | D |
| Tagatose | 0,482% | 0,482% | 0,482% | |
| Sucralose | 0,003% | 0,003% | 0,003% | |
| Aspartam | 0,005% | 0,005% | 0,005% | |
| Acesulfam K | 0,01% | 0,01% | 0,01% | |
| Rebaudioside A | | | | 0,005% |
| Rubus suavissimus Extrakt | | | | 0,005% |
| Hesperetin | | 0,01% | 0,005% | 0,005% |
| Phloretin | - | - | 0,005% | 0,005% |
| Erdbeeraroma | - | 0,25% | - | 0,25% |
| Himbeeraroma | 0,3% | - | 0,4% | |
| Herba Santa Extrakt (*E. californicum*) nach Bsp. 5 (10% in Ethanol) | 0,5% | | | 0,4% |
| Angereicherter Herba Santa Extrakt (*E. californicum*) nach Bsp. 8 (10% in Ethanol) | | 0, 1% | 0,1% | |
| Joghurt, 0,1% Fett | Ad 100% | Ad 100% | Ad 100% | Ad 100% |

### Anwendungsbeispiel 3: Fettarmer Joghurtdrink, gesüßt

| | Zubereitung (Angaben in Gew.-%) | | |
|---|---|---|---|
| Inhaltsstoff | A | B | C |
| Sucrose | 7% | 4% | 5% |
| Tagatose | - | - | 0,5% |
| Fructose | - | - | 0,05% |
| Hesperetin | - | 0,1% | 0,005% |
| Phloretin | - | - | 0,005% |
| Erdbeeraroma | - | 0,15 % | - |
| Rotfruchtaroma | 0,2% | - | 0,25% |
| Färbendes Lebensmittel Fruchtsaft | 5% | 5% | 5% |
| Herba Santa Extrakt (*E. angustifolium*) nach Beispiel 1 (10% in Ethanol) | 0, 35% | 0, 2% | |
| Angereicherter Herba Santa Extrakt (E. angustifolium) nach Bsp. 4 (10% in Ethanol) | | | 0,15% |
| Joghurt, 0,1% Fett | 60% | 60% | 60% |
| Wasser | Ad 100% | Ad 100% | Ad 100% |

### Anwendungsbeispiel 4: zuckerfreie Hartkaramelle

| Inhaltsstoff | A (Gew.-%) | A (Gew.-%) |
|---|---|---|
| Palatinit, Typ M | Ad 100% | Ad 100% |
| Wasser | 24,82% | 24,82% |
| Pfefferminzaroma | 0,15% | 0,05% |
| Hesperetin | | 0,10% |
| Trans-Pellitorin (10% in Ethanol) | 0,01% | |
| Herba Santa Extrakt (*E*. *angustifolium*) nach Bsp. 1 (10% in Ethanol) | 0,1 % | |
| Angereicherter Herba Santa Extrakt (*E. angustifolium*) nach Bsp. 4 (10% in Ethanol) | | 0,05% |

Palatinit wurde mit Wasser gemischt und die Mischung bei 165°C aufgeschmolzen und anschließend auf 115°C abgekühlt. Aroma und erfindungsgemäß hergestellter Extrakt, sowie trans-Pellitorin im Falle A und Hesperetin im Falle B, wurden zugegeben und nach dem Durchmischen in Formen gegossen, nach dem Erstarren aus den aus der Folie entfernt und anschließend einzeln verpackt.

### Anwendungsbeispiel 5: Schwarz-, Grün- oder Kräutertee

| Inhaltsstoff | Anteil in Gewichts% | | | | |
|---|---|---|---|---|---|
| | A | B | C | D | E |
| Schwarztee (Ceylon) Blätter | 99,4 | --- | --- | --- | --- |
| Grüntee (China), Blätter | --- | 99,2 | --- | --- | --- |
| Matetee (Peru), Blätter | --- | --- | 99,5 | --- | |
| Rooibostee (Südafrika), Blätter | --- | --- | --- | 99,6 | --- |
| Honeybushtee (Südafrika), Blätter | --- | --- | --- | --- | 99,6 |
| Herba Santa-Extrakt (*E. angustifolium*) nach Bsp. 1 | 0,3 | 0,8 | --- | 0,4 | 0,2 |
| Herba Santa-Extrakt (*E. californicum*) nach Bsp. 5 | 0,3 | --- | 0,5 | --- | 0,2 |

### Anwendungsbeispiel 6: Sojagetränk

| | Zubereitung (Angaben in Gew.-%) | | |
|---|---|---|---|
| Inhaltsstoff | A | B | C |
| Sucrose | 5% | 5% | 3,5% |
| Tagatose | - | - | 0,5% |
| Fructose | - | - | 0,05% |
| Hesperetin | - | - | 0,005% |
| Phloretin | - | - | 0,005% |
| Schokoladenaroma | - | 0,15% | - |
| Vanillearoma | 0,1% | - | 0,1% |
| Herba Santa Extrakt (*E. angustifolium*) nach Beispiel 1 (10% in Ethanol) | 0,08% | 0,1 | |
| Angereicherter Herba Santa Extrakt (E. californicum) nach Bsp. 8 (10% in Ethanol) | | | 0,05% |
| Ungesüßte Sojamilch-Base | Ad 100% | Ad 100% | Ad 100% |

### Anwendungsbeispiel 7: Fruchtiger Müsliriegel

| | |
|---|---|
| Saccharose | 15,992% |
| Glucosesirup | 14,0% |
| Sorbit P300 | 5,0% |
| Pflanzenfett | 5,0% |
| Wasser | 3,0% |
| Haferflocken | 7,3% |
| Haferflakes | 7,0% |
| Cornflakes | 4,5% |
| Rice Crispies | 15,0% |
| Korinthen | 3% |
| Getrocknete Heidelbeeren | 20% |
| Zitronensäurepulver | 0,2% |
| Angereicherter Herba Santa Extrakt nach Bsp. 4 | 0,008% |

### Anwendungsbeispiel 8: Kaugummi gegen schlechten Atem

| | I (%) | II (%) | III (%) | IV (%) |
|---|---|---|---|---|
| Kaugummi-Base | 21,00 | 21,00 | 21,00 | 21,00 |
| Glukosesirup | 16,80 | 16,80 | 16,50 | 16,50 |
| Glycerin | 0,50 | 0,50 | 0,50 | 0,50 |
| Puderzucker | 60,00 | 60,00 | 60,40 | 60,40 |
| Spearmint-Aroma | 1,50 | 1,50 | 1,50 | 1,50 |
| Herba Santa Extrakt (*E. angustifolium*) nach Bsp. 1 10% in Ethanol | 0,2 | | | |
| Herba Santa Extrakt (*E. californicum*) nach Bsp. 5 10% in Ethanol | | 0,2 | | |
| Angereicherter Extrakt aus *E. angustifolium* nach Bsp. 4 10% in Ethanol | | | 0,1 | |
| Angereicherter Extrakt aus *E. californicum* nach Bsp. 7 10% in Ethanol | | | | 0,1 |

### Anwendungsbeispiel 9: Zuckerfreier Kaugummi gegen schlechten Atem

| | I (%) | II (%) | III (%) | IV (%) |
|---|---|---|---|---|
| Kaugummi-Base | 30,00 | 30,00 | 30,00 | 30,00 |
| Sorbitol, Pulver | 38,25 | 38,25 | 38,40 | 38,40 |
| Palatinit | 9,50 | 9,50 | 9,50 | 9,50 |
| Xylitol | 2,00 | 2,00 | 2,00 | 2,00 |
| Mannitol | 3,00 | 3,00 | 3,00 | 3,00 |
| Aspartam | 0,10 | 0,10 | 0,10 | 0,10 |
| Acesulfam K | 0,10 | 0,10 | 0,10 | 0,10 |
| Emulgum / Emulgator | 0,30 | 0,30 | 0,30 | 0,30 |
| Sorbitol 70 %, in Wasser | 14,00 | 14,00 | 14,00 | 14,00 |
| Glycerin | 1,00 | 1,00 | 1,00 | 1,00 |
| Zimt/Menthol-Aroma | 1,50 | 1,50 | 1,50 | 1,50 |
| Herba Santa Extrakt | 0,25 | | | |
| (*E. angustifolium*) nach Bsp. 1 10% in Ethanol | | | | |
| Herba Santa Extrakt | | 0,2 | | |
| (*E. californicum*) nach Bsp. 5 10% in Ethanol | | | | |
| Angereicherter Extrakt aus | | | 0,1 | |
| *E. angustifolium* nach Bsp. 4 10% in Ethanol | | | | |
| Angereicherter Extrakt aus | | | | 0,1 |
| *E. californicum* nach Bsp. 7 10% in Ethanol | | | | |

### Anwendungsbeispiel 10: Fertige Mundspüllösung mit Fluorid gegen schlechten Atem

| | I (%) | II (%) | III (%) |
|---|---|---|---|
| Ethanol | 7,00 | 7,00 | 7,00 |
| Glycerin | 12,00 | 12,00 | 12,00 |
| Na fluorid | 0,05 | 0,05 | 0,05 |
| Pluronic F-127^{®} (BASF, surface-active substance) | 1,40 | 1,40 | 1,40 |
| Na phosphat Puffer pH 7.0 | 1,10 | 1,10 | 1,10 |
| Sorbinsäure | 0,20 | 0,20 | 0,20 |
| Na Saccharinat | 0,10 | 0,10 | 0,10 |
| Zimt-/Menthol-Aroma | 0,15 | 0,15 | 0,15 |
| Herba Santa Extrakt | 0,3 | | |
| (*E. angustifolium*) nach Bsp. 1 10% in Ethanol | | | |
| Herba Santa Extrakt | | 0,3 | |
| (*E. californicum*) nach Bsp. 5 10% in Ethanol | | | |
| Angereicherter Extrakt aus | | | 0,08 |
| *E. angustifolium* nach Bsp. 4 10% in Ethanol | | | |
| Farbstoff | 0,01 | 0,01 | 0,01 |
| Dest. water | to 100,00 | to 100,00 | to 100.00 |

### Anwendungsbeispiel 11: Mundspüllösung (Konzentrat) gegen schlechten Atem

| Inhaltsstoffe | I (%) | II (%) | III (%) |
|---|---|---|---|
| Ethanol, 95 % | 80,00 | 80,00 | 80,00 |
| Na Cyclamat | 0,15 | 0,15 | 0,15 |
| Eukalyptus/Wintergreen-Aroma | 3,50 | 3,50 | 3,50 |
| Farbstoff | 0,01 | 0,01 | 0,01 |
| Herba Santa Extrakt (*E. angustifolium*) nach Bsp. 1 | 0,50 | | |
| Angereicherter Extrakt aus *E. angustifolium* nach Bsp. 4 | | 0,1 | |
| Angereicherter Extrakt aus *E. californicum* nach Bsp. 7 | | | 0,1 |
| Demin. Wasser | Ad 100,00 | Ad 100,00 | Ad 100,00 |

### Anwendungsbeispiel 12: Mundspüllösung mit Fluorid gegen schlechten Atem

| **Inhaltsstoff** | **INCI** | **Gewichts %** |
|---|---|---|
| Ethylalkohol | Ethylalcohol | 10,00 |
| Cremophor CO 40 | Cremophor CO 40 (PEG 40 hydrogenated castor oil) | 1,00 |
| Benzoesäure | Benzoic acid | 0,12 |
| Aroma (PF1, PF2, PF3 oder PF4) | Flavour | 0,25 |
| Demin. Wasser | Water (deionized) | 83,28 |
| Sorbitol 70% | Sorbitol 70% | 5,00 |
| Natriumsaccharin | Sodiumsaccharin 450 | 0,07 |
| Natriumfluorid | Sodiumfluoride | 0,18 |
| Herba Santa Extrakt (*E. angustifolium*) nach Bsp. 1 (10% in Ethanol) | | 0,10 |

### Anwendungsbeispiel 13: Zahnpasta

| **Inhaltsstoff** | **INCI** | **Gewichts %** |
|---|---|---|
| Demin. Wasser | Water (deionized) | 26,31 |
| Sorbitol 70% | Sorbitol 70% | Sorbitol 70% |
| Solbrol M (Na Salz) | Solbrol M (Sodium salt) (Methylparaben) | 0,15 |
| Trisodiumphosphat | Trisodiumphosphate | 0,10 |
| Saccharin | Saccharin | 0,20 |
| Sodiummonofluorophosphat | Sodiummonofluorophosphate | 1,14 |
| PEG 1500 | PEG 1500 | 5,00 |
| Sident 9 (abrasives Kieselgel) | Sident 9 (abrasive silica) | 10,00 |
| Sident 22 S (Verdickungsmittel) | Sident 22 S (Thickening silica) | 8,00 |
| Sodiumcarboxymethylcellulose | Sodiumcarboxymethylcellulose | 1,10 |
| Titanium (IV) oxide | Titanium (IV) oxide | 0,50 |
| Sodiumlaurylsulfat (SLS) | Sodiumlaurylsulfate (SLS) | 1,50 |
| Aroma (PF1, PF2, PF3 or PF4) | Flavour | 1,00 |
| Angereicherter Herba Santa Extrakt (*E. californicum*) nach Bsp. 7 (10% in Ethanol) | | 0,40 |

### Anwendungsbeispiel 14: Zahncreme gegen Plaque

| **Inhaltsstoff** | **Gewichts %** | **Gewichts %** |
|---|---|---|
| Carrageenan | 0,90 | 0,90 |
| Glycerol | 15,00 | 15,00 |
| Sorbitol 70 %, in Wasser | 25,00 | 25,00 |
| PEG 1000 | 3,00 | 3,00 |
| Na fluorid | 0,24 | 0,24 |
| Tetrakaliumdiphosphat | 4,50 | 4,50 |
| Tetranatriumdiphosphat | 1,50 | 1,50 |
| Na saccarinate | 0,40 | 0,40 |
| Precipit. Kieselgel | 20,00 | 20,00 |
| Titaniumdioxid | 1,00 | 1,00 |
| Triclosan | 0,30 | 0,30 |
| PHB methylester | 0,10 | 0,10 |
| Spearmint flavor (enthaltend 60 wt.% L-Carvone und 25 wt.% L-Menthol) | 1,00 | 1,20 |
| Angereicherter Herba Santa Extrakt (*E. californicum*) nach Bsp. 7 (10% in Ethanol) | 0,30 | --- |
| Herba Santa Extrakt (*E*. *californicum*) nach Bsp. 1 (10% in Ethanol) | --- | 0,50 |
| Natriumdodecylsulfat | 1,30 | 1,30 |
| Demin. Wasser | Ad 100 | Ad 100 |

### Anwendungsbeispiel 15: Zahncreme gegen schmerzempfindliche Zähne

| **Inhaltsstoff** | **Gewichts %** | **Gewichts %** |
|---|---|---|
| Na Carboxymethylcellulose | 0.70 | 0.70 |
| Xanthan gum | 0.50 | 0.50 |
| Glycerol | 15.00 | 15.00 |
| Sorbitol 70 %, in Wasser | 12.00 | 12.00 |
| Kaliumnitrat | 5.00 | 5.00 |
| Natriummonofluorophosphat | 0.80 | 0.80 |
| PHB methylester | 0.15 | 0.15 |
| PHB propylester | 0.05 | 0.05 |
| Na Saccharinat | 0.20 | 0.20 |
| Flavor (PF1, PF2, PF3 oder PF4) | 1.00 | 1.00 |
| Herba Santa Extrakt (*E*. *angustifolium*) nach Bsp. 1 (10% in Ethanol) | 0.50 | --- |
| Herba Santa Extrakt (*E. californicum*) nach Bsp. 5 (10% in Ethanol) | --- | 0.25 |
| Ca-Carbonat | 35.00 | 35.00 |
| Silicondioxid | 1.00 | 1.00 |
| Sodiumdodecylsulfat (SDS) | 1.50 | 1.50 |
| Demin. Wasser | ad 100.00 | ad 100.00 |

### Anwendungsbeispiel 16: Gelatine Kapsel gegen schlechten Atem für direkten Verzehr

| | I (%) | II (%) | III (%) |
|---|---|---|---|
| Gelatine Hülle | | | |
| Glycerin | 2.014 | 2.014 | 2.014 |
| Gelatine 240 Bloom | 7.91 | 7.91 | 7.91 |
| Sucralose | 0.065 | 0.065 | 0.065 |
| Allura Red | 0.006 | 0.006 | 0.006 |
| Brilliant Blue | 0.005 | 0.005 | 0.005 |
| Kernfüllung: | | | |
| Pflanzenöl triglyceride | 82.00 | 74.00 | 60.00 |
| Aroma B | 7.9 | 15.50 | 29.5 |
| Herba Santa Extrakt (*E. angustifolium*) nach Bsp. 1 (10% in Ethanol) | 0.10 | 0.50 | - |
| Herba Santa Extrakt (*E. californicum*) nach Bsp. 5 (10% in Ethanol) | 0,10 | - | 0,50 |

Aroma B hatte die folgende Zusammensetzung (Angaben in Gewichts%): 0.1 % Neotame (Pulver), 0.05 % Aspartam, 29.3 % Pfefferminzöl (Avensis), 29.3 % Pfefferminzöl (Piperita; Willamette), 2.97 % Sucralose, 2.28 % Triacetin, 5.4 % Diethyltartrat, 12.1 % Pfefferminzöl (Yakima), 0.7 % Ethanol, 3.36 % 2-Hydroxyethylmenthylcarbonat, 3.0 % 2-Hydroxy-propylmenthylcarbonat, 0.27 % Vanillin, 5.5% D-Limonen, 5.67% L-Menthylacetat.

Die Gelatine Kapsel, die für den direkten Verzehr geeignet ist, hat einen Durchmesser von 5 mm; das Gewichtsverhältnis zwischen Kern- und Hüllmaterial liegt bei 90:10. Die Kapseln öffnen sich im Mund in weniger als 10 sec. und lösen sich vollständig innerhalb von 50 sec. auf.

### Anwendungsbeispiel 17: Syndet - Seifenfreies Waschstück

| **Inhaltsstoff** | **INCI Name** | **Gewichts %** | **Gewichts %** |
|---|---|---|---|
| Zetesap 813 A | Disodium Lauryl Sulfosuccinate, Sodium Lauryl Sulfate, Corn Starch, Cetearyl Alcohol, Paraffin, Titanium Dioxide | 92,0 | 91,9 |
| Amphotensid GB 2009 | Disodium Cocoamphodiacetate | 6,0 | 6,0 |
| Allantoin | Allantoin | 1,0 | 1,0 |
| Parfümöl P2, P4, P6 oder P7 | Fragrance | 1,0 | - |
| Perfümöl P1, P3 oder P5 | Fragrance | - | 1,0 |
| Herba Santa Extrakt (*E. angustifolium*) nach Beispiel 1 (10 % in Ethanol) | | 0,5 | - |
| Angereicherter Herba Santa Extrakt (*E*. *angustifolium*) nach Beispiel 4 (10 % in Ethanol) | | - | 0,1 |

### Anwendungsbeispiel18: Seifenstück

| **Inhaltsstoff** | **INCI** | **Gewichts %** | **Gewichts %** |
|---|---|---|---|
| Demin. Wasser | Water | 2,5 | 2,5 |
| Seifenbasen-Mix | Sodium tallowates / palmitates | 95,5 | 95,8 |
| Titaniumdioxid | Titanium dioxide | 1,0 | 1,0 |
| Parfümöl P2, P4, P6 oder P7 | Fragrance | 0,8 | - |
| Perfümöl P1, P3 oder P5 | Fragrance | - | 0,5 |
| Angereicherter Herba Santa Extrakt (*E. angustifolium*) nach Beispiel 4 (10 % in Ethanol) | | 0,2 | - |
| Angereicherter Herba Santa Extrakt (*E. californicum*) nach Beispiel 7 (10 % in Ethanol) | - | 0,2 | |

### Anwendungsbeispiel 19: Antimikrobielles Seifenstück

| **Inhaltsstoff** | **Gewichts %** | **Gewichts %** |
|---|---|---|
| Natriumseife aus Talg | 60,0 | 60,0 |
| Natriumseife aus Palmöl | 27,0 | 27,0 |
| Glycerol | 2,0 | 2,0 |
| Natriumchlorid | 0,5 | 0,5 |
| 1-Hydroxyethan-1,1-diphosphorsäure, Tetranatriumsalz | 0,3 | 0,3 |
| Alpha-Tocopherol | 0,1 | 0,1 |
| Pigment Gelb 1 | 0,02 | 0,02 |
| Wasser | Ad 100 | Ad 100 |
| Parfümöl P2, P4, P6 or P7 | 3,0 | - |
| Parfümöl P1, P3 or P5 | - | 3,0 |
| Herba Santa Extrakt (*E*.) nach Beispiel 1 (10 % in Ethanol) | 0,5 | |
| Angereicherter Herba Santa Extrakt (*E. angustifolium*) nach Beispiel 7 (10 % in Ethanol) | - | 0,2 |

### Anwendungsbeispiel 20: Flüssigseife

| **Inhaltsstoff** | **INCI Name** | **Gewichts %** | **Gewichts %** |
|---|---|---|---|
| Tagat O 2 | PEG-20 Glyceryl Oleate | 2,5 | 2,5 |
| Kokosfettsäure diethanolamid | Cocamide DEA | 5,0 | 5,0 |
| Abil B 8842 | Cyclomethicone | 0,5 | 0,5 |
| Sodium laurylethersulfat, 28% | Sodium Laureth Sulfate | 35,0 | 35,0 |
| Tego-Betaine L7 | Cocamidopropyl Betaine | 5,0 | 5,0 |
| Seife, 25% | Coconut acid, Potassium salt, Potassium Oleate | 20,0 | 20,0 |
| Wasser | Water | Ad 100 | Ad 100 |
| Konservierungsmittel | DMDM Hydantoin | | |
| Parfümöl P2, P4, P6 oder P7 | Fragrance | 0,4 | - |
| Parfümöl P1, P3 oder P5 | Fragrance | - | 0,4 |
| Angereicherter Herba Santa Extrakt (*E. angustifolium*) nach Bsp. 4 (10% in Ethanol) | | 0,4 | 0,3 |

### Anwendungsbeispiel 21: Flüssigseife (Syndet)

| **Inhaltsstoffe** | **INCI Name** | **Gewichts %** | **Gewichts %** |
|---|---|---|---|
| Elfan OS 46 | Sodium Olefin C14-C16 Sulfonate | 35,5 | 35,5 |
| Armoteric LB | Lauryl Betaine | 8,0 | 8,0 |
| Elfan SG | | 10,0 | 10,0 |
| Elfacos GT 282 L | Talloweth-60 Myristyl Glycol | 3,0 | 3,0 |
| PCL-Liquid 100 | Cetearyl Ethylhexanoate | 4,0 | 4,0 |
| Wasser | Water | Ad 100 | Ad 100 |
| Konservierungsmittel | Methylchloroisothiazolinone, Methylisothiazinone | | |
| Parfümöl P2, P4, P6 oder P7 | Fragrance | 0,4 | - |
| Parfümöl P1, P3 oder P5 | Fragrance | - | 0,4 |
| Herba Santa Extrakt (*E. angustifolium*) nach Bsp. 1 (10% in Ethanol) | 0,5 | | |
| Herba Santa Extrakt (*E. californicum*) nach Bsp. 5 (10% in Ethanol) | | 0,3 | |

### Anwendungsbeispiel 22: Shampoo

| **Inhaltsstoffe** | **Gewichts %** | **Gewichts %** |
|---|---|---|
| Natriumlauryl ether sulfat (z.B. Texapon NSO) | 12 | 12 |
| Cocamidopropylbetain (z.B. Dehyton K) | 2 | 2 |
| Natriumchlorid | 1,4 | 1,4 |
| Zitronensäure | 1,3 | 1,3 |
| Phenoxyethanol, Methyl-, Ethyl-, Butyl- und Propylparaben | 0,5 | 0,5 |
| Parfümöl P2, P4, P6 oder P7 | 0,3 | - |
| Parfümöl P1, P3 oder P5 | - | 0,3 |
| Herba Santa Extrakt (*E. californicum*) nach Bsp. 5 (10% in Ethanol) | 0,25 | - |
| Herba Santa Extrakt (*E. californicum*) nach Bsp. 7 (10% in Ethanol) | | 0,15 |
| Wasser | Ad 100 | Ad 100 |

### Anwendungsbeispiel 23: 2 in 1 - Shampoo

| **Inhaltsstoffe** | **INCI Name** | **Gewichts %** | **Gewichts %** |
|---|---|---|---|
| Demineralisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butylparaben, Propylparaben, Isobutylparaben | 0,5 | 0,5 |
| Natriumchlorid | Sodium Chloride | 1,4 | 1,4 |
| Zitronensäure Monohydrat (kristallin) | Citric acid | 0,1 | 0,1 |
| Parfümöl P2, P4, P6 oder P7 | Fragrance | 0,5 | - |
| Parfümöl P1, P3 oder P5 | Fragrance | - | 0,5 |
| Angereicherter Herba Santa Extrakt (*E. angustifolium*) nach Bsp. 4 (10% in Ethanol) | | 0,15 | - |
| Herba Santa Extrakt (*E. californicum*) nach Bsp. 8 (10% in Ethanol) | | - | 0,15 |

### Anwendungsbeispiel 24: Anti-Schuppen Shampoo

| Inhaltsstoffe | (INCI Name) | Gewichts % | Gewichts % |
|---|---|---|---|
| Climbazol | Climbazole | 0,50 | 0,50 |
| Phenoxyethanol, Methylparaben, Ethylparaben, Butylparaben, Propylparaben, Isobutylparaben | Phenoxyethanol, Methylparaben, Ethylparaben, Butylparaben, Propylparaben, Isobutylparaben | 0,70 | 0,70 |
| Herba Santa Extrakt (*E. angustifolium*) nach Bsp. 1 (10% in Ethanol) | | 0,60 | - |
| Angereicherter Herba Santa Extrakt (*E. angustifolium*) nach Bsp. 4 (10% in Ethanol) | | - | 0,40 |
| Natriumlaureth Sulfat | Sodium Laureth Sulfate | 37,00 | 37,00 |
| Cocamidopropyl Betain | Cocamidopropyl Betaine | 8,00 | 8,00 |
| PEG-6 | PEG-6 Caprylic/Capric Glycerides | 2,50 | 2,50 |
| Laureth -2 | Laureth-2 | 2,00 | 2,00 |
| Thymian-Extrakt | Water (Aqua), Glycerol, Thymus Vulgaris (Thyme), Flower/Leaf Extract | 0,50 | 0,50 |
| Rosmarin-Extrakt | Rosmarinus Officinalis (Rosemary) Leaf Water, Water (Aqua), Butylene Glycol, Pentylene Glycol | 0,50 | 0,50 |
| Bisabolol | Bisabolol | 0,10 | 0,10 |
| Panthenol | Panthenol | 0,50 | 0,50 |
| Parfümöl P2, P4, P6 oder P7 | Fragrance | 0,50 | - |
| Parfümöl P1, P3 oder P5 | Fragrance | - | 0,50 |
| Wasser | Water (Aqua) | 46,30 | 46,30 |
| Polyquaternium-10 | Polyquaternium-10 | 0,40 | 0,40 |

### Anwendungsbeispiel 25: Duschgel

| **Inhaltsstoffe** | **INCI** | **Gewichts %** | **Gewichts %** |
|---|---|---|---|
| Demin. Wasser | Water | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butylparaben, Propylparaben, Isobutylparaben | 0,5 | 0,5 |
| Natriumchlorid | Sodium Chloride | 1,4 | 1,4 |
| Zitronensäure Monohydrat (kristallin) | Citric Acid | 1,3 | 1,3 |
| Parfümöl P2, P4, P6 oder P7 | Fragrance | 0,6 | - |
| Parfümöl P1, P3 oder P5 | Fragrance | - | 0,6 |
| Herba Santa Extrakt (*E. angustifolium*) nach Beispiel 1 (10% in Ethanol) | | 0,1 | |
| Angereicherter Herba Santa Extrakt (*E. angustifolium*) nach Beispiel 4 (10% in Ethanol) | | | 0,05 |

### Anwendungsbeispiel 26: Rasierschaum

| **Inhaltsstoffe** | **Gewichts %** | **Gewichts %** |
|---|---|---|
| Demin. Wasser | 77,2 | 77,22 |
| Triethanolamin | 4,0 | 4,0 |
| Edenor L2 SM (Stearinsäure, Palmitinsäure) (Cognis) | 5,3 | 5,3 |
| Laureth-23 | 3,0 | 3,0 |
| Stearylalkohol | 0,5 | 0,5 |
| euxyl® K220 (Methylisothiazolinon, Ethylhexylglycerol) | 0,8 | 0,8 |
| Natriumlaurylsulfate | 3,0 | 3,0 |
| Extrapone Seaweed/ Alge (water, propylene glycol, potassium iodide, Fucus Vesiculosus Extract) | 1,0 | 1,0 |
| Dragosantol (Bisabolol, Farnesol) | 0,1 | 0,1 |
| Parfümöl P2, P4, P6 oder P7 | 1,0 | - |
| Parfümöl P1, P3 oder P5 | - | 1 |
| Herba Santa Extrakt (*E. californicum*) nach Beispiel 5 (10 % in Ethanol) | 0,1 | - |
| Angereicherter Herba Santa Extrakt (*E. californicum*) nach Beispiel 8 (10 % in Ethanol) | - | 0,08 |
| Propan, Butan 4,2 Bar | 4,0 | 4,0 |

### Anwendungsbeispiel 27: Aftershave

| **Inhaltsstoffe** | **INCI** | **Gewichts %** | **Gewichts %** |
|---|---|---|---|
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 3,00 | 3,00 |
| SymSitive® 1609 | Pentylene Glycol, 4-t-Butylcyclohexanol | 1,00 | 1,00 |
| Frescolat® ML | Menthyl Lactate | 0,30 | 0,30 |
| Glycerol 99,5 P. | Glycerol | 5,00 | 5,00 |
| Wasser | Water (Aqua) | Ad 100 | Ad 100 |
| Extrapone® Glacier Water GW | Glycerol, Water (Aqua) | 1,00 | 1,00 |
| SymCalmin® | Butylene Glycol, Pentylene Glycol, Hydroxyphenyl Propamidobenzoic Acid | 0,50 | 0,50 |
| Dragosine® | Carnosine | 0,10 | 0,10 |
| Hydrolite® 5 | Pentylene Glycol | 5,00 | 5,00 |
| Ethanol 96 % | Alcohol Denat. | 5,00 | 5,00 |
| Farbpigment | Colour Pigment | 0,05 | 0,05 |
| Parfümöl P2, P4, P6 oder P7 | Parfum | 0,15 | - |
| Parfümöl P1, P3 oder P5 | Parfum | - | 0,15 |
| Herba Santa Extrakt (*E. angustifolium*) nach Beispiel 1 (10 % in Ethanol) | | 0,15 | - |
| Angereicherter Herba Santa Extrakt (*E.* a*ngustifolium*) nach Beispiel 4 (10 % in Ethanol) | | - | 0,08 |

### Anwendungsbeispiel 28: Deodorant Formulierung (Roll-on Gel)

| **Inhaltsstoffe** | **Gewichts %** | **Gewichts %** |
|---|---|---|
| 1,3-Butyleneglycol | 2,00 | 2,00 |
| PEG-40-hydrogen. Rizinusöl | 2,00 | 2,00 |
| Hydroxyethylcellulose | 0,50 | 0,50 |
| Konservierungsmittel (Phenoxyethanol) | 0,30 | 0,30 |
| Parfümöl P2, P4, P6 oder P7 | 0,30 | - |
| Parfümöl P1, P3 oder P5 | - | 0,30 |
| Herba Santa Extrakt (*E. angustifolium*) nach Beispiel 1 (10 % in Ethanol) | 0,30 | - |
| Angereicherter Herba Santa Extrakt (*E. angustifolium*) nach Beispiel 4 (10 % in Ethanol) | - | 0,10 |
| Wasser | ad 100,00 | ad 100,00 |

### Anwendungsbeispiel 29: Deostift

| **Inhaltsstoffe** | **Gewichts %** | **Gewichts %** |
|---|---|---|
| Natriumstearat | 8,00 | 8,00 |
| PPG-3 Myristylether | 70,00 | 70,00 |
| 1,2-Propylenglycol | 10,00 | 10,00 |
| 1,1-Dimethyl-3-phenylpropanol | 0,20 | 0,25 |
| 2-Butyloctansäure | 0,20 | 0,20 |
| Parfümöl P2, P4, P6 oder P7 | 0,60 | - |
| Parfümöl P1, P3 oder P5 | - | 0,60 |
| Herba Santa Extrakt (*E. angustifolium*) nach Beispiel 1 (10 % in Ethanol) | 0,30 | - |
| Angereicherter Herba Santa Extrakt (*E. californicum*) nach Beispiel 7 (10 % in Ethanol) | - | 0,20 |
| Wasser | Ad 100 | Ad 100 |

### Anwendungsbeispiel 30:Antitranspirantien

| **Inhaltsstoffe** | **Gewichts %** | **Gewichts %** |
|---|---|---|
| Reach AZP-908 SUF | 24,00 | 22,00 |
| Cyclomethicon (Pentamer) | Ad 100 | Ad 100 |
| Polydecen (Silkflo 364 NF) | 17,50 | 20,00 |
| Neo Heliopan OS (Ethylhexylsalicylat) | 2,50 | 1,00 |
| L-Menthyllactat (Frescolate ML) | 0,25 | - |
| Polyethylen | 3,00 | 3,00 |
| Hydrogen. Rizinusöl | 2,00 | 2,00 |
| Promyristyl PM-3 | 7,00 | 7,00 |
| PEG-8 Distearat | 3,00 | 3,00 |
| Silicondioxid (Cab-O-Sil M-5) | 1,00 | 1,00 |
| Stearylalkohol | 15,00 | 10,00 |
| Octyldodecanol | - | 8,00 |
| Parfümöl P2, P4, P6 oder P7 | 0,80 | - |
| Parfümöl P1, P3 oder P5 | - | 0,80 |
| Herba Santa Extrakt (*E. angustifolium*) nach Beispiel 1 (10 % in Ethanol) | 0,30 | - |
| Angereicherter Herba Santa Extrakt (*E. angustifolium*) nach Beispiel 4 (10 % in Ethanol) | - | 0,30 |

### Anwendungsbeispiel 31: O/W Lotion

| **Inhaltsstoffe** | **INCI** | **Gewichts %** | **Gewichts %** |
|---|---|---|---|
| Paraffinöl | Paraffin oil | 5,00 | 5.00 |
| Isopropylpalmitat | Isopropyl palmitate | 5,00 | 5,00 |
| Cetylalkohol | Cetyl alcohol | 2,00 | 2,00 |
| Bienenwachs | Beeswax | 2,00 | 2,00 |
| Ceteareth-20 | Ceteareth-20 | 2,00 | 2,00 |
| PEG-20 Glyceryl stearat | PEG-20-glyceryl stearate | 1,50 | 1,50 |
| Glycerol | Glycerol | 3,00 | 3,00 |
| Parfümöl P2, P4, P6 oder P7 | Perfume | 0,30 | - |
| Parfümöl P1, P3 oder P5 | Perfume | - | 0,30 |
| Herba Santa Extrakt (*E. californicum*) nach Beispiel 5 (10 % in Ethanol) | | 0,25 | - |
| Angereicherter Herba Santa Extrakt (*E*. *californicum*) nach Beispiel 7 (10 % in | | - | 0,10 |
| Ethanol) | | | |
| Methylparaben | Methylparabene | 0,30 | 0,30 |
| Wasser | Water | Ad 100,00 | Ad 100,00 |

### Anwendungsbeispiel 32: Bodylotion

| **Inhaltsstoffe** | **INCI** | **Gewichts %** | **Gewichts %** |
|---|---|---|---|
| Cetearylalkohol | Cetearyl Alcohol | 2,00 | 2,00 |
| Ethylhexylisononanoat | Ethylhexyl Isononanoate | 5,00 | 5,00 |
| Cetearylethylhexanoat, Isopropylmyristat | Cetearyl Ethylhexanoate, Isopropyl Myristate | 3,00 | 3,00 |
| Glyceryloleatcitrat, Caprin-/Capryltriglycerid | Glyceryl Oleate Citrate, Caprylic/Capric Triglyceride | 4,00 | 4,00 |
| Wasser | Water (Aqua) | 79,50 | 79,50 |
| Carbomer | Carbomer | 0,30 | 0,30 |
| Natriumbenzoat | Sodium Benzoate | 0,10 | 0,10 |
| Propyleneglykol | Propylene Glycol | 5,00 | 5,00 |
| Triethylenglykol, Imidazolidinylharnstoff, Methylparaben, Propylparaben, Dehydroessigsäure | Triethylene Glycol, Imidazolidinyl Urea, Methylparaben, Propylparaben, Dehydroacetic Acid | 0,30 | 0,30 |
| Natriumhydroxyd Lsg. (30%) | Sodium Hydroxide 30% solution | 0,30 | 0,30 |
| Parfümöl P2, P4, P6 oder P7 | Perfume | 0,30 | - |
| Parfümöl P1, P3 oder P5 | Perfume | - | 0,30 |
| Herba Santa Extrakt (*E. angustifolium*) nach Beispiel 1 (10 % in Ethanol) | | 0,10 | - |
| Herba Santa Extrakt (*E. californicum*) nach Beispiel 5 (10 % in Ethanol) | | - | 0,10 |

### Anwendungsbeispiel 32: Hand- und Körpercreme:

| **Inhaltsstoffe** | **INCI** | **Gewichts %** | **Gewichts %** |
|---|---|---|---|
| Dracorin® GOC | Glyceryl Oleate Citrate, Caprylic/Capric Triglyceride | 2,00 | 2,00 |
| PCL-Solid | Stearyl Heptanoate, Stearyl Caprylate | 2,50 | 2,50 |
| Lanette® O | Cetearyl Alcohol | 1,50 | 1,50 |
| Cutina® GMS-V | Glyceryl Stearate | 1,00 | 1,00 |
| Dragoxat® 89 | Ethylhexyl Isononanoate | 3,00 | 3,00 |
| PCL-Liquid 100 | Cetearyl Ethylhexanoate | 7,00 | 7,00 |
| Isodragol® | Triisononanoin | 4,00 | 4,00 |
| Xiameter® PMX-0345 Cyclosiloxan | Cyclopentasiloxane (and) Cyclohexasiloxane | 0,50 | 0,50 |
| Wasser | Water (Aqua) | Ad 100 | Ad 100 |
| Carbopol® Ultrez 21 | Acrylates/C10-30 Alkyl Acrylate Crosspolymer | 0,20 | 0,20 |
| Keltrol® CG-RD | Xanthan Gum | 0,10 | 0,10 |
| Glycerol 85 P. | Glycerol | 3,00 | 3,00 |
| DragoBetaGlucan | Water (Aqua), Butylene Glycol, Glycerol, Avena Sativa (Oat) Kernel Extract | 1,50 | 1,50 |
| Kaliumsorbat | Potassium Sorbate | 0,10 | 0,10 |
| euxyl® K300 | Methyl-, Butyl-, Ethyl-, Propyl, Isobutylparaben, Phenoxyethanol. | 0,80 | 0,80 |
| Natriumhydroxid 10% Lösung | Sodium Hydroxide | 0,50 | 0,50 |
| Parfümöl P2, P4, P6 oder P7 | Parfum | 0,20 | - |
| Parfümöl P1, P3 oder P5 | Parfum | - | 0,20 |
| Herba Santa Extrakt (*E. angustifolium*) nach Beispiel 1 (10 % in Ethanol) | | 0,20 | -- |
| Angereicherter Herba Santa Extrakt (*E*. *angustifolium*) nach Beispiel 4 (10 % in Ethanol) | | - | 0,15 |

### Anwendungsbeispiel 33: Gesichtscreme

| **Inhaltsstoffe** | **INCI** | **Gewichts %** | **Gewichts %** |
|---|---|---|---|
| Emulsiphos® | Potassium Cetyl Phosphate, Hydro-genated Palm Glycerides | 1,50 | 1,50 |
| Cutina® GMS-V | Glyceryl Stearate | 1,70 | 1,70 |
| Lanette® O | Cetearyl Alcohol | 3,00 | 3,00 |
| Tegosoft® MM | Myristyl Myristate | 1,00 | 1,00 |
| PCL-Liquid 100 | Cetearyl Ethylhexanoate | 1,00 | 1,00 |
| Isodragol® | Triisononanoin | 3,00 | 3,00 |
| Dragoxat® 89 | Ethylhexyl Isononanoate | 4,00 | 4,00 |
| Avocadoöl | Persea Gratissima (Avocado) Oil | 3,00 | 3,00 |
| Abil® 350 | Dimethicone | 0,50 | 0,50 |
| Covi-ox® T-70 | Tocopherol | 0,10 | 0,10 |
| Edeta® BD | Disodium EDTA | 0,10 | 0,10 |
| Carbopol® Ultrez 21 | Acrylates/C10-30 Alkyl Acrylate Crosspolymer | 0,30 | 0,30 |
| Keltrol® CG-RD | Xanthan Gum | 0,15 | 0,15 |
| Wasser | Water (Aqua) | Ad 100 | Ad 100 |
| Glycerol 99,5 P. | Glycerol | 4,00 | 4,00 |
| Propylenglycol -1,2 99 P GC | Propylene Glycol | 3,00 | 3,00 |
| Euxyl® K712 | Sodium Benzoate, Potassium Sorbate | 0,80 | 0,80 |
| Symmatrix® | Maltodextrin, Rubus Fruticosus (Blackberry) Leaf Extract | 0,50 | 0,50 |
| Natriumhydroxid 10% solution | Sodium Hydroxide | 0,50 | 0,50 |
| Parfümöl P2, P4, P6 oder P7 | Parfum | 0,30 | - |
| Parfümöl P1, P3 oder P5 | Parfum | - | 0,30 |
| Angereicherter Herba Santa Extrakt (*E*. *angustifolium*) nach Beispiel 4 (10 % in Ethanol) | | 0,10 | - |
| Angereicherter Herba Santa Extrakt (*E*. *californicum*) nach Beispiel 8 (10 % in Ethanol) | | - | 0,10 |

### Anwendungsbeispiel 34: Antifaltencreme

| **Inhaltsstoffe** | **Gewichts %** | **Gewichts %** |
|---|---|---|
| Glycerylstearat Citrat | 1,00 | 1,00 |
| Glyceryllaurat | 1,00 | 1,00 |
| Cetearylalcohol, | 2,00 | 2,00 |
| Myristylmyristat | 1,00 | 1,00 |
| Cetearylethylhexanoat | 4,00 | 4,00 |
| Mineralöl | 4,00 | 4,00 |
| Cyclopentasiloxan, Cyclohexasiloxan | 0,50 | 0,50 |
| Acrylate/C10-30 Alkylarylate Crosspolymer | 0,20 | 0,20 |
| Konservierungsstoff (Phenoxyethanol) | 1,00 | 1,00 |
| Wasser | Ad 100 | Ad 100 |
| Xanthan Gum | 0,10 | 0,10 |
| 1,2-Hexanediol | 2,00 | 2,00 |
| Natriumhydroxid 10% Lsg | 0,10 | 0,10 |
| Narcissus Tazetta Extrakt | 1,00 | 1,00 |
| Parfümöl P2, P4, P6 oder P7 | 0,30 | - |
| Parfümöl P1, P3 oder P5 | - | 0,30 |
| Angereicherter Herba Santa Extrakt (*E*. *angustifolium*) nach Beispiel 4 (10 % in Ethanol) | 0,10 | - |
| Angereicherter Herba Santa Extrakt (*E*. *californicum*) nach Beispiel 8 (10 % in Ethanol) | - | 0,10 |

### Anwendungsbeispiel 35: Wasch- und Reinigungsgel

| **Inhaltsstoff** | **INCl** | **Gewichts %** | **Gewichts %** |
|---|---|---|---|
| Wasser | Water (Aqua) | Ad 100 | Ad 100 |
| Pionier® NP 37 G | Sodium Carbomer | 1,50 | 1,50 |
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 5,00 | 5,00 |
| Hydroviton® 24 | Water (Aqua), Pentylene Glycol, Glycerol, Sodium Lactate, Lactic Acid, Serine, Urea, Sorbitol, Sodium Chloride, Allantoin | 1,00 | 1,00 |
| Extrapone® Silk GW | Water (Aqua), Glycerol, Hydrolyzed Silk | 1,00 | 1,00 |
| Hydrolite® 5 | Pentylene Glycol | 4,00 | 4,00 |
| Konservierungsmittel | Phenoxyethanol | | |
| Actipearls Red Star # DH 10402/6 | Water (Aqua), Propylene Glycol, Algin, Gellan Gum, Xanthan Gum, CalciumChloride, Cl 12490 (Pigment Red 5), Mica (Cl 77019), Titanium Dioxide (Cl 77891) | 1,00 | 1,00 |
| Parfümöl P2, P4, P6 oder P7 | Parfum | 0,50 | - |
| Parfümöl P1, P3 oder P5 | Parfum | - | 0,50 |
| Herba Santa Extrakt Extrakt (*E. angustifolium*) nach Beispiel 1 (10 % in Ethanol) | | 0,2 | - |
| Angereicherter Herba Santa Extrakt (*E. californicum*) nach Beispiel 7 (10 % in Ethanol) | | - | 0,25 |

### Anwendungsbeispiel 36: Sonnenschutzspray

| **Inhaltsstoff** | **INCI** | **Gewichts %** | **Gewichts %** |
|---|---|---|---|
| Demineralisiertes Wasser | Water (aqua) | 69,40 | 69,40 |
| Glycerol | Glycerol | 4,00 | 4,00 |
| 1,3 Butylenglycol | Butylene glycol | 5,00 | 5,00 |
| D-Panthenol | Panthenol | 0,50 | 0,50 |
| Lara Care A-200 | Galactoarabinan | 0,25 | 0,25 |
| Baysilonöl M 10 | Dimethicone | 1,00 | 1,00 |
| Edeta BD | Disodium EDTA | 0,10 | 0,10 |
| Copherol 1250 | Tocopheryl acetate | 0,50 | 0,50 |
| Cetiol OE | Dicaprylyl ether | 3,00 | 3,00 |
| Neo Heliopan^{®} HMS | Homosalate | 5,00 | 5,00 |
| Neo Heliopan^{®} AV | Ethylhexyl methoxycinnamate | 6,00 | 6,00 |
| Neo Heliopan^{®} 357 | Butyl methoxydibenzoylmethane | 1,00 | 1,00 |
| Corapan TQ | Diethylhexylnaphthalate | 2,00 | 2,00 |
| Alpha Bisabolol | Bisabolol | 0,10 | 0,10 |
| Pemulen TR-2 | Acrylates/C10-30 alkyl acrylate crosspolymer | 0,25 | 0,25 |
| Phenoxyethanol | Phenoxyethanol | 0,70 | 0,70 |
| Solbrol M | Methylparaben | 0,20 | 0,20 |
| Solbrol P | Propylparaben | 0,10 | 0,10 |
| NaOH, 10% | Sodium hydroxide | 0,60 | 0,60 |
| Parfümöl P2, P4, P6 oder P7 | Fragrance | 0,20 | - |
| Parfümöl P1, P3 oder P5 | Fragrance | - | 0,20 |
| Angereicherter Herba Santa Extrakt (*E. californicum*) nach Beispiel 8 (10 % in Ethanol) | | 0,10 | - |
| Angereicherter Herba Santa Extrakt (*E. angustifolium*) nach Beispiel 4 (10 % in Ethanol) | | - | 0,10 |

### Anwendungsbeispiel 37: Sonnenschutzmilch (W/O)

| **Inhaltsstoffe** | **INCI** | **Gewichts %** | **Gewichts %** |
|---|---|---|---|
| Dehymuls PGPH | Polyglyceryl-2 dipolyhydroxystearate | 3,00 | 3,00 |
| Bienenwachs 8100 | Beeswax | 1,00 | 1,00 |
| Monomuls 90-0-18 | Glyceryl oleate | 1,00 | 1,00 |
| Zinkstearat | Zinc stearate | 1,00 | 1,00 |
| Cetiol SN | Cetearyl isononanoate | 5,00 | 5,00 |
| Cetiol OE | Dicaprylyl ether | 5,00 | 5,00 |
| Tegosoft TN | C12-15 alkyl benzoate | 4,00 | 4,00 |
| Vitamin E | Tocopherol | 0,50 | 0,50 |
| Neo Heliopan^{®} OS | Ethylhexyl salicylate | 5,00 | 5,00 |
| Neo Heliopan^{®} AV | Ethylhexyl methoxycinnamate | 7,50 | 7,50 |
| Uvinul^{®} T150 | Ethylhexyl triazone | 1,50 | 1,50 |
| Demin. Wasser | Water (Aqua) | To 100 | To 100 |
| Trilon BD | Disodium EDTA | 0,10 | 0,10 |
| Glycerol | Glycerol | 5,00 | 5,00 |
| Solbrol M | Methylparaben | 0,20 | 0,20 |
| Phenoxyethanol | Phenoxyethanol | 0,70 | 0,70 |
| Solbrol P | Propylparaben | 0,10 | 0,10 |
| Neo Heliopan^{®} AP 10% Lösung, neutralisiert mit NaOH | Disodium phenyl dibenzimidazole tetrasulfonate | 15,00 | 15,00 |
| Parfümöl P2, P4, P6 oder P7 | Parfum | 0,25 | - |
| Parfümöl P1, P3 oder P5 | Parfum | - | 0,25 |
| Herba Santa Extrakt (*E. angustifolium*) nach Beispiel 1 (10 % in Ethanol) | | 0,15 | - |
| Angereicherter Herba Santa Extrakt (*E. angustifolium*) nach Beispiel 4 (10 % in Ethanol) | | - | 0,1 |
| Alpha bisabolol | Bisabolol | 0,10 | 0,10 |

### Anwendungsbeispiel 37: After-Sun Gel

| **Inhaltsstoffe** | **INCl** | **Gewichts %** | **Gewichts %** |
|---|---|---|---|
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 3,000 | 3,000 |
| Glycerol 99,5 P. | Glycerol | 5,000 | 5,000 |
| SymHelios® 1031 | Benzylidene Dimethoxydimethylin danone | 0,100 | 0,100 |
| Wasser | Water (Aqua) | Ad 100 | Ad 100 |
| Pemulen® TR-2 | Acrylates/C10-30 Alkyl Acrylate Crosspolymer | 1,000 | 1,000 |
| D-Panthenol 75 W | Panthenol | 0,500 | 0,500 |
| SymFinity® 1298 | Echinacea Purpurea Extract | 0,100 | 0,100 |
| Extrapone® Pearl GW | Water (Aqua), Glycerol, Hydrolyzed Pearl, Xanthan Gum | 1,000 | 1,000 |
| Natriumhydroxid 10% Lsg. | Sodium Hydroxide | 2,500 | 2,500 |
| Konservierungsmittel | Methyl-, Butyl-, Ethyl-, Propylparaben, Phenoxyethanol | 1,000 | 1,000 |
| Ethanol 96 % | Alcohol Denat. | 15,000 | 15,000 |
| Parfümöl P2, P4, P6 oder P7 | Parfum | 0,20 | - |
| Parfümöl P1, P3 oder P5 | Parfum | - | 0,20 |
| Herba Santa Extrakt (*E. angustifolium*) nach Beispiel 1 (10 % in Ethanol) | | 0,1 | - |
| Angereicherter Herba Santa Extrakt (*E. angustifolium*) nach Beispiel 4 (10 % in Ethanol) | | - | 0.05 |

### Anwendungsbeispiel 38: After-Sun Lotion

| **Inhaltsstoffe** | **Gewichts %** | **Gewichts %** |
|---|---|---|
| Acrylat/C10-30 Alkylacrylat Crosspolymer | 0,4 | 0,4 |
| Cetearylethylhexanoat | 15,0 | 15,0 |
| Bisabolol | 0,2 | 0,2 |
| Tocopherylacetat | 1,0 | 1,0 |
| Panthenol | 1,0 | 1,0 |
| Alcohol | 15,0 | 15,0 |
| Glycerol | 3,0 | 3,0 |
| Parfümöl P2, P4, P6 oder P7 | 0,30 | - |
| Parfümöl P1, P3 oder P5 | - | 0,30 |
| Herba Santa Extrakt (*E. angustifolium*) nach Beispiel 1 (10 % in Ethanol) | 0,25 | - |
| Angereicherter Herba Santa Extrakt (*E*. *angustifolium*) nach Beispiel 4 (10 % in Ethanol) | - | 0,15 |
| Pentylene glycol | 4,0 | 4,0 |
| Konservierungsmittel (Methyl-, Butyl-, Ethyl-, Propylparaben, Phenoxyethanol) | 1,0 | 1,0 |
| Demin. Wasser | Ad 100 | Ad 100 |
| Triethanolamin | 0,2 | 0,2 |

### Anwendungsbeispiel 39: Lösung für Feuchttücher

| **Inhaltsstoffe** | **INCI** | **Gewichts %** | **Gewichts %** |
|---|---|---|---|
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 2,00 | 2,00 |
| Dragosantol® 100 | Bisabolol | 0,10 | 0,10 |
| Glycerol 99,5 P. | Glycerol | 5,00 | 5,00 |
| Wasser | Water (Aqua) | Ad 100 | Ad 100 |
| Hydrolite® 5 | Pentylene Glycol | 5,00 | 5,00 |
| Konservierungsmittel | Phenoxyethanol | | |
| D-Panthenol 75 W | Panthenol | 0,80 | 0,80 |
| DragoCalm® | Water (Aqua), Glycerol, Avena Sativa (Oat) Kernel Extract | 1,00 | 1,00 |
| Hamamelis-Distillat | Hamamelis Virginiana (Witch Hazel) Water, Water (Aqua), Alcohol | 1,0 | 1,00 |
| Allplant Essence® Org. Rose Geranium P | Pelargonium Graveolens Flower/Leaf/Stem Water | 1,00 | 1,00 |
| Parfümöl P2, P4, P6 oder P7 | Parfum | 0,10 | - |
| Parfümöl P1, P3 oder P5 | Parfum | - | 0,10 |
| Herba Santa Extrakt nach Beispiel 1 (*E. angustifolium*) (10 % in Ethanol) | | 0,30 | - |
| Angereicherter Herba Santa Extrakt (*E*. *californicum*) nach Beispiel 8 (10 % in Ethanol) | | - | 0,20 |

### Beispiel TS: Teststudie

### TS1: Antiinflammatorische Wirkung in LPS-indizierten humanen Monozyten

Der antiinflammatorische Test wurde in einem Zellkultursystem unter Verwendung humaner Monocyten durchgeführt. Humane Monozyten sind einer der Hauptzelltypen, die an entzündlichen Prozessen in Geweben beteiligt sind; sie sind die Zellen, die hauptsächlich von den von gram-negativen Bakterien produzierten Lipopolysacchariden (LPS) beeinflusst. Darüber hinaus, stellen sie den ersten Schritt in der Kaskade der Entzündungsreaktionen dar, indem sie verschiedene Zytokine, z.B. Interleukin-1 beta, Interleukin-6, Interleukin-8 und den Tumornekrose Faktor alpha (TNFα), aber auch andere Entzündungsparameter (z.B. Prostaglandin E2) ausschütten. Die hier gemessenen Parameter sind anerkannte Entzündungsmediatoren. Die Verwendung von primären humanen Monozyten erlaubt eine realitätsnahe Abbildung der pathophysiologischen Situation.

Humane primäre Monozyten wurden für die Experimente in 24-well-plates gesät (ca. 500 000 cells/ml in 1 ml). Die Zellviabilität wurde mit Hilfe der AlamarBlue-Methode oder durch Messung des intrazellulären ATP-Levels bestimmt.

Die Zellen wurden mit dem Stimulus (LPS) für 24 h inkubiert. Lösungen des Herba Santa-Rohextrakt, zweier Flavonoidfraktionen und HED (vorliegend als Na⁺ Salz) wurden 30 min vor der Behandlung mit LPS zugegeben. Nach 24 h wurde der Überstand entfernt, zentrifugiert und nach den Arbeitsanweisungen des jeweiligen Herstellers der verwendeten Immunoassays untersucht.

## Patentansprüche

1. Verbindung der Formel (X) 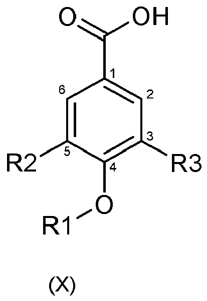 oder
- Salz einer Verbindung der Formel (X)
oder
- Mischung umfassend oder bestehend aus
zwei oder mehr unterschiedlichen Verbindungen der Formel (X),
zwei oder mehr unterschiedlichen Salzen von Verbindungen der Formel (X)
oder
einer oder mehreren unterschiedlichen Verbindungen der Formel (X) und einem oder mehreren unterschiedlichen Salzen von Verbindungen der Formel (X),
wobei für R1, R2 und R3 unabhängig voneinander in jeder Verbindung der Formel (X) gilt:
- R1 bedeutet Wasserstoff oder Methyl,
- R2 bedeutet einen organischen Rest mit 5 Kohlenstoffatomen und einem oder keinem Sauerstoffatom und
- R3 bedeutet einen organischen Rest mit 10 Kohlenstoffatomen und einem oder
mehreren, vorzugsweise einem oder zwei, Sauerstoffatomen,
oder
- R1 und R2 bilden zusammen mit den Kohlenstoffatomen in den Positionen 4 und 5 und dem an das Kohlenstoffatom in Position 4 gebundenen Sauerstoffatom einen Ring und umfassen 5 Kohlenstoffatome sowie ein oder kein Sauerstoffatom, und
- R3 bedeutet einen organischen Rest mit 10 Kohlenstoffatomen und einem oder
mehreren, vorzugsweise einem oder zwei, Sauerstoffatomen,
zur Anwendung in einem Verfahren zur Prophylaxe und/oder Behandlung von Entzündungen, insbesondere von Entzündungen der Haut, insbesondere in einem Verfahren
- zum Reduzieren der Freisetzung von TNF-alpha, und/oder
- zum Reduzieren der Freisetzung eines Interleukins, vorzugsweise von IL-1, IL-6 und/oder IL-8, und/oder
- zum Reduzieren der Freisetzung eines Prostaglandins, vorzugsweise von PGE2, und/oder
- zum Reduzieren der Freisetzung von Interferon-gamma und/oder NF-κB.

2. Verbindung, Salz oder Mischung nach Anspruch 1, wobei das Verfahren zur Prophylaxe und/oder Behandlung von Entzündungen
(f) ein Verfahren zur Prophylaxe und/oder zum Behandeln von chronisch entzündlichen Erkrankungen, insbesondere Darmerkrankungen,
und/oder
(g) ein Verfahren zum Stärken von geschädigter oder ungeschädigter Haut, insbesondere Schleimhaut,
und/oder
(h) ein Verfahren zum Reduzieren von Gewebeschäden, insbesondere Gewebeschäden im Darm,
und/oder
(i) ein Verfahren zum Wiederherstellen einer normalen zellulären Zusammensetzung im Darm,
und/oder
(j) ein Verfahren zum Wiederherstellen oder Stabilisieren der Funktion von Haut, insbesondere von Schleimhaut,
ist bzw. umfasst.

3. Verbindung, Salz oder Mischung nach Anspruch 1 oder 2, wobei für die Gruppen R1, R2 und R3 in der Verbindung der Formel (X) bzw. unabhängig voneinander in einer, mehreren oder sämtlichen, vorzugsweise sämtlichen, Verbindung(en) der Formel (X) gilt:
R3 bedeutet 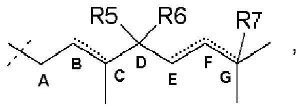 wobei
die gepunktete Linie, welche die mit B und C bezeichneten Kohlenstoffatome verbindet, bedeutet, dass zwischen diesen Kohlenstoffatomen eine Einfachbindung oder eine Doppelbindung vorhanden ist, und
die gepunktete Linie, welche die mit E und G bezeichneten Kohlenstoffatome verbindet, eine einzelne Doppelbindung bedeutet, die entweder zwischen den mit F und G bezeichneten Kohlenstoffatomen oder zwischen den mit E und F bezeichneten Kohlenstoffatomen angeordnet ist,
R7, für den Fall, dass die Doppelbindung zwischen den mit E und F bezeichneten Kohlenstoffatomen angeordnet ist, eine Hydroxygruppe bedeutet oder, für den Fall, dass die Doppelbindung zwischen den mit F und G bezeichneten Kohlenstoffatomen angeordnet ist, entfällt,
R5 und R6 ein Wasserstoffatom und eine Hydroxygruppe bedeuten oder zusammen ein Sauerstoffatom bedeuten,
die gestrichelte Linie die Bindung markiert, die R3 mit dem Kohlenstoffatom in Position 3 verknüpft;
- R1 bedeutet Wasserstoff oder Methyl
und R2 bedeutet 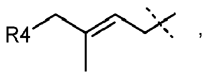 wobei R4 Wasserstoff oder eine Hydroxygruppe bedeutet und die gestrichelte Linie die Bindung markiert, die R2 mit dem Kohlenstoffatom in Position 5 verknüpft
oder
- R1 und R2 zusammen bedeuten 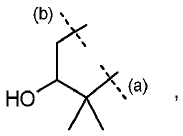 wobei die gestrichelte Linie (a) die Bindung markiert, die das tertiäre Kohlenstoffatom mit dem an das Kohlenstoffatom in Position 4 gebundenen Sauerstoffatom verknüpft und die gestrichelte Linie (b) die Bindung markiert, die das sekundäre Kohlenstoffatom mit dem Kohlenstoffatom in Position 5 verknüpft.

4. Verbindung, Salz oder Mischung nach einem der vorangehenden Ansprüche, wobei die bzw. eine, mehrere oder sämtliche Verbindung(en) der Formel (X) ausgewählt ist bzw. jeweils unabhängig voneinander ausgewählt sind aus der Gruppe bestehend aus den folgenden Verbindungen **(1)** bis **(10):**

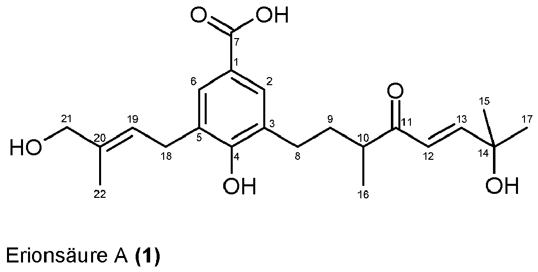

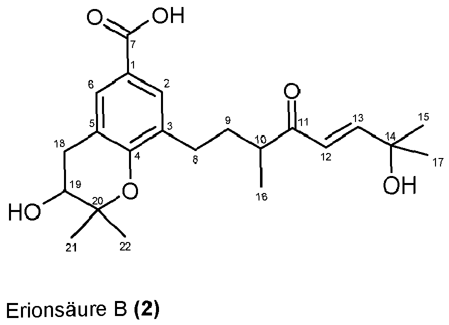

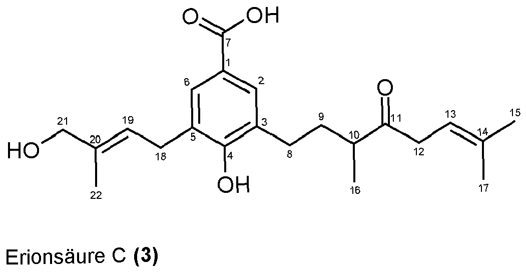

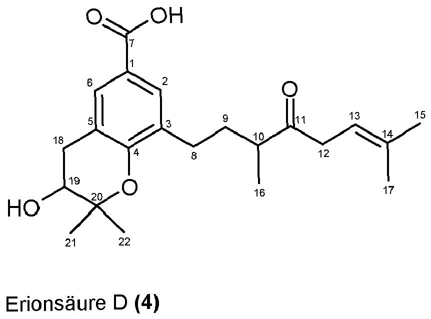

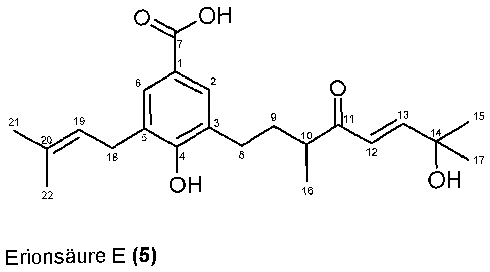

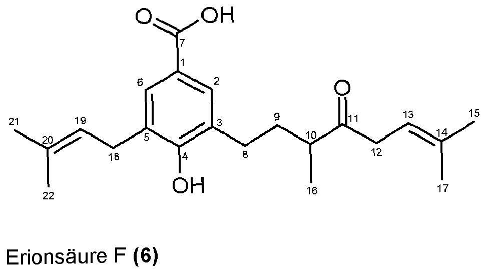

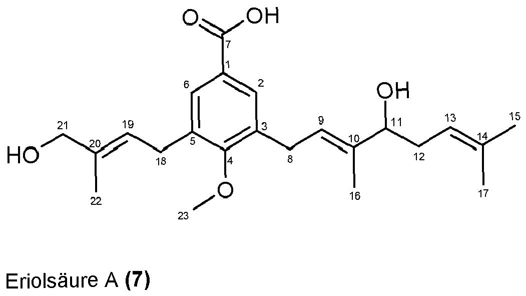

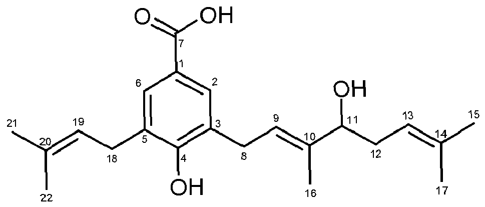

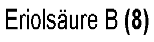

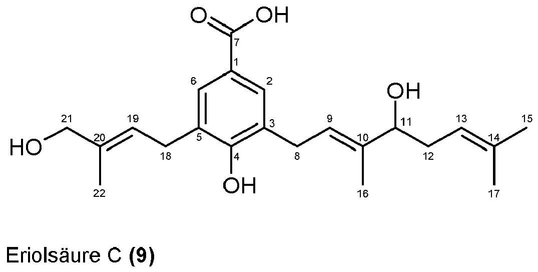

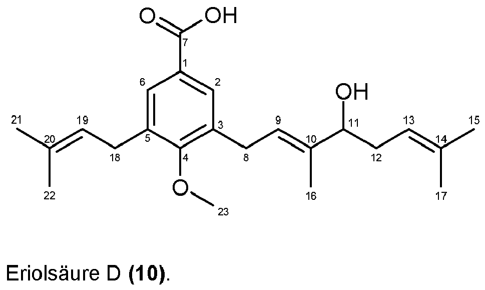

5. Mischung nach einem der vorangehenden Ansprüche, umfassend oder bestehend aus zwei oder mehr unterschiedlichen Verbindungen der Formel (X), vorzugsweise aus zwei, drei, vier, fünf, sechs, sieben, acht, neun oder zehn unterschiedlichen Verbindungen der Formel (X), vorzugsweise ausgewählt aus der Gruppe bestehend aus den Verbindungen **(1)** bis **(10)** wie in Anspruch 4 definiert.

6. Mischung zur Anwendung in einem Verfahren zur Prophylaxe und/oder Behandlung von Entzündungen, umfassend eine Verbindung der Formel (X) nach einem der vorangehenden Ansprüche oder ein Salz der Formel (X) nach einem der vorangehenden Ansprüche, oder Mischung nach einem vorangehenden Ansprüche, zudem umfassend
- ein Hydroxyflavon der Formel (Y) 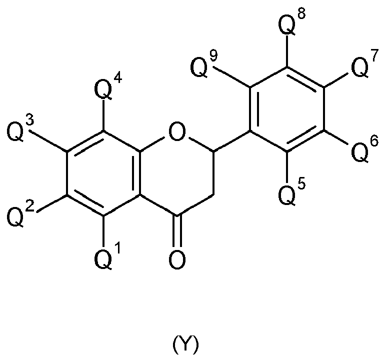 oder
- ein Salz eines Hydroxyflavons der Formel (Y)
oder
- eine Mischung umfassend oder bestehend aus zwei oder mehr unterschiedlichen Hydroxyflavonen der Formel (Y), zwei oder mehr unterschiedlichen Salzen von Hydroxyflavonen der Formel (Y) oder einem oder mehreren unterschiedlichen Hydroxyflavonen der Formel (Y) und einem oder mehreren unterschiedlichen Salzen von Hydroxyflavonen der Formel (Y),
wobei für Q1, Q2, Q3, Q4, Q5, Q6, Q7, Q8 und Q9 unabhängig voneinander in jedem Hydroxyflavon der Formel (Y) gilt:
Q1 bis Q9 bedeuten unabhängig voneinander Wasserstoffatome, Hydroxygruppen, Methyl-, Ethyl-, 1-Propyl-, Methoxy-, Ethoxy-, 1-Propyloxy- oder 2-Propyloxygruppen, mit der Maßgabe, dass mindestens einer der Reste Q1 bis Q9 eine Hydroxygruppe darstellt,
und wobei vorzugsweise gilt:
Q2, Q4, Q5, Q8 und Q9 stellen Wasserstoffatome dar,
Q1, Q3 und Q6 bedeuten unabhängig voneinander Wasserstoffatome, Hydroxy- oder Methoxygruppen, mit der Maßgabe, dass mindestens einer der Reste Q1 und Q3 eine Hydroxygruppe darstellt,
und
Q7 stellt eine Hydroxygruppe dar.

7. Mischung nach Anspruch 6, umfassend eine, mehrere oder sämtliche Verbindungen der Formel (Y) ausgewählt aus der Gruppe bestehend aus Homoeriodictyol, Sterubin, Eriodictyol, Hesperetin, Chrysoeriol und Luteolin, vorzugsweise umfassend Homoeriodictyol.

8. Mischung nach einem der vorangehenden Ansprüche, insbesondere nach einem der Ansprüche 6 oder 7, wobei
- der Anteil der Gesamtmenge an Verbindungen der Formel (X) und Salzen von Verbindungen der Formel (X) in der Mischung, bezogen auf das Gesamtgewicht der Mischung, 1 bis 99 Gew.-%, vorzugsweise 10 bis 99 Gew.-%, besonders bevorzugt 20 bis 80 Gew.-%, beträgt,
und/oder
- der Anteil der Gesamtmenge an Verbindungen der Formel (Y) und Salzen von Verbindungen der Formel (Y) in der Mischung, bezogen auf das Gesamtgewicht der Mischung, 1 bis 99 Gew.-%, vorzugsweise 10 bis 99 Gew.-%, besonders bevorzugt 20 bis 80 Gew.-%, beträgt,
vorzugsweise wobei der Anteil der Gesamtmenge an Verbindungen der Formel (X), Verbindungen der Fomel (Y), Salzen von Verbindungen der Formel (X) und Salzen von Verbindungen der Formel (Y) in der Mischung, bezogen auf das Gesamtgewicht der Mischung, 0,0001 bis 100 Gew.-%, vorzugsweise 0,001 bis 100 Gew.-%, besonders bevorzugt 0,1 bis 90 Gew.-%, weiter bevorzugt 1 bis 90 Gew.-% beträgt.

9. Mischung nach einem der vorangehenden Ansprüche, wobei die Mischung einen pflanzlichen Extrakt umfasst oder daraus besteht, vorzugsweise einen Extrakt aus *Eriodictyon ssp*., besonders bevorzugt einen Extrakt aus *Eriodictyon californicum* und/oder *Eriodictyon angustifolium*, wobei der Anteil der Gesamtmenge an Verbindungen der Formel (X) und Salzen von Verbindungen der Formel (X) in der Mischung, bezogen auf das Gesamtgewicht der Mischung, vorzugsweise 0,1 bis 100 Gew.-%, weiter bevorzugt 1 bis 100 Gew.-%, besonders bevorzugt 10 bis 100 Gew.-%, weiter bevorzugt von 10 bis 90 Gew.-% beträgt.

10. Mischung nach einem der Ansprüche 6 bis 9, wobei das Verhältnis der Gesamtmenge an Verbindungen der Formel (X) und Salzen von Verbindungen der Formel (X) zu der Gesamtmenge an Verbindungen der Formel (Y) und Salzen von Verbindungen der Formel (Y) im Bereich von 0,00001 : 1 bis 1 : 0,00001, insbesondere im Bereich von 0,0001 : 1 bis 1 : 0,0001, bevorzugt im Bereich von 0,001 : 1 bis 1 : 0,001, vorzugsweise im Bereich von 0,01 : 1 bis 1 : 0,01, besonders bevorzugt im Bereich von 0,1 : 1 bis 1 : 0,1, weiter bevorzugt im Bereich von 0,5 : 1 bis 1 : 0,5 liegt, jeweils bezogen auf das Gewicht.

11. Zubereitung, insbesondere der Ernährung oder dem Genuss dienende Zubereitung, pharmazeutische Zubereitung, kosmetische Zubereitung oder dermatologische Zubereitung, umfassend eine Verbindung, ein Salz oder eine Mischung wie in einem der vorangehenden Ansprüche definiert,
zur Anwendung in einem Verfahren zur Prophylaxe und/oder Behandlung von Entzündungen, insbesondere von Entzündungen der Haut, insbesondere in einem Verfahren
- zum Reduzieren der Freisetzung von TNF-alpha, und/oder
- zum Reduzieren der Freisetzung eines Interleukins, vorzugsweise von IL-1, IL-6 und/oder IL-8, und/oder
- zum Reduzieren der Freisetzung eines Prostaglandins, vorzugsweise von PGE2, und/oder
- zum Reduzieren der Freisetzung von Interferon-gamma und/oder NF-κB.

12. Zubereitung nach Anspruch 11, wobei das Verfahren zur Prophylaxe und/oder Behandlung von Entzündungen
(f) ein Verfahren zur Prophylaxe und/oder zum Behandeln von chronisch entzündlichen Erkrankungen, insbesondere Darmerkrankungen,
und/oder
(g) ein Verfahren zum Stärken von geschädigter oder ungeschädigter Haut, insbesondere Schleimhaut,
und/oder
(h) ein Verfahren zum Reduzieren von Gewebeschäden, insbesondere Gewebeschäden im Darm,
und/oder
(i) ein Verfahren zum Wiederherstellen einer normalen zellulären Zusammensetzung im Darm,
und/oder
(j) ein Verfahren zum Wiederherstellen oder Stabilisieren der Funktion von Haut, insbesondere von Schleimhaut,
ist bzw. umfasst.

13. Mischung nach Anspruch 9, vorzugsweise nach Anspruch 10 in Verbindung mit Anspruch 9, wobei die Mischung herstellbar ist durch ein Verfahren mit folgenden Schritten:
(a) Extrahieren von Pflanzenmaterial aus *Eriodictyon ssp*., bevorzugt aus *Eriodictyon californicum* und/oder *Eriodictyon angustifolium*, so dass eine Mischung entsteht, die Verbindungen der Formel (X), gegebenenfalls Verbindungen der Formel (Y) sowie sonstige extrahierte Verbindungen umfasst, und
(b) Anreichern von extrahierten Verbindungen der Formel (X) und/oder Salzen der extrahierten Verbindungen der Formel (X) sowie gegebenenfalls Verbindungen der Formel (Y) und/oder Salzen der extrahierten Verbindungen der Formel (Y) in der Mischung durch teilweises oder vollständiges Entfernen sonstiger extrahierter Verbindungen und gegebenenfalls Entfernen von Extraktions- und/oder Lösemitteln,
vorzugsweise so dass der Anteil der Gesamtmenge an Verbindungen der Formel (X) und Salzen von Verbindungen der Formel (X) in der Mischung bezogen auf das Gesamtgewicht der Mischung 0,1 bis 100 Gew.-%, weiter bevorzugt 1 bis 100 Gew.-%, besonders bevorzugt 10 bis 100 Gew.-%, weiter bevorzugt von 10 bis 90 Gew.-% beträgt.

14. Mischung oder Zubereitung nach einem der vorangehenden Ansprüche, insbesondere Zubereitung nach Anspruch 11 oder 12, zudem umfassend einen oder mehrere weitere Bestandteile ausgewählt aus der Gruppe bestehend aus probiotische Bakterien, Präbiotika, Synbiotika, Ballaststoffe, Molkenproteine, Sojaproteine, Mineralstoffe, Tocopherole, Vanille, Vanille-Extrakte, Omega-3-Fettsäuren, Citrus-, Apfel-, Traubenkern-, Grüntee-, Rosmarin-, Estragon-, Thymian-, Meerrettich- und Macis-Extrakte, Tannine, Tomaten-, Melonen- und Hagebutten-Extrakte, beta-Carotin; Aubergine, Rhabarber, rote Zwiebel, Rotkohl, Schwarzkarotte, Superfruits, insbesondere Açai, Noni, Goji, Granatapfel, Mangosteen, Johannisbeeren, Erdbeeren, Aronia, Blaubeeren und/oder Holunderbeeren, bevorzugt in Form von getrockneten Früchten, Extrakten oder Fruchtzbereitungen; Soja-Isoflavone, nichtsteroidale Antiphlogistika, Antibiotika, Budesonid, systemisch wirksame Steroide, Sulfasalazin, Azathioprin/6-Mercaptopurin, Methotrexat, anti-TNF-alpha-Antikörper, Bisabolol, Natriumlaurylsulfat, Chlorhexidin, Metall-Fluoride, organische und anorganische Fluoride, Aromastoffe, ätherische Öle, Kühlwirkstoffe, insbesondere Menthol, Extrakte oder Reinstoffe aus Eukalyptus, Thymian, Wintergrün, Spearmint und Pfefferminze.

15. Verbindung, Salz, Mischung oder Zubereitung wie in einem der vorangehenden Ansprüche definiert, zur Anwendung in einem Verfahren zur Behandlung von tierischer oder menschlicher Haut, die einer Behandlung mit anti-inflammatorischen Wirkstoffen bedarf.
